# EUROPEAN PATENT APPLICATION

(11) **EP 4 477 674 A1**
(43) Date of publication of application: **18.12.2024**
(21) Application number: 23752906.0
(22) Date of filing: 08.02.2023
(51) Int. Cl.: C07K 19/00, A61K 39/395, A61K 47/68, A61K 49/00, A61P 35/00, C07K 14/00, C07K 14/52, C07K 16/00, C07K 16/18, C07K 16/28, C12N 1/15, C12N 1/19, C12N 1/21, C12N 5/10, C12N 15/13, C12P 21/02, C12P 21/08, C40B 40/10, G01N 33/531

(54) **ENVIRONMENTALLY RESPONSIVE MASKED ANTIBODY AND USE THEREOF**

(30) Priority: 09.02.2022 JP 2022019125; 08.12.2022 JP 2022196524
(71) Applicant: Daiichi Sankyo Company, Limited, Tokyo 103-8426 (JP)
(72) Inventor: KUDO, Shota, Tokyo 103-8426 (JP); SAEKI, Kazunori, Tokyo 103-8426 (JP)
(74) Representative: Marks & Clerk LLP
(86) International application number: PCT/JP2023/004196
(87) International publication number: WO 2023/153442

(57) **Abstract**

This invention provides a masked antibody having performance superior to that of conventional masked antibodies. The masked antibody is a target-antigen-binding molecule comprising a moiety [a], a moiety [b], and a moiety [c]: i.e., a moiety [a]: a moiety binding to a target antigen; a moiety [b]: a first peptide recognizing a target antigen-binding site in the moiety [a]; and a moiety [c]: a second peptide consisting of an amino acid sequence comprising amino acids that are converted from an uncharged state to a positively charged state in response to a pH change from neutral to acidic conditions at a pH 7.5 or lower.

## Description

### Technical Field

The present invention relates to a masked antibody activated in a particular environment.

### Background Art

Antibodies have very high recognition specificity or binding intensity to antigens; i.e., target molecules, and a wide variety of molecules such as low-molecular-weight compounds, peptides, and proteins, can be antigens. In addition, antibody drugs can exert high productivity and stability *in vivo,* and, therefore, development thereof targeting a wide variety of diseases, such as cancer, immunity disorders, and infections, has been in progress. In recent years, research and development of antibody drugs with higher efficacy, such as antibody drug conjugates (ADCs) comprising cytotoxic agents bound to antibodies and bispecific antibodies capable of crosslinking target cells to cytotoxic T cells to attack the target cells (T-cell engaging (TCE)), have been in progress. ADC and TCE exert potent anti-tumor activity. When target molecules are expressed in healthy tissue, however, ADC and TCE become toxic on the healthy tissue through the target molecules (Non-Patent Literature 1).

Modified antibodies that specifically act on tumor tissue with the utilization of the properties of the tumor microenvironment (e.g., enhanced protease activity in tumor tissue); that is, masked antibodies, have been known. Since binding intensity of masked antibodies is suppressed in healthy tissue, masked antibodies are expected as antibody drugs with high safety because of the low toxicity on healthy tissue through the target molecule (Non-Patent Literature 2). A masked antibody is composed of an antibody, a masking domain for inhibiting antibody binding, and a cleavable linker linking the antibody to the masking domain, which can be cleaved by a protease (Patent Literature 1). As masking domains, for example, a coiled-coil domain that does not directly interact with an antibody has been known in addition to a mimotope peptide that binds to a complementarity determining region (CDR) of an antibody (Non-Patent Literature 3). A cleavable linker comprises substrates for extracellular proteases exhibiting enhanced activity in tumor tissue, such as matrix metalloproteinase (MMP) and urokinase type plasminogen activator (uPA). This enables activation of tumor-tissue-selective antibodies (Patent Literature 2). In the preceding study on masking of the anti-EGFR antibody (Cetuximab), for example, activation of masked antibodies in tumor tissue, lowering in the exposure to the skin where EGFR is expressed, the prolonged blood half life, and improved safety have been reported (Non-Patent Literature 4). In addition, masking techniques have been applied to a wide variety of biological molecules, such as bispecific antibodies or cytokines (Patent Literatures 3 and 4).

As the features of the tumor microenvironment, acidification of the extracellular pH (pHe) is known in addition to the enhanced protease activity. Acidification of tumor tissue is considered to result from sugar-metabolism-dependent accumulation of lactic acid in cancer cells, and various transporters, such as a Na⁺/H⁺ exchanger (NHE), a monocarboxylic acid transporter, and a H⁺-ATPase, are reported to be associated with extracellular proton release. Lowering of the pHe in tumor tissue is associated with activation of a secreted lysosome protease and assists metastasis and invasion of cancer cells. In addition, such lowering influences drug resistance and immune escape of cancer cells (Non Patent Literature 5).

As antibody techniques aimed at improved tumor tissue specificity with the use of lowering of the pHe in the tumor microenvironment, a method of introducing a mutation into an antibody CDR region to enhance the antigen-binding intensity at low pH (Patent Literature 5, Non Patent Literature 6) and a technique of a masked antibody having a masking domain that binds to an antibody in a pH-dependent manner (Patent Literature 6) are known. In the latter case, a masking peptide that binds to the anti-CTLA-4 antibody under neutral pH conditions but does not bind thereto under acidic pH conditions is reported.

### Citation List

### Patent Literature

Patent Literature 1: WO 2009/025846
Patent Literature 2: WO 2010/081173
Patent Literature 3: WO 2016/014974
Patent Literature 4: WO 2020/069398
Patent Literature 5: WO 2010/104821
Patent Literature 6: WO 2018/218076

### Non Patent Literature

Non Patent Literature 1: Nature Review Drug Discovery; 17: 197-223, 2018
Non Patent Literature 2: Expert Opin. Biol. Ther.; 14 (8): 1049-53, 2014
Non Patent Literature 3: ACS Cent. Sci.; 7: 724-38, 2021
Non Patent Literature 4: Sci. Transl. Med.; 5, 207: 1-10, 2013
Non Patent Literature 5: Cancer Cell International., 13 (89): 1-8, 2013
Non Patent Literature 6: Proc. Natl. Acd. Sci., 118 (9): 1-10, 2021

### Summary of Invention

### Technical Problem

The present invention provides a masked antibody with performance superior to that of conventional masked antibodies.

### Solution to Problem

The present inventors have conducted concentrated studies in order to dissolve the problems described above. They focused on acidification of the pHe in the tumor environment and improved a linker that would ligate the masking peptide of the masked antibody to the antibody. This has led to the completion of the present invention.

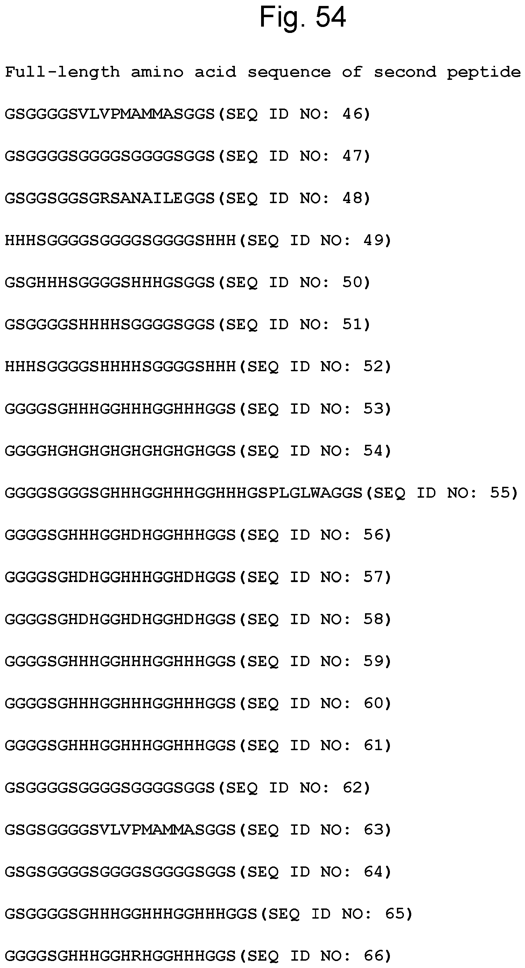

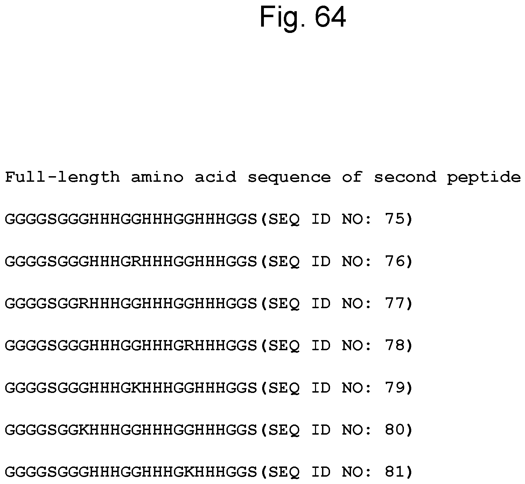

Specifically, the present invention includes the following.
[1] A molecule comprising a moiety [a], a moiety [b], and a moiety [c] indicated below and binding to a target antigen:
   a moiety [a]: a moiety binding to the target antigen;
   a moiety [b]: a first peptide recognizing a target antigen-binding site in the moiety [a]; and
   a moiety [c]: a second peptide consisting of an amino acid sequence comprising amino acids that are converted from an uncharged state to a positively charged state in response to a pH change from neutral to acidic conditions at a pH 7.5 or lower.
[2] The molecule according to [1], which exhibits higher binding affinity to the target antigen under acidic conditions than under neutral conditions.
[3] The molecule according to [1] or [2], which comprises one or two or more of the moiety [a], the moiety [b], and the moiety [c].
[4] The molecule according to any one of [1] to [3], which comprises one or two or more of the moiety [a], the moiety [b], and the moiety [c] and the number of the moiety [a], that of the moiety [b], and that of the moiety [c] are identical to each other.
[5] The molecule according to [1] or [2], which comprises 2 moieties [a], 2 moieties [b], and 2 moieties [c].
[6] The molecule according to any one of [1] to [5], wherein the second peptide consists of an amino acid sequence comprising 4 or more amino acids that are converted from an uncharged state to a positively charged state in response to a pH change from neutral to acidic conditions at a pH 7.5 or lower and has a pI value exceeding 6.4.
[7] The molecule according to any one of [1] to [6], wherein the second peptide consists of an amino acid sequence comprising 4 or more amino acids that are converted from an uncharged state to a positively charged state in response to a pH change from neutral to acidic conditions at a pH 7.5 or lower and has a pI value of 6.8 or higher.
[8] The molecule according to any one of [1] to [7], wherein the second peptide consists of an amino acid sequence comprising 4 or more amino acids that are converted from an uncharged state to a positively charged state in response to a pH change from neutral to acidic conditions at a pH 7.5 or lower and has a pI value of 7.2 or higher.
[9] The molecule according to any one of [1] to [8], wherein the second peptide consists of an amino acid sequence comprising 4 or more amino acids that are converted from an uncharged state to a positively charged state in response to a pH change from neutral to acidic conditions at a pH 7.5 or lower and has a pI value of 7.6 or higher.
[10] The molecule according to any one of [1] to [9], wherein the second peptide consists of an amino acid sequence comprising no negatively charged amino acids under acidic pH conditions.
[11] The molecule according to any one of [1] to [10], wherein the second peptide consists of an amino acid sequence that does not comprise aspartic acid and/or glutamic acid.
[12] The molecule according to any one of [1] to [11], wherein the second peptide consists of an amino acid sequence having the configurations (1) and (2) below:
   (1) at least 1 of (a) an amino acid sequence consisting of 2 or more consecutive amino acids having basic functional groups with pKa of 7 or lower in side chains or (b) an amino acid sequence derived from the amino acid sequence (a) with insertion or addition of an arginine or lysine residue at any position (including an amino- or carboxyl terminus) (the amino acid sequence (a) and the amino acid sequence (b) are each referred to as a cluster of basic amino acids); and
   (2) an amino acid sequence comprising 4 or more of the amino acids having basic functional groups with pKa of 7 or lower in side chains.
[13] The molecule according to any one of [1] to [12], wherein the second peptide consists of an amino acid sequence having the configurations (1) and (2) below:
   (1) comprising at least 1 amino acid sequence derived from the amino acid sequence consisting of 2 or more consecutive amino acids having basic functional groups with pKa of 7 or lower in side chains with insertion or addition of an arginine residue at any site (including an amino or carboxyl terminus); and
   (2) comprising an amino acid sequence comprising 4 or more of the amino acids having basic functional groups with pKa of 7 or lower in side chains.
[14] The molecule according to any one of [1] to [13], wherein the second peptide consists of an amino acid sequence having the configurations (1) and (2) below:
   (1) comprising an amino acid sequence derived from the amino acid sequence consisting of 2 or more consecutive amino acids having basic functional groups with pKa of 7 or lower in side chains with insertion or addition of an arginine residue at any site (including an amino or carboxyl terminus); and
   (2) comprising an amino acid sequence comprising 4 or more of the amino acids having basic functional groups with pKa of 7 or lower in side chains.
[15] The molecule according to any one of [1] to [14], wherein the second peptide consists of an amino acid sequence having the configurations (1) and (2) below:
   (1) comprising an amino acid sequence represented by RHHH; and
   (2) comprising an amino acid sequence comprising 4 or more of the amino acids having basic functional groups with pKa of 7 or lower in side chains.
[16] The molecule according to any one of [1] to [12], wherein the second peptide consists of an amino acid sequence comprising at least 1 of the 2 or more consecutive amino acids having basic functional groups with pKa of 7 or lower in side chains and 4 or more of the amino acids having basic functional groups with pKa of 7 or lower in side chains.
[17] The molecule according to any one of [1] to [11], wherein the second peptide consists of an amino acid sequence comprising amino acids having basic functional groups with pKa of 7 or lower in side chains at every other position.
[18] The molecule according to any one of [12] to [17], wherein the basic functional group has pKa of 5.0 to 7.0.
[19] The molecule according to any one of [12] to [18], wherein the basic functional group has pKa of 5.5 to 6.5.
[20] The molecule according to any one of [12] to [19], wherein the amino acids comprising basic functional groups with pKa of 7 or lower in side chains are natural amino acids.
[21] The molecule according to any one of [12] to [19], wherein the amino acids comprising basic functional groups with pKa of 7 or lower in side chains are unnatural amino acids.
[22] The molecule according to any one of [12] to [20], wherein the amino acids comprising basic functional groups with pKa of 7 or lower in side chains are histidine residues.
[23] The molecule according to any one of [1] to [22], wherein the second peptide consists of an amino acid sequence comprising 1 to 4 clusters of basic amino acids.
[24] The molecule according to any one of [1] to [23], wherein the second peptide consists of 10 to 60 amino acids.
[25] The molecule according to any one of [1] to [24], wherein the second peptide consists of 12 to 50 amino acids.
[26] The molecule according to any one of [1] to [25], wherein the second peptide consists of 15 to 40 amino acids.
[27] The molecule according to any one of [1] to [26], wherein the second peptide consists of 20 to 35 amino acids.
[28] The molecule according to any one of [1] to [16] and [18] to [27], wherein the second peptide comprises an amino acid sequence represented by any one of SEQ ID NOs: 49 to 53, 55, 59 to 61, 65, 66, and 75 to 81 (Figure 54 and Figure 64).
[29] The molecule according to any one of [17] to [27], wherein the second peptide comprises an amino acid sequence represented by SEQ ID NO: 54 (Figure 54).
[30] The molecule according to any one of [1] to [29], wherein the EC₅₀ ratio is 3 or higher.
[31] The molecule according to any one of [1] to [30], wherein the EC₅₀ ratio is 10 or higher.
[32] The molecule according to any one of [1] to [31], wherein the EC₅₀ ratio is 30 or higher.
[33] The molecule according to any one of [1] to [32], wherein the second peptide consists of an amino acid sequence comprising 10 or fewer amino acids having basic functional groups with pKa of 7 or lower in side chains.
[34] The molecule according to any one of [1] to [33], wherein the amino acid sequence of the second peptide comprises 2 or more clusters of basic amino acids, a cluster of basic amino acids is positioned at the amino or carboxyl terminus of the amino acid sequence, and other clusters of basic amino acids are not positioned at the amino or carboxyl terminus of the amino acid sequence.
[35] The molecule according to any one of [1] to [33], wherein the amino acid sequence of the second peptide comprises 2 or more clusters of basic amino acids, a cluster of basic amino acids is positioned at the amino terminus of the amino acid sequence, and another cluster of basic amino acids is positioned at the carboxyl terminus.
[36] The molecule according to any one of [1] to [33], wherein none of the clusters of basic amino acids comprised in the amino acid sequence of the second peptide are positioned at the amino or carboxyl terminus of the amino acid sequence.
[37] The molecule according to any one of [1] to [34] and [36], wherein the amino acid sequence of the second peptide comprises an amino acid sequence cleaved by an intracellular and/or extracellular protease and an amino acid sequence comprising a cluster of basic amino acids, wherein the amino acid sequence of the second peptide comprises:
   (1) an amino acid sequence comprising a cluster of basic amino acids and an amino acid sequence cleaved by an intracellular and/or extracellular protease connected in that order from the amino terminus toward the carboxyl terminus; or
   (2) an amino acid sequence cleaved by an intracellular and/or extracellular protease and an amino acid sequence comprising a cluster of basic amino acids connected in that order from the carboxyl terminus toward the amino terminus.
[38] The molecule according to any one of [12] to [37], wherein the number of amino acids having basic functional groups with pKa of 7 or lower in side chains in the amino acid sequence of the second peptide is 5% to 30% of the total number of amino acids comprised in the amino acid sequence.
[39] The molecule according to any one of [1] to [38], which comprises the first peptide, the second peptide, and the moiety binding to a target antigen connected in that order.
[40] The molecule according to any one of [1] to [39], wherein the moiety binding to a target antigen is a polypeptide consisting of an amino acid sequence that is not comprised in the first peptide or the second peptide.
[41] The molecule according to any one of [1] to [40], which consists of a polypeptide.
[42] The molecule according to [41], which comprises to the first peptide, the second peptide, and the moiety binding to a target antigen connected from the amino terminus toward the carboxyl terminus.
[43] The molecule according to any one of [1] to [42], wherein a moiety selected from the group consisting of the first peptide, the second peptide, and the moiety binding to a target antigen is connected to either or both of the other two moieties by a linker or spacer (or linkers or spacers).
[44] The molecule according to any one of [1] to [43], wherein a pH of acidic conditions is 6.5 or lower.
[45] The molecule according to any one of [1] to [44], wherein a pH of acidic conditions is 6.0 or lower.
[46] The molecule according to any one of [1] to [45], wherein a pH of acidic conditions is 5.5 or lower.
[47] The molecule according to any one of [1] to [46], wherein the target antigen is an antigen that is present in tumor tissue, an endosome, or a lysosome.
[48] The molecule according to any one of [1] to [47], wherein another moiety [d] binds to a moiety binding to the target antigen and the moiety [d] does not comprise the first peptide and the second peptide.
[49] The molecule according to [48], wherein the moiety [d] is at least 1 substance selected from the group consisting of an antibody or an antigen-binding fragment thereof that is not a moiety binding to the target antigen, a peptide comprising an amino acid sequence that is not comprised in the first peptide or the second peptide, a cytokine, a toxin, a radioactive isotope, a label molecule, a photosensitizer, an immunostimulant, an antitumor compound, a drug, a payload, and a polymer.
[50] The molecule according to [49], wherein the antitumor compound is a camptothecin derivative or a pyrrolobenzodiazepine derivative and the camptothecin derivative is preferably N-[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]-2-hydroxyacetamide.
[51] The molecule according to [49], wherein the immunostimulant is a cyclic dinucleotide derivative.
[52] The molecule according to any one of [48] to [51], which consists of a polypeptide or the moiety [d] and a polypeptide.
[53] The molecule according to any one of [1] to [52], wherein the first peptide is obtained by a method comprising steps (i) to (iii):
   (i) a step of bringing a peptide library comprising a repeat sequence of aromatic amino acid and Pro into contact with the moiety binding to a target antigen as defined in [1];
   (ii) a step of collecting a peptide binding to the moiety binding to a target antigen; and
   (iii) a step of preparing the peptide by recombination, chemical synthesis, or peptide synthesis.
[54] A method for producing the molecule according to any one of [1] to [52], which comprises a step of preparing a peptide comprising an amino acid sequence comprised in the first peptide and/or an amino acid sequence comprised in the second peptide by recombination, *in vitro* translation, chemical synthesis, or peptide synthesis.
[55] A molecule binding to a target antigen, which is obtained by the method according to [54].
[56] A method for producing the molecule according to any one of [41], [42], and [52] comprising a step (iv) below:
   (iv) a step of introducing a polynucleotide comprising a nucleotide sequence encoding an amino acid sequence comprised in a moiety binding to a target antigen and, optionally, an amino acid sequence comprised in the first peptide, and/or an amino acid sequence comprised in the second peptide into a cell, culturing the cell, and collecting a polypeptide binding to the target antigen from the culture product.
[57] A molecule binding to a target antigen, which is obtained by the method according to [56].
[58] A pharmaceutical composition comprising the molecule according to any one of [1] to [53], [55], and [57], a salt thereof, or a hydrate of the molecule or salt.
[59] The pharmaceutical composition according to [58], which is an anti-cancer agent.
[60] The pharmaceutical composition according to [58] or [59], wherein an antigen-binding intensity of a molecule binding to a target antigen under neutral pH conditions is adjusted to be lower than the antigen-binding intensity under acidic pH conditions, so as to reduce the toxicity of the molecule binding to a target antigen imposed on a subject compared with the toxicity of a molecule comprising no amino acids converted from an uncharged state to a positively charged state in response to a pH change of the second peptide from neutral to acidic conditions at a pH 7.5 or lower.
[61] The pharmaceutical composition according to [58] or [59], wherein an antigen-binding intensity of a molecule binding to a target antigen under neutral pH conditions is adjusted to be lower than the antigen-binding intensity under acidic pH conditions, so as to increase the blood concentration of the molecule which binds to the target antigen in a subject, compared with that of a molecule comprising no amino acids that are converted from an uncharged state to a positively charged state in response to a pH change of the second peptide from neutral to acidic conditions at a pH 7.5 or lower
[62] A test drug, diagnostic drug, or reagent comprising the molecule according to any one of [1] to [53], [55], and [57] or the pharmaceutical composition according to [58].
[63] A polynucleotide comprising a nucleotide sequence encoding the amino acid sequence of the molecule according to [41] or [52].
[64] A vector comprising the polynucleotide according to [63].
[65] A cell comprising the polynucleotide according to [63] or the vector according to [64] or producing the molecule according to [41] or [52].

In addition, the present invention includes the following:
[i] a pH-responsive peptide linker comprising the amino acid sequence of the second peptide as defined in [1];
[ii] the peptide linker as defined in [i] comprising an amino acid sequence represented by any one of SEQ ID NOs: 49 to 55, 59 to 61, 65, 66, and 75 to 81 (Figure 54 and Figure 64);
[iii] a polynucleotide comprising a nucleotide sequence encoding the amino acid sequence of the peptide linker as defined in [i] or [ii];
[iv] a vector comprising the polynucleotide as defined in [iii]; and
[v] a cell comprising the polynucleotide as defined in [iii] or the vector as defined in [iv] or producing the peptide linker as defined in [i] or [ii].

The description incorporates the contents disclosed by JP Patent Application No. 2022-19125 and JP Patent Application No. 2022-196524, based on which the priority of the present application claims.

### Brief Description of Drawings

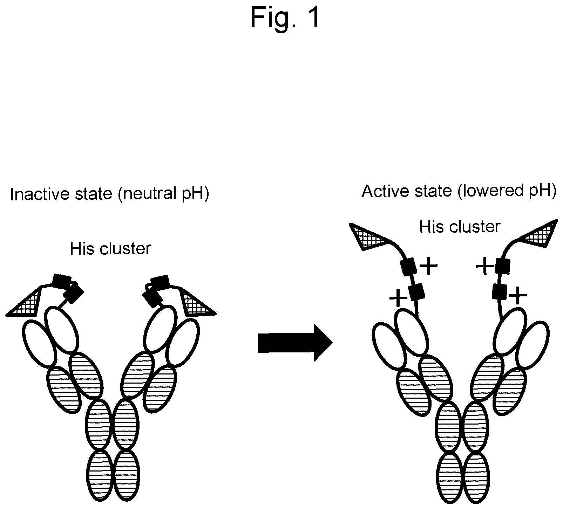

[Figure 1] Figure 1 shows a concept of the present invention. Figure 1 shows an example of a format of a masked antibody that is activated in response to pH changes. A masking domain (lattice) binding to a variable region (white) of an antibody is fused to a variable region of an antibody heavy chain through a linker. In a linker, there is a pH-responsive cluster of histidine residues (His cluster (solid black)), and a masking domain binds to an antibody variable region under neutral pH conditions. At acidic pH, however, histidine residues in the His cluster are positively charged, a structure change is induced in a linker, and a masking domain is detached from the antibody; that is, an antibody can bind to an antigen.
[Figure 2] Figure 2 shows the results of ELISA evaluation of the interaction between the conventional anti-TROP2 antibody HT1-11 described in WO 2015/098099 and the human TROP2 antigen. HT1-11 was found to have bound to the antigen in a concentration-dependent manner.
[Figure 3] Figure 3 shows the output/input ratio of a degree of concentration of a peptide binder after panning of the anti-TROP2 antibody HT1-11 evaluated by a pull-down experiment. A higher value in HT1-11 than in human serum-derived IgG (IgG mixture) indicates concentration of the peptide binding specifically to HT1-11.
[Figure 4-1] Figure 4-1 shows the results of ELISA evaluation of the interaction between the scFv-type anti-TROP2 antibody and the human TROP2 antigen. Binding intensity was evaluated under the condition without the addition of MMP (solid line) and under the condition with the addition of MMP1 (dotted line). (A) HT1-11-scFv-HL exhibited an equivalent binding intensity regardless of the addition of MMP. (B) HT1-11-scFv-LH exhibited an equivalent binding intensity regardless of the addition of MMP.

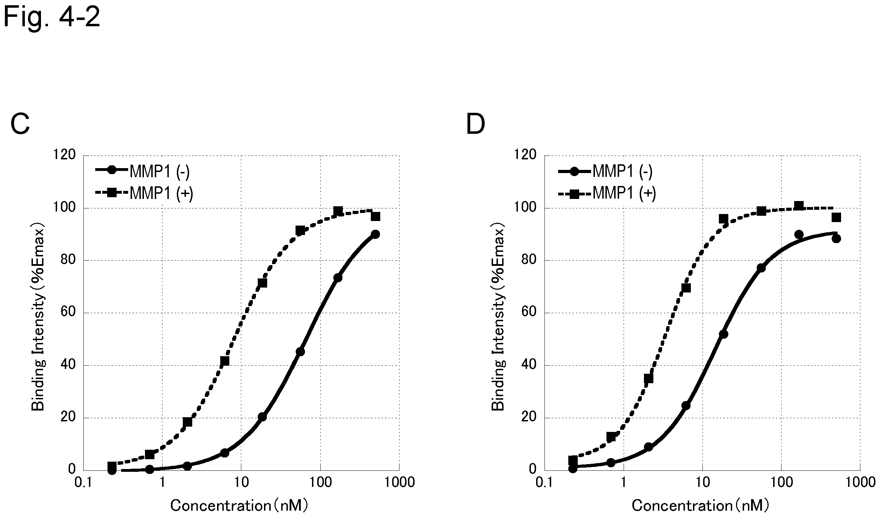

[Figure 4-2] Figure 4-2 shows the results of ELISA evaluation of the interaction between the scFv-type anti-TROP2 antibody and the human TROP2 antigen. Binding intensity was evaluated under the condition without the addition of MMP (solid line) and under the condition with the addition of MMP1 (dotted line). (C) MHT1001 exhibited a higher binding intensity under the condition with the addition of MMP1 than under the condition without the addition of MMP1. (D) MHT1002 exhibited a higher binding intensity under the condition with the addition of MMP1 than under the condition without the addition of MMP1.
[Figure 5-1] Figure 5-1 shows the results of ELISA evaluation of the interaction between the IgG-type anti-TROP2 masked antibody and the human TROP2 antigen. Binding intensity was evaluated under the condition without the addition of a protease (solid line) and under the condition with the addition of a protease (dotted line). (A) HT1-11 exhibited an equivalent binding intensity regardless of the addition of MMP. (B) MHT1007 exhibited a higher binding intensity under the condition with the addition of MMP1 than under the condition without the addition of MMP1.
[Figure 5-2] Figure 5-2 shows the results of ELISA evaluation of the interaction between the IgG-type anti-TROP2 masked antibody and the human TROP2 antigen. Binding intensity was evaluated under the condition without the addition of a protease (solid line) and under the condition with the addition of a protease (dotted line). (C) MHT1008 exhibited an equivalent binding intensity regardless of the addition of MMP. (D) MHT1009 exhibited a higher binding intensity under the condition with the addition of uPA than under the condition without the addition of uPA.
[Figure 6-1] Figure 6-1 shows the results of ELISA evaluation of the interaction between the anti-TROP2 masked antibody comprising the His cluster and the human TROP2 antigen under neutral pH conditions (pH 7.5, solid line) or under acidic pH conditions (pH 5.5, dotted line). (A) MHT1008 exhibited an equivalent binding intensity under both pH conditions. (B) MHT1803 exhibited a higher binding intensity under acidic pH conditions than under neutral pH conditions.
[Figure 6-2] Figure 6-2 shows the results of ELISA evaluation of the interaction between the anti-TROP2 masked antibody comprising the His cluster and the human TROP2 antigen under neutral pH conditions (pH 7.5, solid line) or under acidic pH conditions (pH 5.5, dotted line). (C) MHT1804 exhibited a higher binding intensity under acidic pH conditions than under neutral pH conditions. (D) MHT1805 exhibited a higher binding intensity under acidic pH conditions than under neutral pH conditions.
[Figure 6-3] Figure 6-3 shows the results of ELISA evaluation of the interaction between the anti-TROP2 masked antibody comprising the His cluster and the human TROP2 antigen under neutral pH conditions (pH 7.5, solid line) or under acidic pH conditions (pH 5.5, dotted line). (E) MHT1806 exhibited a higher binding intensity under acidic pH conditions than under neutral pH conditions. (F) MHT1808 exhibited a higher binding intensity under acidic pH conditions than under neutral pH conditions.
[Figure 6-4] Figure 6-4 shows the results of ELISA evaluation of the interaction between the anti-TROP2 masked antibody comprising the His cluster and the human TROP2 antigen under neutral pH conditions (pH 7.5, solid line) or under acidic pH conditions (pH 5.5, dotted line). (G) MHT1809 exhibited a higher binding intensity under acidic pH conditions than under neutral pH conditions. (H) MHT1810 exhibited a higher binding intensity under acidic pH conditions than under neutral pH conditions.

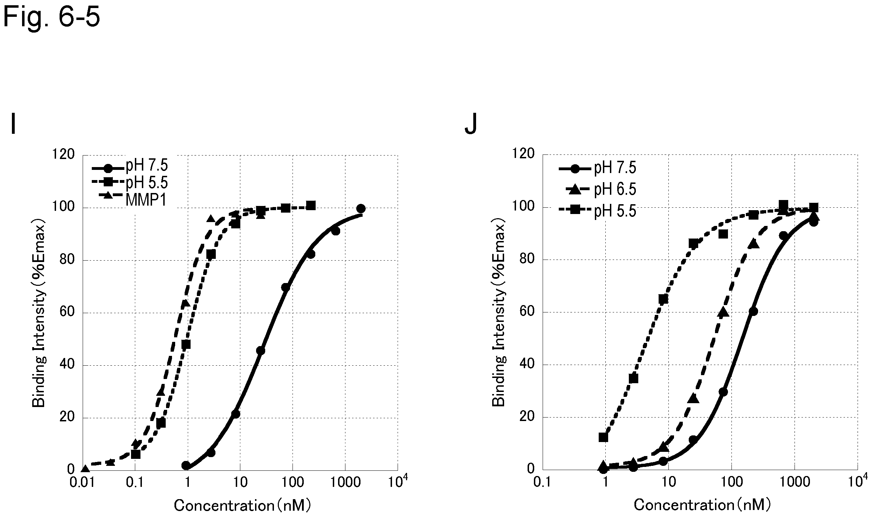

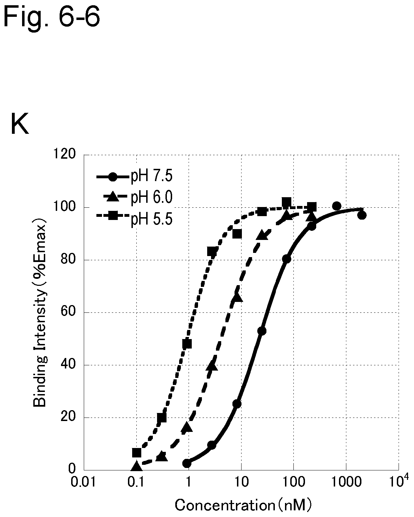

[Figure 6-5] Figure 6-5 shows the results of ELISA evaluation of the interaction between the anti-TROP2 masked antibody comprising the His cluster and the human TROP2 antigen under neutral pH conditions (pH 7.5, solid line), when it was allowed to react with MMP1 (broken line in (I)), under acidic pH conditions (pH 6.5, broken line in (J)), or under acidic pH conditions (pH 5.5, dotted line). (I) MHT1811 comprising the His cluster and the MMP substrate on the linker exhibited a higher binding intensity under acidic pH conditions than under neutral pH conditions. When it was allowed to react with MMP1 (broken line), it exhibited high binding intensity. (J) MHT1806 exhibited a higher binding intensity under acidic pH conditions at a pH 6.5 or lower (broken line) than under neutral pH conditions.
[Figure 6-6] Figure 6-5 shows the results of ELISA evaluation of the interaction between the anti-TROP2 masked antibody comprising the His cluster and the human TROP2 antigen under neutral pH conditions (pH 7.5, solid line), under acidic pH conditions (pH 6.0, broken line), or under acidic pH conditions (pH 5.5, dotted line). (K) MHT1808 exhibited a higher binding intensity under acidic pH conditions at a pH 6.0 or lower (broken line) than under neutral pH conditions.
[Figure 7] Figure 7 shows binding intensities of MHT1808, MHT1817, MHT1818, and MHT1819 at a pH 7.5 (black), a pH 6.5 (gray), and a pH 6.0 (white).
[Figure 8-1] Figure 8-1 shows the results of ELISA evaluation of (A) the interaction between the conventional anti-CD98 antibody hM23H1L1 described in WO 2015/146132 and the human CD98 antigen and (B) the interaction between the conventional EGFR antibody Cetuximab and human EGFR. The antibodies were found to have bound to the antigens in a concentration-dependent manner.
[Figure 8-2] Figure 8-2 shows the results of ELISA evaluation of (C) the interaction between the conventional anti-GPRC5D antibody C3022 described in WO 2018/147245 and the human GPRC5D antigen. The antibody was found to have bound to the antigen in a concentration-dependent manner.
[Figure 9-1] Figure 9-1 shows (A) types of peptide libraries and (B) the output/input ratio of a degree of concentration of a peptide binder after panning of the anti-CD98 antibody hM23H1L1 evaluated by a pull-down experiment. A higher value in the target antibody than in human serum-derived IgG (IgG mixture) indicates concentration of the peptide in a target-antibody-specific manner.
[Figure 9-2] Figure 9-2 shows the output/input ratio of a degree of concentration of a peptide binder after panning of (C) the anti-EGFR antibody Cetuximab or (D) the anti-GPRC5D antibody C3022 with the use of the peptide library shown in Figure 9-1 (A) evaluated by a pull-down experiment. A higher value in the target antibody than in human serum-derived IgG (IgG mixture) indicates concentration of the peptide in a target-antibody-specific manner.
[Figure 10-1] Figure 10-1 shows the results of ELISA evaluation of (A) the interaction between the anti-CD98 masked antibody MhM8001 comprising a pH-responsive linker and the human CD98 antigen and (B) the interaction between the anti-EGFR masked antibody MCE-2101 comprising the same linker and human EGFR. Binding was evaluated under neutral pH conditions (pH 7.5, solid line) or under acidic pH conditions (pH 5.5, dotted line). Both antibodies were found to exhibit a higher binding intensity under acidic pH conditions than under neutral pH conditions.
[Figure 10-2] Figure 10-2 shows the results of ELISA evaluation of (C) the interaction between the anti-GPRC5D masked antibody MC3-9001 comprising the same pH-responsive linker as described above and the human GPRC5D antigen. Binding was evaluated under neutral pH conditions (pH 7.5, solid line) or under acidic pH conditions (pH 5.5, dotted line). Both antibodies were found to exhibit a higher binding intensity under acidic pH conditions than under neutral pH conditions.

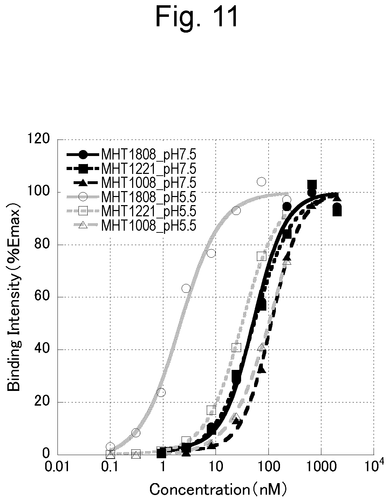

[Figure 11] Figure 11 shows the results of ELISA evaluation of the interaction between the anti-TROP2 masked antibody and the human TROP2 antigen. Binding was evaluated under neutral pH conditions (pH 7.5, black symbol) or under acidic pH conditions (pH 5.5, white symbol). MHT1808 (closed black circle and open white circle) exhibited a higher binding intensity under acidic pH conditions than under neutral pH conditions. MHT1221 (closed black box and open white box) and MHT1008 (black triangle and white triangle) exhibited an equivalent binding intensity under neutral pH conditions and under acidic pH conditions.
[Figure 12] Figure 12 shows the N297 glycan of a glycan-modified antibody (the MSG1-type glycan comprising an azide group introduced into a sialic acid at the non-reducing terminus (WO 2019/065964)).
[Figure 13] Figure 13 shows (A) the results of evaluation by SPR (surface plasmon resonance) of binding intensity of hM23-M1 comprising a point mutation introduced into CDR1 of the hM23H1L1 heavy chain variable region to the human CD98 antigen and (B) the results of ELISA evaluation of binding intensity of MhM1013-M1 comprising the aforementioned mutation introduced into MhM1013 to the human CD98 antigen. MhM1013-M1 (gray) exhibited masking effects equivalent to or higher than those of MhM1013 (black).
[Figure 14] Figure 14 shows the results of ELISA evaluation of the interaction between the anti-CD98 masked antibody and the human CD98 antigen. Binding was evaluated under neutral pH conditions (pH 7.5, black symbol) or under acidic pH conditions (pH 5.5, white symbol). MhM1018-M1 (closed black circle and open white circle) exhibited an equivalent binding intensity under neutral pH conditions and under acidic pH conditions. MhM1024-M1 (closed black box and open white box) exhibited a higher binding intensity under acidic pH conditions than under neutral pH conditions.
[Figure 15-1] Figure 15-1 shows the *in vitro* growth inhibitory activity of the masked antibody ADC at a pH 7.4 (open white circle, gray solid line), a pH 6.5 (square, gray dotted line), a pH 6.0 (closed black circle, black dotted line), and a pH 5.5 (closed black box, black solid line). (A) MHT1808-PBD-ADC exhibited higher growth inhibitory activity as a pH was lowered. (B) MHT1221-PBD-ADC exhibited an equivalent growth inhibitory activity under any conditions. An error bar in the figure indicates a standard error (n = 3).
[Figure 15-2] Figure 15-2 shows the *in vitro* growth inhibitory activity of the masked antibody ADC at a pH 7.4 (open white circle, gray solid line), a pH 6.5 (square, gray dotted line), a pH 6.0 (closed black circle, black dotted line), and a pH 5.5 (closed black box, black solid line). (C) MhM1024-M1-DXd-ADC exhibited higher growth inhibitory activity as a pH was lowered. (D) MhM1018-M1-DXd-ADC exhibited an equivalent growth inhibitory activity under any conditions. An error bar in the figure indicates a standard error (n = 3).
[Figure 16] Figure 16 shows the full-length amino acid sequence of the HT1-11 H chain (SEQ ID NO: 1).
[Figure 17] Figure 17 shows the full-length amino acid sequence of the HT1-11 L chain (SEQ ID NO: 2).
[Figure 18] Figure 18 shows the full-length amino acid sequence of MHT1001 (SEQ ID NO: 3).
[Figure 19] Figure 19 shows the full-length amino acid sequence of MHT1002 (SEQ ID NO: 4).
[Figure 20] Figure 20 shows the full-length amino acid sequence of HT1-11-scFv-HL (SEQ ID NO: 5).
[Figure 21] Figure 21 shows the full-length amino acid sequence of HT1-11-scFv-LH (SEQ ID NO: 6).
[Figure 22] Figure 22 shows the full-length amino acid sequence of the MHT1007 H chain (SEQ ID NO: 7).
[Figure 23] Figure 23 shows the full-length amino acid sequence of the MHT1008 H chain (SEQ ID NO: 8).
[Figure 24] Figure 24 shows the full-length amino acid sequence of the MHT1009 H chain (SEQ ID NO: 9).
[Figure 25] Figure 25 shows the full-length amino acid sequence of the MHT1803 H chain (SEQ ID NO: 10).
[Figure 26] Figure 26 shows the full-length amino acid sequence of the MHT1804 H chain (SEQ ID NO: 11).
[Figure 27] Figure 27 shows the full-length amino acid sequence of the MHT1805 H chain (SEQ ID NO: 12).
[Figure 28] Figure 28 shows the full-length amino acid sequence of the MHT1806 H chain (SEQ ID NO: 13).
[Figure 29] Figure 29 shows the full-length amino acid sequence of the MHT1808 H chain (SEQ ID NO: 14).
[Figure 30] Figure 30 shows the full-length amino acid sequence of the MHT1809 H chain (SEQ ID NO: 15).
[Figure 31] Figure 31 shows the full-length amino acid sequence of the MHT1810 H chain (SEQ ID NO: 16).
[Figure 32] Figure 32 shows the full-length amino acid sequence of the MHT1811 H chain (SEQ ID NO: 17).
[Figure 33] Figure 33 shows the full-length amino acid sequence of the MHT1817 H chain (SEQ ID NO: 18).
[Figure 34] Figure 34 shows the full-length amino acid sequence of the MHT1818 H chain (SEQ ID NO: 19).
[Figure 35] Figure 35 shows the full-length amino acid sequence of the MHT1819 H chain (SEQ ID NO: 20).
[Figure 36] Figure 36 shows the full-length amino acid sequence of the hM23H1L1 H chain (SEQ ID NO: 21).
[Figure 37] Figure 37 shows the full-length amino acid sequence of the hM23H1L1 L chain (SEQ ID NO: 22).
[Figure 38] Figure 38 shows the full-length amino acid sequence of the Cetuximab H chain (SEQ ID NO: 23).
[Figure 39] Figure 39 shows the full-length amino acid sequence of the Cetuximab L chain (SEQ ID NO: 24).
[Figure 40] Figure 40 shows the full-length amino acid sequence of the C3022 H chain (SEQ ID NO: 25).
[Figure 41] Figure 41 shows the full-length amino acid sequence of the C3022 L chain (SEQ ID NO: 26).
[Figure 42] Figure 42 shows the full-length amino acid sequence of the MhM8001 L chain (SEQ ID NO: 27).
[Figure 43] Figure 43 shows the full-length amino acid sequence of the MCE-2101 L chain (SEQ ID NO: 28).
[Figure 44] Figure 44 shows the full-length amino acid sequence of the MC3-9001 H chain (SEQ ID NO: 29).
[Figure 45] Figure 45 shows the full-length amino acid sequence of the MHT1221 H chain (SEQ ID NO: 30).
[Figure 46] Figure 46 shows the full-length amino acid sequence of the hM23-M1 H chain (SEQ ID NO: 31).
[Figure 47] Figure 47 shows the full-length amino acid sequence of the hM23-M1 L chain (SEQ ID NO: 32).
[Figure 48] Figure 48 shows the full-length amino acid sequence of the MhM1013 L chain (SEQ ID NO: 33).
[Figure 49] Figure 49 shows the full-length amino acid sequence of the MhM1018-M1 L chain (SEQ ID NO: 34).
[Figure 50] Figure 50 shows the full-length amino acid sequence of the MhM1024-M1 L chain (SEQ ID NO: 35).
[Figure 51] Figure 51 shows the amino acid sequences recognized by human uPA and serving as substrates therefor and the amino acid sequences recognized by human MMP1 or human MMP9 and serving as substrates therefor (SEQ ID NOs: 36 to 41).
[Figure 52] Figure 52 shows the amino acid sequences of second peptides (SEQ ID NOs: 42 to 44).
[Figure 53] Figure 53 shows the amino acid sequence of the amino terminal peptide of human GPRC5D (SEQ ID NO: 45).
[Figure 54] Figure 54 shows the amino acid sequences of the second peptides (SEQ ID NOs: 46 to 66).
[Figure 55] Figure 55 shows the amino acid sequence recognized by human CAPN1 and serving as a substrate therefor (SEQ ID NO: 67).
[Figure 56-1] Figure 56-1 shows the results of ELISA evaluation of the interaction between the anti-CD98 masked antibody and the human CD98 antigen. (A) and (B) each show the binding intensity of MhM1026-M1 (closed black circle), that of MhM1028-M1 (closed black box), that of MhM1042-M1 (circle), and that of MhM1043-M1(square) at a pH 7.5 and a pH 5.5.
[Figure 56-2] Figure 56-2 shows the results of ELISA evaluation of the interaction between the anti-CD98 masked antibody and the human CD98 antigen. (C) and (D) each show the binding intensity of MhM1026-M1 (closed black circle), that of MhM1044-M1 (closed black box), that of MhM1045-M1 (circle), and that of MhM1046-M1(square) at a pH 7.5 and a pH 5.5.
[Figure 57] Figure 57 shows the full-length amino acid sequence of the MhM1026-M1 L chain (SEQ ID NO: 68).
[Figure 58] Figure 58 shows the full-length amino acid sequence of the MhM1028-M1 L chain (SEQ ID NO: 69).
[Figure 59] Figure 59 shows the full-length amino acid sequence of the MhM1042-M1 L chain (SEQ ID NO: 70).
[Figure 60] Figure 60 shows the full-length amino acid sequence of the MhM1043-M1 L chain (SEQ ID NO: 71).
[Figure 61] Figure 61 shows the full-length amino acid sequence of the MhM1044-M1 L chain (SEQ ID NO: 72).
[Figure 62] Figure 62 shows the full-length amino acid sequence of the MhM1045-M1 L chain (SEQ ID NO: 73).
[Figure 63] Figure 63 shows the full-length amino acid sequence of the MhM1046-M1 L chain (SEQ ID NO: 74).
[Figure 64] Figure 64 shows the amino acid sequences of the second peptides (SEQ ID NOs: 75 to 81).

### Description of Embodiments

Hereafter, the present invention is described in detail.

### 1. Definition

The term "DXd" used herein refers to "N-[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]-2-hydroxyacetamide."

The term "PBD" used herein refers to "pyrrolobenzodiazepine."

The term "mimotope" used herein refers to a "peptide binding to a complementarity determining region (CDR) of an antibody." The amino acid sequence of the mimotope is not necessarily consistent with the amino acid sequence (epitope) of the antigen that is recognized by an antibody (Molecular Immunology; 23 (7): 709-715, 1986).

The term "amino acid" used herein refers to a natural or unnatural amino acid.

Natural amino acids are 20 common amino acids, pyrrolidine, and selenocysteine. The 20 common amino acids are alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine.

Unnatural amino acids are those other than the 20 common amino acids, pyrrolidine, and selenocysteine. Examples of other terms that can be synonymous with "unnatural amino acid" include "an amino acid that is not naturally encoded," "an unnatural-type amino acid," and "an amino acid that is not present in nature." Unnatural amino acids encompass an amino acid that is present in nature as a result of modification of a natural amino acid but is not incorporated into a polypeptide chain that grows with the aid of a translation complex. In addition, examples of unnatural amino acids include, but are not limited to, amino acids that are not present in nature but can be synthesized and amino acids that can be obtained by modification of natural amino acids.

The term "antibody" used herein refers to immunoglobulin comprising a constant region and a variable region. An antibody is not particularly limited, and it may be a naturally occurring or partially or completely synthesized immunoglobulin.

A basic four-chain antibody structure is composed of two identical light chains (L chains) and two identical heavy chains (H chains). A light chain binds to a heavy chain by a single covalent disulfide bond. Two heavy chains are bound to each other by one or more disulfide bonds in accordance with heavy chain isotypes. A light chain and a heavy chain each have an intra-chain disulfide bond at regular intervals. In a light chain and a heavy chain, there are a constant region exhibiting very high amino acid sequence similarity and a variable region exhibiting low amino acid sequence similarity. A light chain comprises, at its amino terminus, a variable region (VL) adjacent to a constant region (CL). A heavy chain comprises, at its amino terminus, a variable region (VH) adjacent to 3 constant regions (CH1/CH2/CH3). VL is paired with VH, and CL is aligned with a first constant region of a heavy chain (CH1). VL is paired with VH to form a single antigen-binding site.

Constant regions of the antibody of the present invention are not particularly limited. The antibody of the present invention to be used for treatment or prevention of human diseases preferably comprises constant regions of a human antibody. Examples of heavy chain constant regions of a human antibody include Cγ1, Cγ2, Cγ3, Cγ4, Cµ, Cδ, Cα1, Cα2, and Cε. Examples of light chain constant regions of a human antibody include Cκ and Cλ.

Fab comprises a heavy chain VH, CH1 adjacent thereto, a light chain VL, and CL adjacent thereto. VH and VL each comprise a complementarity determining region (CDR). A linker or joint may be present between VH and CH1 and between VL and CL.

Fc (also referred to as an "Fc region") is a carboxyl terminal region of a heavy chain constant region, it comprises CH2 and CH3, and it is a dimer. Fc of the present invention may comprise a naturally occurring (wild type) sequence or it may comprise a sequence derived from the naturally occurring sequence by mutation (referred to as "mutant Fc"). In the polyspecific molecule and the bispecific molecule of the present invention, a Fc region is preferably mutant Fc, and more preferably a combination of Fc regions capable of forming a heterodimer. An example of a combination of Fc regions is a combination of Fc (i) in the first polypeptide and Fc (ii) in the second polypeptide described below. A combination is not limited thereto, provided that such combination of Fc regions is capable of aggregation (formation of a heterodimer).

Examples of mutant Fc include, but are not limited to, a modified Fc region comprised in a heteropolymer with improved stability (including a heterodimeric Fc region) disclosed in WO 2013/063702, Fc including an immunoglobulin CH3 region induced from the IgG antibody with a "knob" and a "hole" comprised in a heteropolymer disclosed in WO 1996/27011, Fc including a CH3 domain comprised in a heterodimer that becomes electrostatically advantageous by substitution of one or more amino acids with charged amino acids disclosed in WO 2009/089004, a heterodimeric Fc region comprised in a heterodimer involving steric mutation and/or pI (isoelectric point) mutation disclosed in WO 2014/110601, and a heterodimeric Fc region including a CH3 domain with a modification to eliminate or reduce the binding to protein A disclosed in WO 2010/151792.

A variable region is composed of a region with an extreme variability referred to as a hypervariable region (HVR) and relatively invariable regions referred to as framework regions (FRs) divided by the HVR. Naturally occurring heavy chain and light chain variable regions comprise 4 FRs connected by 3 hypervariable regions, a hypervariable region of each chain and a hypervariable region of other chains are maintained very close thereto by FRs, and such regions contribute to formation of an antigen-binding site of an antibody.

A heavy chain and a light chain of an antibody molecule are known to comprise 3 complementarity determining regions (CDRs). A complementarity determining region is also referred to as a hypervariable region, it is present within variable regions of a heavy chain and a light chain of the antibody where variability of a primary structure is particularly high, and, in general, it is separated in 3 positions in a primary structure of a polypeptide chain of a heavy chain and a light chain. In the present invention, complementarity determining regions of a heavy chain of an antibody are denoted as CDRH1, CDRH2, and CDRH3 from the amino terminus of the heavy chain amino acid sequence, and complementarity determining regions of a light chain are denoted as CDRL1, CDRL2, and CDRL3 from the amino terminus of the light chain amino acid sequence. These regions are adjacent to each other sterically and determine specificity to the antigens to which they bind.

In the present invention, the position and the length of CDR were determined in accordance with the definition of IMGT (Developmental and Comparative Immunology 27, 2003, 55-77).

FR is a variable region other than CDR. In general, a variable region comprises 4 FRs; i.e., FR1, FR2, FR3, and FR4.

CDRs and FRs comprised in the heavy chain and in the light chain are provided in the orders of FRH1-CDRH1-FRH2-CDRH2-FRH3-CDRH3-FRH4 and FRL1-CDRL1-FRL2-CDRL2-FRL3-CDRL3-FRL4, respectively, from the amino terminus toward the carboxyl terminus.

CDR and FR positions can be determined in accordance with various definitions well known in the art, such as the definitions of Kabat, Chothia, AbM, contact, in addition to IMGT.

In the present invention, a "site" to which an antibody binds; i.e., a "site" that is recognized by an antibody, is a partial peptide or a partial higher-order structure of an antigen to which an antibody binds or which is recognized by the antibody.

In the present invention, such site is referred to as an epitope or an antibody binding site. In the present invention, a "mutant antibody" refers to a polypeptide having an amino acid sequence derived from the amino acid sequence of the original antibody by substitution, deletion, or addition ("addition" encompasses "insertion") (hereafter, collectively referred to as "mutation") of amino acids and binding to the target antigen of the present invention. The number of mutant amino acids in such mutant antibody is 1, 2, 3, 4, 5, 6, 7, 8, 9, 10, 12, 15, 20, 25, 30, 40, or 50. Such mutant antibody is within the scope of the "antibody" of the present invention.

In the present invention, the term "several" in "one or several" indicates 2 to 10.

The term "molecule" used herein indicates a molecule comprising the antibody or the antigen-binding fragment of the antibody described above. In addition, the term "molecule" encompasses a polyspecific molecule formed of an antibody or a plurality of antigen-binding fragments derived therefrom.

In the present invention, the phrase "A has B" indicates that "A comprises B" or "B is bound, added, or fused to A." For example, "an antibody having a substrate" can be understood as "an antibody comprising a substrate," "an antibody to which a substrate is bound," "an antibody to which a substrate is added," or "an antibody to which a substrate is fused."

### 2. Molecule that binds to target antigen

The present invention relates to a molecule that binds to a target antigen, which binds specifically to the target antigen in a particular environment.

The term "particular environment" refers to an environment in particular tissue or a cell organelle. Examples thereof include a cancer microenvironment, the environment in the endosome, and the environment in the lysosome.

The term "cancer microenvironment" refers to an environment in cancer tissue, and examples thereof include the acidic pH environment and the environment in which a protease is present. While a pH of healthy tissue is neutral, such as a pH of 7.0 to 7.5, a pH of cancer tissue is 7.0 or lower, preferably 6.5 or lower, and more preferably 6.0 or lower.

The molecule that binds to a target antigen according to the present invention acquires responsiveness in a particular environment. Accordingly, it may be referred to as an "environmentally-responsive masked antibody molecule." When a molecule is an antibody, it is referred to as an "environmentally-responsive masked antibody."

The molecule that binds to the target antigen of the present invention comprises a moiety binding to a target antigen (a moiety [a]), a first peptide recognizing the target antigen-binding site in the moiety [a] (a moiety [b]), and a second peptide comprising an amino acid sequence comprising amino acids that are converted from an uncharged state to a positively charged state in response to a pH change from neutral to acidic conditions at a pH 7.5 or lower (a moiety [c]). It is preferable that these moieties be directly or indirectly connected to each other. When these moieties are "indirectly connected," these moieties are connected to each other via a linker or the like. A moiety binding to a target antigen is preferably a polypeptide. Such moiety binds to a target antigen by the antibody-antigen reaction or the protein (e.g., receptor)-ligand binding. A moiety binding to a target antigen is more preferably an antibody binding to a target antigen by the antibody-antigen reaction or an antigen-binding fragment of an antibody. The first peptide binds to a target antigen-binding site in the moiety binding to the target antigen and masks the target antigen-binding site. As a result, it is impossible or difficult that the target antigen-binding site binds to the target antigen.

While the number of the moiety [a], that of the moiety [b], and that of the moiety [c] comprised in the target-antigen-binding molecule according to the present invention are not particularly limited, preferably, the molecule comprise one or more than two of the moiety [a], the moiety [b], and the moiety [c], it is more preferable that the molecule comprise one or more than two of the moiety [a], the moiety [b], and the moiety [c] and that the number of the moiety [a], that of the moiety [b], and that of the moiety [c] be identical to each other, and it is further preferable that the molecule comprise 2 moieties [a], 2 moieties [b], and 2 moieties [c]. Figure 1 shows a concept of the present invention, which is an example of a format of a masked antibody that is activated in response to pH changes, although the present invention is not limited thereto. In the molecule, a moiety binding to a target antigen is a divalent antibody (IgG), and the second peptide comprises a cluster of pH-responsive histidine residues (the histidine (His) cluster, closed black square in Figure 1), and the first peptide indicated with lattice in Figure 1 binds to and masks the antigen-binding site of the antibody. The second peptide connects the first peptide to the moiety binding to the target antigen and shows pH-responsiveness. Accordingly, the second peptide is also referred to as a "pH-responsive linker." The first peptide can recognize and bind to the target antigen-binding site. When the second peptide is cleaved and the first peptide is present by itself, however, the first peptide binds and dissociates depending on physicochemical conditions. Thus, the first peptide may not be able to continually bind to the target antigen-binding site. In the target-antigen-binding molecule according to the present invention, for example, the first peptide binds to the moiety binding to a target antigen via the second peptide. Accordingly, a molecule binding to a target antigen may have a conformation in which the first peptide is positioned in the vicinity of the target antigen-binding site in the moiety binding to a target antigen and a majority of the target antigen-binding site of the first peptide would be masked under equilibrium conditions, unless the molecular conformation is changed (Figure 1, left). It should be noted that the mechanism is not limited thereto. Under acidic pH conditions, histidine residues in the His cluster of the second peptide are positively charged, a structural change is induced in the second peptide, which is a linker, by the repulsive force between histidine residues, the first peptide is dissociated from a molecule binding to a target antigen, and the first peptide cannot keep binding to the target antigen-binding site (Figure 1, right, "+" indicates positive charge). As a result, the target antigen-binding site comprised in the moiety binding to a target antigen can bind to the target antigen, and the target antigen-binding site has higher binding affinity to the target antigen than the binding affinity before structural change is induced in the second peptide. Specifically, the target-antigen-binding molecule according to the present invention can bind to the target antigen with higher affinity under acidic pH conditions than under neutral pH conditions. A structural change in the second peptide caused by the repulsive force between histidine residues is a reversible phenomenon caused by pH changes. When a molecule binding to a target antigen is transferred from acidic pH conditions to neutral pH conditions, accordingly, a structural change opposite from the structural change caused upon transfer from neutral pH conditions to acidic pH conditions is induced, and the first peptide binds to the target antigen-binding site. Thus, affinity of a target-antigen-binding molecule to the target antigen may be lowered. While the target-antigen-binding molecule according to the present invention may be able to exert its effects primarily by the mechanism described above, the mechanism is not limited thereto, and the molecule may exert its effects with the aid of or in combination with other mechanisms.

The expression "a target-antigen-binding molecule binds to the target antigen with higher affinity under acidic pH conditions than under neutral pH conditions" used herein may be expressed as follows. That is, affinity of a target-antigen-binding molecule to the target antigen under neutral pH conditions is adjusted to be lower than affinity thereof to the target antigen under acidic pH conditions. In the present invention, specifically, a difference between affinity of a target-antigen-binding molecule to the target antigen under acidic pH conditions and affinity thereof to the target antigen under neutral pH conditions may be increased (e.g., the EC₅₀ ratio (neutral pH conditions/acidic pH conditions) may be increased as described below). In order to increase a difference between affinity of a target-antigen-binding molecule to the target antigen under acidic pH conditions and affinity thereof to the target antigen under neutral pH conditions, for example, affinity to the target antigen under acidic pH conditions may be increased, affinity to the target antigen under neutral pH conditions may be increased, or both thereof may be increased.

In the present invention, a cluster of basic amino acids is introduced into the second peptide, so as to impart pH responsiveness to the linker portion. While the masked peptide portion (the first peptide in the present invention) may be provided with pH responsiveness, such constitution necessitates pH-responsive peptides of different sequences for each moiety binding to a target antigen. In contrast, a linker portion having a common sequence motif (a cluster of basic amino acids) of the second peptide according to the constitution of the present invention can be generally used for moieties binding to different target antigens. Such constitution is advantageous because it eliminates the need for a pH responsive peptide of a different sequence for each of the moieties binding to different target antigens.

A target antigen-binding molecule binds to the target antigen with higher affinity under acidic pH conditions than under neutral pH conditions, so as to prevent the target-antigen-binding molecule from binding to the antigen in healthy tissue and lower the toxicity. In order to "lower the toxicity," unwanted effects other than the drug efficacy of interest would be reduced when the target-antigen-binding molecule is administered to animals, such as humans, mice, rats, monkeys, rabbits, or dogs. Examples of toxicity include death, near-death, weight loss, and organ toxicity (e.g., skin and liver) after drug administration. Whether or not "toxicity is lowered" can be determined by methods known in the art, such as measurement of body weight of the subject or verification of the pathological data after administration of the target-antigen-binding molecule.

The target-antigen-binding molecule binds to the target antigen with higher affinity under acidic pH conditions than under neutral pH conditions. This is deduced to suppress binding thereof to the antigen expressed in healthy tissue and increase the blood concentration thereof. In order to "increase the blood concentration" of the target-antigen-binding molecule upon administration thereof to animals, such as humans, mice, rats, monkeys, rabbits, or dogs, the maximum blood concentration (Cmax) thereof is to be increased and the period until the target-antigen-binding molecule disappears from the blood is to be prolonged. In the target-antigen-binding molecule in the blood, a moiety binding to a target antigen may or may not be masked, provided that the target-antigen-binding molecule can function in a particular environment. Whether or not "the blood concentration is increased" can be determined on the basis of the results as to, for example, whether or not the maximum blood concentration (Cmax) thereof is increased or whether or not the period until the target-antigen-binding molecule disappears from the blood is prolonged after the target-antigen-binding molecule is administered. Such determination can be performed by measuring the blood drug concentration or parameters, such as the plasma half life, the mean residence time in the plasma, or the plasma clearance of the target-antigen-binding molecule, after administration of the target-antigen-binding molecule by methods known in the art.

In the present invention, a difference between affinity to the target antigen under acidic pH conditions and affinity to the target antigen under neutral pH conditions is not particularly limited, provided that affinity to the target antigen under acidic pH conditions is higher than affinity to the target antigen under neutral pH conditions. In an embodiment, for example, a larger EC₅₀ ratio (neutral pH conditions/acidic pH conditions) is preferable. The term "EC₅₀" used herein refers to the 50% effective concentration or the half-maximal effective concentration, and the term refers to the concentration of a molecule, such as a drug or antibody, which induces a response halfway between the minimum and the maximum. The EC₅₀ ratio (neutral pH conditions/acidic pH conditions) is a ratio of EC₅₀ of the target-antigen-binding molecule under neutral pH conditions to EC₅₀ of the target-antigen-binding molecule under acidic pH conditions. Accordingly, a larger EC₅₀ ratio (neutral pH conditions/acidic pH conditions) of the target-antigen-binding molecule; i.e., a smaller EC₅₀ under acidic pH conditions than EC₅₀ under neutral pH conditions, indicates higher affinity of the molecule to the target antigen under acidic pH conditions than under neutral pH conditions. A smaller EC₅₀ ratio (neutral pH conditions/acidic pH conditions) of the target-antigen-binding molecule; i.e., a larger EC₅₀ under acidic pH conditions than EC₅₀ under neutral pH conditions, indicates lower affinity of the molecule to the target antigen under acidic pH conditions than under neutral pH conditions. The EC₅₀ ratio (neutral pH conditions/acidic pH conditions) of the target-antigen-binding molecule is preferably 3 or higher, more preferably 10 or higher, and further preferably 30 or higher. The upper limit of the EC₅₀ ratio (neutral pH conditions/acidic pH conditions) is not particularly limited, and it may be, for example, 100, 400, 1000, or 10000, provided that a person skilled in the art can prepare the target-antigen-binding molecule. Specifically, the EC₅₀ ratio (neutral pH conditions/acidic pH conditions) of the target-antigen-binding molecule is preferably 3 to 100, 3 to 400, 3 to 1000, or 3 to 10000, more preferably 10 to 100, 10 to 400, 10 to 1000, or 10 to 10000, and further preferably 30 to 100, 30 to 400, 30 to 1000, or 30 to 10000.

In the case of a soluble antigen, KD (dissociation constant) may be used as a value indicating affinity to a target antigen. In the case of a membrane-type antigen, an apparent dissociation constant (KD) may be used. KD (dissociation constant) and apparent KD (apparent dissociation constant) may be measured by a method known to a person skilled in the art. For example, Biacore (GE healthcare), ELISA (enzyme linked immunosorbent assay), or FACS can be employed.

### Antibody

Examples of the antibodies of the present invention include an antibody derived from a non-human animal (a non-human animal antibody), a human antibody, a chimerized antibody (also referred to as a "chimeric antibody"), and a humanized antibody, with the human antibody or the humanized antibody being preferable. The antibody of the present invention encompasses a mutant of an antibody (the "mutant antibody" described below). For example, the human antibody encompasses a human mutant antibody and the humanized antibody encompasses a humanized mutant antibody.

Examples of non-human animal antibodies include antibodies derived from vertebrates, such as mammalians and birds. Examples of mammalian-derived antibodies include antibodies derived from rodents, such as mouse antibody and rat antibody, and antibodies derived from camels. An example of a bird-derived antibody is a chicken antibody.

Examples of chimeric antibodies include, but are not limited to, antibodies comprising a variable region derived from a non-human animal antibody bound to a constant region derived from a human antibody (human immunoglobulin).

Examples of humanized antibodies include, but are not limited to, a humanized antibody prepared by transplanting CDR in a variable region of a non-human animal antibody into a human antibody (a variable region of human immunoglobulin), a humanized antibody prepared by transplanting, in addition to CDR, a part of a sequence of a framework region of a non-human animal antibody into a human antibody, and a humanized antibody prepared by substituting 1 or more than two amino acids derived from a non-human animal antibody with amino acids derived from a human antibody.

An antibody can be prepared by a variety of known techniques. For example, an antibody can be prepared by a method involving the use of a hybridoma, cell-mediated immunity, or genetic recombination. Also, a phage-display-derived human antibody selected from a human antibody library can be obtained. In a phage display method, for example, a human antibody variable region may be expressed as scFv on a phage surface, and an antigen-binding phage may then be selected. The gene of the phage selected upon its binding to the antigen may be analyzed, so that a DNA sequence encoding a human antibody variable region binding to the antigen can be determined. If a DNA sequence of the antigen-binding scFv is elucidated, an expression vector comprising such sequence may be prepared, introduced into an adequate host cell, and expressed therein. Thus, a human antibody can be obtained (WO 1992/01047, WO 1992/20791, WO 1993/06213, WO 1993/11236, WO 1993/19172, WO 1995/01438, WO 1995/15388, Annu. Rev. Immunol., 1994, 12, 433-455). A human antibody can be obtained by the method involving the use of a human antibody-producing mouse having a human genome DNA fragment comprising the human antibody heavy chain and light chain genes (see, for example, Tomizuka, K. et al., Nature Genetics, 1997, 16, pp. 133-143; Kuroiwa, Y. et. al., Nuc. Acids Res., 1998, 26, pp. 3447-3448; Yoshida, H. et.al., Animal Cell Technology: Basic and Applied Aspects vol. 10, pp. 69-73 (Kitagawa, Y., Matuda, T. and Iijima, S. eds.), Kluwer Academic Publishers, 1999; Tomizuka, K. et. al., Proc. Natl. Acad. Sci., U.S.A., 2000, 97, pp. 722-727), although the method is not limited thereto. As constant regions of an antibody used for the treatment or prevention of human diseases, constant regions of a human antibody are preferably used. Examples of heavy chain constant regions of a human antibody include Cγ1, Cγ2, Cγ3, Cγ4, Cµ, Cδ, Cα1, Cα2, and Cε. Examples of light chain constant regions of a human antibody include Cκ and Cλ.

The term "an antigen-binding fragment of an antibody" refers to a partial fragment of an antibody having activity of binding to an antigen, which is composed of a heavy chain variable region and a light chain variable region. Examples of an antigen-binding fragments of an antibody include, but are not limited to, antigen-binding fragments, such as Fab, F(ab')₂, scFv, Fab', Fv, and single-domain antibody (sdAb). Such antigen-binding fragments of the antibody may be obtained by treating a full-length molecule of an antibody protein with an enzyme such as papain or pepsin, or it may be a recombinant protein produced in an adequate host cell with the use of a recombinant gene.

### Mutant of antibody or binding fragment thereof

A mutant of the antibody according to the present invention or an antigen-binding fragment thereof can be preferably provided with, for example, lowered susceptibility to protein degradation or oxidation, maintained or improved biological activity or functions, suppression of lowering or change in such activity or functions, improved or regulated antigen-binding ability, physicochemical properties, or functional properties. A protein is known to change its functions or activity upon alternation of a particular amino acid side chain on its surface, and examples include deamidation of an asparagine side chain and isomerization of an aspartic acid side chain. An antibody resulting from substitution of a particular amino acid with another amino acid so as to prevent the amino acid side chain from changing is within the scope of the mutant antibody of the present invention.

Examples of the mutant antibody of the present invention include an antibody comprising an amino acid sequence derived from the amino acid sequence of the original antibody or its antigen-binding fragment by conservative amino acid substitution and an antigen-binding fragment thereof. Conservative amino acid substitution occurs within a group of amino acids with similar amino acid side chains.

Preferable amino acid groups are as follows: the acidic group: aspartic acid and glutamic acid; the basic group: lysine, arginine, and histidine; the non-polar group: alanine, valine, leucine, isoleucine, proline, phenylalanine, methionine, and tryptophan; and the uncharged polar family: glycine, asparagine, glutamine, cysteine, serine, threonine, and tyrosine. Other preferable amino acid groups are as follows: the aliphatic hydroxy group: serine and threonine; the amide-containing group: asparagine and glutamine; the aliphatic group: alanine, valine, leucine, and isoleucine; and the aromatic group: phenylalanine, tryptophan, and tyrosine. In such mutant antibody, amino acid substitution is preferably carried out by refraining from lowering the antigen-binding intensity of the original antibody. Among the amino acids indicated above, the negative logarithms of the dissociation constants of groups other than the α-amino group and the α-carboxy group in amino acids having an acidic or basic functional group in side chains are known as follows: arginine: 12.48; aspartic acid: 3.65; cysteine: 8.18; glutamic acid: 4.25; histidine: 6.00; lysine: 10.53; and tyrosine: 10.07 (D. R. Lide, Handbook of Chemistry and Physics,72nd Edition, CRC Press, Boca Raton, FL, 1991).

### Modified antibody, modified binding fragment thereof, or complex thereof

The present invention provides a modified antibody or a modified binding fragment thereof. The modified antibody according to the present invention or the modified binding fragment thereof has been subjected to chemical or biological modification. Examples of chemical modification include a bond of a chemical portion to the amino acid skeleton and chemical modification of N-bound or O-bound carbohydrate chains. Examples of biological modification include post-translational modification (e.g., glycan addition to an N-bond or O-bond, remodeling of glycans, processing of the amino terminal or carboxyl terminal region, deamidation, aspartic acid isomerization, and methionine oxidation), and methionine addition to the amino terminus by expression in a prokaryotic host cell. Also, labels that enable detection or isolation of the antibody or antigen according to the present invention, such as an enzyme label, a fluorescence label, and an affinity label, are within the scope of the modified antibody or antigen as described above. The modified antibody according to the present invention or the binding fragment thereof as described above is useful for improvement of stability and retentivity in blood of the original antibody according to the present invention or the binding fragment thereof, reduction of the antigenicity, detection or isolation of the antibody or antigen, and other purposes.

Examples of chemical portions comprised in the chemically modified antibody or binding fragment thereof include water-soluble polymers, such as polyethylene glycol (PEG), ethylene glycol/propylene glycol polymer, carboxymethyl cellulose, dextran, and polyvinyl alcohol.

Examples of biologically modified antibody or binding fragment thereof include a modified antibody or binding fragment thereof prepared by enzyme treatment or cell treatment, a fused antibody or binding fragment thereof comprising a tag or other peptide added thereto by gene recombination, and an antibody or binding fragment thereof prepared with the use of a host cell expressing an endogenous or exogenous glycan modifying enzyme.

Such modification may be provided at any desired position in the antibody or the binding fragment thereof, and the same or two or more different types of modification may be provided at one or more positions.

However, deletion of such heavy chain sequence or modification of a heavy chain or light chain sequence has a little effect on the antigen-binding ability and effector functions of the antibody (e.g., complement activation or antibody-dependent cytotoxicity), and such effect is preferably insignificant.

Accordingly, the present invention encompasses the antibody subjected to such deletion or modification. Examples include a deletion mutant lacking 1 or 2 amino acids from the heavy chain carboxyl terminus (Journal of Chromatography A; 705; 129-134, 1995), a deletion mutant lacking 2 amino acids (glycine and lysine) from the heavy chain carboxyl terminus and additionally subjected to amidation of proline at the carboxyl terminus (Analytical Biochemistry, 360: 75-83, 2007), and an antibody resulting from pyroglutamilation of an amino-terminal glutamine or glutamic acid of the antibody heavy chain or light chain (WO 2013/147153) (they are collectively referred to as "deletion mutants"). As long as the antigen-binding ability and effector functions are retained, the antibody of the present invention lacking the heavy chain and light chain carboxyl termini is not limited to the deletion mutants described above. When the antibody of the present invention comprises 2 or more chains (e.g., heavy chains), such 2 or more chains (e.g., heavy chains) may be either or both of the full-length heavy chain or a heavy chain selected from the group consisting of the deletion mutants described above. While the quantitative ratio or the number ratio of molecules of the deletion mutant would be influenced by the type and culture conditions of cultured cells of mammalian animals producing the antibody of the present invention, main components of the antibody of the present invention can be either one or both of the 2 heavy chains lacking 1, 2, or several amino acids from the carboxyl terminus.

In addition, the antibody of the present invention or an antigen-binding fragment thereof (e.g., those comprised in the molecule, the polyspecific molecule, and the bispecific molecule of the present invention) comprising one to several amino acids derived from the expression vector and/or signal sequence added to the amino terminus and/or carboxy terminus (and partially or entirely modified as described above) are within the scope of the modified antibody of the present invention or the modified antigen-binding fragment thereof, as long as the antigen-binding intensity of interest is maintained. A molecule comprising such modified antibody or modified antigen-binding fragment thereof is within the scope of the molecule of the present invention.

In the present invention, the "the antibody or the binding fragment thereof" encompasses "the modified antibody or the modified antigen-binding fragment thereof." In addition, the "the antibody or antigen-binding fragment thereof" comprised in the molecule, the polyspecific molecule, and the bispecific molecule of the present invention encompasses "the modified antibody or the modified antigen-binding fragment thereof."

Antibody dependent cellular cytotoxicity can be potentiated by regulation (glycosylation, fucose removal, and the like) of modification of a glycan bound to the antibody of the present invention. Known techniques for regulation of antibody glycan modification are disclosed in, for example, WO 1999/54342, WO 2000/61739, WO 2002/31140, and WO 2007/133855, although techniques are not limited thereto.

### Moiety binding to target antigen: moiety [a]

A target antigen is a molecule that is associated with a particular disease. When a subject is afflicted with a disease of an antigen or molecule causing a particular disease, a molecule that is associated with the particular disease is expressed or shows enhanced expression in a diseased cell that develops in the case of such disease. When the moiety binding to a target antigen binds to the molecule, the molecule can attack the diseased cell, relieve the disease symptom, or treat the disease. The diseased cell is preferably a tumor cell or a stromal cell. In the tumor cell, a target antigen is a tumor antigen. In the stromal cell, a target antigen is a molecule that is expressed in the stromal cell. The stromal cell interacts with the tumor cell and plays a key role in cancer growth and progression. The tumor antigen is expressed in the tumor cell or a tumor cell resulting from canceration of the healthy cell. When the moiety binding to a target antigen binds to the tumor antigen, it inhibits tumor cell growth, damages the tumor cell, or kills the tumor cell (apoptosis or necrosis).

Examples of tumor antigens include CD98, TROP2, EGFR, GPRC5D, CD33, CD37, DR5, EPHA2, FGFR2, FGFR4, FOLR1, VEGF, CD20, CD22, CD70, PD-L1, CTLA-4, CD166, CD71, CD47, CDH6, CD147, Mesothelin, A33, CanAg, G250, MUC1, GPNMB, Integrin, Tenascin-C, CLDN6, DLL-3, and SLC44A4.

Examples of antibodies reacting with the tumor antigens include the anti-CD98 antibody (described in, for example, JP 2017-114763 A, WO 2007/114496, WO 2008/017828, WO 2009/043922, WO 2009/090553, JP 2012-092068 A, WO 2011/118804, and WO 2013/078377), the anti-TROP2 antibody (described in, for example, Linnenbach A. J. et al., Proc. Natl. Acad. Sci., vol. 86 (No. 1), pp. 27-31, 1989, WO 2008/144891, WO 2011/145744, WO 2011/155579, WO 2013/077458, WO 2003/074566, WO 2011/068845, WO 2013/068946, US 7999083, and WO 2015/098099), the anti-EGFR antibodies, such as panitumumab, nimotuzumab, cetuximab , ametumumab (SY-101), SYN-004, SCT-200, tomuzotuximab, GC-1118, GR-1401, depatuxizumab (ABT-806), Serclutamab, AMG595, and matuzumab, and the anti-GPRC5D antibodies (described in, for example, WO 2018/147245 and WO 2016/090329). The moiety binding to a target antigen according to the present invention may be derived from or comprise such antibodies.

An example of a molecule that is expressed in a stromal cell is a fibroblast activation protein (FAP).

A moiety binding to a target antigen preferably comprises an amino acid sequence that is not comprised in the first peptide or the second peptide, and such moiety is more preferably a polypeptide consisting of the aforementioned amino acid sequence.

A target antigen and a moiety binding to the target antigen may be an antigen and an antibody that binds to the antigen or an antigen-binding fragment of an antibody. The target antigen and the moiety binding to the target antigen are not limited to the combination indicated above, and examples thereof include a ligand and a receptor that binds to the ligand (or vice versa) and a cytokine and a receptor to which the cytokine binds (or vice versa). Examples of molecules other than antibodies include a non-immunoglobulin protein that binds to a target antigen, a nucleic acid molecule such as a nucleic acid aptamer, and a low-molecular-weight compound.

### First peptide: moiety [b]

A first peptide that recognizes a target antigen-binding site comprised in a moiety binding to the target antigen (a moiety [a]) recognizes the target antigen-binding site comprised in the moiety binding to the target antigen and masks the site. Thus, the first peptide disables, makes it difficult, or inhibits or impedes the moiety binding to a target antigen to bind to the target antigen. When a moiety binding to a target antigen is an antibody or an antigen-binding fragment of an antibody (hereafter referred to as "antibody or the like"), the first peptide binds to an antibody or the like at its antigen-binding region. When a peptide "recognizes" the target antigen-binding site, for example, the peptide "binds" to the target antigen-binding site, or the peptide "has affinity to" the target antigen-binding site. An antigen-binding region of an antibody or the like is present in a variable region of an antibody or the like, and, in particular, it is present in a complementarity determining region (CDR). Since an antibody or the like binds to an epitope (antigen determining group) of an antigen, the first peptide is a peptide that mimics an epitope. A peptide that mimics an epitope of an antigen is referred to as a "mimotope." In the present invention, the first peptide is preferably a mimotope that binds to CDR. A mimotope is a peptide consisting of 6 to 30, preferably 10 to 20, more preferably 13 to 17, and particularly preferably 15 amino acids. A mimotope can be prepared by, for example, preparing various types of display (e.g., phage display, ribosome display, nucleic acid display, or bacteria display) libraries consisting of the number of amino acids indicated above, screening the libraries by panning, selecting phage particles displaying peptides that recognize the target antigen-binding site comprised in a moiety binding to a target antigen, and obtaining DNA encoding the mimotope and a nucleotide sequence thereof from the phage particles. Whether or not a peptide of interest is a mimotope (i.e., a peptide binding to an antibody CDR) can be determined by, for example, crystallizing a masked antibody and subjecting the crystallized antibody to X-ray crystal structure analysis. If the peptide binding to an antibody (competitively) with the antigen is confirmed by SPR or other means, the results of observation strongly suggest that the peptide is a mimotope that binds to the antibody CDR (see Example 4).

As described above, a moiety binding to a target antigen may be a molecule other than an antibody or an antigen-binding fragment of an antibody (hereafter referred to as "antibody or the like"). When a target antigen is a ligand, an example of the first peptide other than the mimotope is a peptide that recognizes the ligand-binding site in a receptor to which the ligand binds. When a target antigen is a cytokine, an example of the first peptide is a peptide that recognizes the cytokine-binding site in a receptor to which the cytokine binds. When a moiety binding to a target antigen is a non-immunoglobulin protein, the first peptide is a peptide that recognizes the target antigen site in the protein. When a moiety binding to a target antigen is a nucleic acid molecule, the first peptide is a peptide that recognizes the target antigen site in the nucleic acid molecule. When a moiety binding to a target antigen is a low-molecular-weight compound, the first peptide is a peptide that recognizes the target antigen site in the low-molecular-weight compound.

### Second peptide: moiety [c]

The second peptide comprises amino acids that are converted from an uncharged state to a positively charged state in response to a pH change from neutral to acidic conditions at a pH 7.5 or lower. Under acidic pH conditions, the amino acids are positively charged, and a structural change is induced in a linker. At "a pH 7.5 or lower," a pH range may be any of a pH 1.0 to 7.5, a pH 2.0 to 7.5, a pH 3.0 to 7.5, a pH 4.0 to 7.5, a pH 5.0 to 7.5, a pH 6.0 to 7.5, or a pH 7.0 to 7.5. In addition, a "pH 7.5 or lower" may (or may not) include the entire pH range of 7.5 or lower, and an uncharged state is converted to a positively charged state in response to a pH change at a pH 7.5 or lower. When the amino acid charge state is converted in response to a pH change from neutral to acidic conditions within a pH range of 5.5 to 7.5, for example, a pH is definitely at 7.5 or lower. When the amino acid charge state is converted in response to a pH change from neutral to acidic conditions within a pH range of 4.5 to 6.5, in addition, a pH 4.5 to 6.5 is within a pH range of 7.5 or lower, and such pH range thus satisfies the condition of "a pH 7.5 or lower." Under neutral pH conditions, a pH is about 7, such as 7.0 to 7.5. Under acidic pH conditions, a pH is lower than 7, such as lower than 7.0, preferably 6.5 or lower, and more preferably 6.0 or lower. The lower limit of the acidic pH conditions is not particularly limited, and it may be any low value, such as 1.0, 2,0, or 3.0, provided that it is practicable in the art. Under acidic pH conditions, specifically, a pH is 1.0 to lower than 7.0, 2.0 to lower than 7.0, or 3.0 to lower than 7.0, preferably 1.0 to 6.5, 2.0 to 6.5, or 3.0 to 6.5, and more preferably 1.0 to 6.0, 2.0 to 6.0, or 3.0 to 6.0. In addition, conversion of the amino acid charge state is a reversible phenomenon caused in response to a pH change. Accordingly, amino acids that are converted from an uncharged state to a positively charged state in response to a pH change "from neutral to acidic conditions" at a pH 7.5 or lower are considered to undergo a change in the charged state in response to a pH change "from acidic conditions to neutral conditions" at a pH 7.5 or lower. The amino acids that are converted from an uncharged state to a positively charged state in response to a pH change from neutral to acidic conditions at a pH 7.5 or lower may be natural or unnatural amino acids. Unnatural amino acids can be adequately selected from among the unnatural amino acids described in, for example, WO 2006/132969, WO 2008/030612, WO 2008/030613, WO 2008/030614, WO 2010/037062, and WO 2018/223108 that have been disclosed when the present application was filed. The second peptide is also referred to as a "pH-responsive linker." The second peptide comprises, as amino acids that are converted from an uncharged state to a positively charged state in response to a pH change from neutral to acidic conditions at a pH 7.5 or lower, amino acids having basic functional groups with pKa of 7 or lower in side chains. pKa is a quantitative indicator of an acid level of an acidic compound or acidic functional group. pKa is represented by the negative common logarithm pKa (pKa = -log₁₀ Ka) of the equilibrium constant Ka for the dissociation reaction in which hydrogen ions are released. pKa is also referred to as an "acid dissociation constant," which is a value at 25°C in water, and it can be determined by the method described in Kagaku Binran (Chemistry Handbook): Basics (Revised 5th Edition), II-331 to II-343 (the Chemical Society of Japan (ed.), Maruzen Publishing Co., Ltd.). As pKa, known numerical values as described in literatures (e.g., Rika Nenpyou (Chronological Scientific Tables), 2013 (desk edition), Maruzen Publishing Co., Ltd.) can be used. pKa is also used as a quantitative indicator of a basicity level in a basic compound or a basic functional group. Specifically, a proton may be added to a basic compound or a basic functional group to prepare a conjugate acid, and a basicity level of the original basic compound can be determined based on the acid level of the conjugate acid.

Amino acids comprised in the second peptide are required to be positively charged under acidic pH conditions to serve as pH-responsive linkers. It is accordingly preferable that the second peptide do not comprise amino acids that are negatively charged under acidic pH conditions. The negatively charged amino acids under acidic pH conditions would neutralize or attenuate the positively charged amino acids under acidic pH conditions, and a structure change would not be induced in the linker. In the present invention, amino acids that are negatively charged under acidic pH conditions are not particularly limited. When the second peptide is composed of natural amino acids, examples of amino acids that are negatively charged under acidic pH conditions include glutamic acid and aspartic acid. Specifically, preferably, the second peptide do not comprise glutamic acid and/or aspartic acid.

The present invention can be described with the use of the "pI value" indicating the isoelectric point of the second peptide. The term "pI value" (it may be abbreviated to as "pH(I)" or the "IEP" value) of a molecule used herein refers to the isoelectric point of a molecule, and, in particular, the isoelectric point of an amino acid, peptide, or protein, which is a pH value at which a particular molecule carries no electrical charge. As the pI value of a molecule is increased; i.e., as the pH value at which a particular molecule carries no electrical charge is increased (basic side), the molecule is likely to be positively charged under acidic conditions. As the pI value of a molecule is decreased; i.e., as the pH value at which a particular molecule carries no electrical charge is decreased (acidic side), the molecule is less likely to be positively charged under acidic conditions. Since an experimental pI value may be slightly different from a calculated pI value, the term "pI value" used herein refers to the calculated pI value. The pI value can be determined based on, for example, pKa of amino acids constituting the peptide to be calculated and pKa of the amino terminal amino groups and the carboxyl terminal carboxyl groups.

Amino acids comprised in the second peptide are required to be positively charged under acidic pH conditions to serve as pH-responsive linkers. Accordingly, the pI value is not particularly limited, provided that the calculated charge is positive under acidic conditions and it is larger than the calculated charge under neutral conditions. In order to induce a structure change in a linker, for example, the calculated charge of the second peptide under acidic conditions at a pH 3.0 to 6.5 needs to be larger than the calculated charge of the second peptide under neutral conditions at a pH 7.0 to 7.5. Accordingly, the pI value of the second peptide is preferably 6.4 or higher, more preferably 6.8 or higher, further preferably 7.2 or higher, and most preferably 7.6 or higher. The upper limit of the pI value is not particularly limited, and it may be any value, such as 12, 13, or 14, provided that it is practicable in the art. Specifically, the pI value of the second peptide is preferably 6.4 to 12, 6.4 to 13, or 6.4 to 14, more preferably 6.8 to 12, 6.8 to 13, or 6.8 to 14, further preferably 7.2 to 12, 7.2 to 13, or 7.2 to 14, and most preferably 7.6 to 12, 7.6 to 13, or 7.6 to 14.

For example, Table 1 shows the pI values concerning the second peptide used in the examples of the present invention calculated in accordance with A. Sillero and J. M. Ribeiro, Analytical Biochem., 179 (2), 1989, 319-325. In Table 1, "pH responsiveness" indicates the occurrence of a structural change under acidic conditions. In Table 1, "pH responsiveness" is indicated as "responsive," "weak," or "not responsive." A change in the binding intensity is visually inspected by ELISA in the manner described in Example 5 below. pH responsiveness is evaluated to be "responsive" when a change is clearly observed in the binding intensity, it is evaluated to be "not responsive" when no change is observed in the binding intensity, and it is evaluated to be "weak" when a slight change is observed.

**[Table 1]**

| Name | pH responsiveness | Amino acid sequence | SEQ ID NO: | Number of residues | pI |
|---|---|---|---|---|---|
| MHT1007 | Not responsive | GSGGGGSVLVPMAMMASGGS | SEQ ID NO: 46 | 20 | 5.7 |
| MHT1008 | Not responsive | GSGGGGSGGGGSGGGGSGGS | SEQ ID NO: 47 | 20 | 5.7 |
| MHT1009 | Not responsive | GSGGSGGSGRSANAILEGGS | SEQ ID NO: 48 | 20 | 6.36 |
| MHT1803 | Responsive | HHHSGGGGSGGGGSGGGGSHHH | SEQ ID NO: 49 | 22 | 1.74 |
| MHT1804 | Responsive | GSGHHHSGGGGSHHHGSGGS | SEQ ID NO: 50 | 20 | 7.74 |
| MHT1805 | Responsive | GSGGGGSHHHHSGGGGSGGS | SEO ID NO: 51 | 20 | 7.64 |
| MHT1806 | Responsive | HHHSGGGGSHHHHSGGGGSHHH | SEQ ID NO: 52 | 22 | 7.88 |
| MHT1808 | Responsive | GGGGSGHHHGGHHHGGHHHGGS | SEQ ID NO: 53 | 22 | 7.85 |
| MHT1809 | Responsive | GGGGSHHGGHHGGHHGGHHGGS | SEO ID NO: 42 | 22 | 7.82 |
| MHT1810 | Responsive | GGGGHGHGHGHGHGHGHGHGGS | SEQ ID NO: 54 | 22 | 7.82 |
| MHT1811 | Responsive | GGGGSGGGSGHHHGGHHHGGHH HGSPLGLWAGGS | SEQ ID NO: 55 | 34 | 7.85 |
| MHT1817 | Weak | GGGGSGHHHGGHDHGGHHHGGS | SEQ ID NO: 56 | 22 | 7.2 |
| MHT1818 | Weak | GGGGSGHDHGGHHHGGHDHGGS | SEQ ID NO: 57 | 22 | 6.79 |
| MHT1819 | Not responsive | GGGGSGHDHGGHDHGGHDHGGS | SEQ ID NO: 58 | 22 | 6.4 |
| MhM8001 | Responsive | GGGGSGHHHGGHHHGGHHHGGS | SEQ ID NO: 59 | 22 | 7.85 |
| MCE-2101 | Responsive | GGGGSGHHHGGHHHGGHHHGGS | SEO ID NO: 60 | 22 | 7.85 |
| MC3-9001 | Responsive | GGGGSGHHHGGHHHGGHHHGGS | SEQ ID NO: 61 | 22 | 7.85 |
| MHT1221 | Not responsive | GSGGGGSGGGGSGGGGSGGS | SEO ID NO: 62 | 20 | 5.7 |
| MhM1013 | Not responsive | GSGSGGGGSVLVPMAMMASGGS | SEQ ID NO: 63 | 22 | 5.7 |
| MhM1018-M1 | Not responsive | GSGSGGGGSGGGGSGGGGSGGS | SEQ ID NO: 64 | 22 | 5.7 |
| MhM1024-M1 | Responsive | GSGGGGSGHHHGGHHHGGHHHGGS | SEQ ID NO: 65 | 24 | 7.85 |
| MHT1814 | Responsive | GGGGSGHHHGGHRHGGHHHGGS | SEQ ID NO: 66 | 22 | 10.13 |
| MhM1026-M1 | Responsive | GGGGSGGGHHHGGHHHGGHHHGGS | SEQ ID NO: 75 | 24 | 7.85 |
| MhM1028-M1 | Responsive | GGGGSGGGHHHGRHHHGGHHHGGS | SEQ ID NO: 76 | 24 | 10.13 |
| MhM1042-M1 | Responsive | GGGGSGGRHHHGGHHHGGHHHGGS | SEQ ID NO: 77 | 24 | 10.13 |
| MhM1043-M1 | Responsive | GGGGSGGGHHHGGHHHGRHHHGGS | SEQ ID NO: 78 | 24 | 10.13 |
| MhM1044-M1 | Responsive | GGGGSGGGHHHGKHHHGGHHHGGS | SEQ ID NO: 79 | 24 | 9.33 |
| MhM1045-M1 | Responsive | GGGGSGGKHHHGGHHHGGHHHGGS | SEO ID NO: 80 | 24 | 9.33 |
| MhM1046-M1 | Responsive | GGGGSGGGHHHGGHHHGKHHHGGS | SEO ID NO: 81 | 24 | 9.33 |

The second peptide preferably consists of an amino acid sequence having the configurations (1) and (2) below:
(1) at least 1 of (a) an amino acid sequence consisting of 2 or more consecutive amino acids having basic functional groups with pKa of 7 or lower in side chains or (b) an amino acid sequence derived from the amino acid sequence (a) by insertion or addition of an arginine or lysine residue at any site (including an amino or carboxyl terminus); and
(2) an amino acid sequence comprising 4 or more of the amino acids having basic functional groups with pKa of 7 or lower in side chains.

The "the amino acid sequence (a)" or "the amino acid sequence (b)" is referred to as a "cluster of basic amino acids." The amino acid sequence consisting of 2 or more consecutive amino acids having basic functional groups with pKa of 7 or lower in side chains is preferably an amino acid sequence consisting of 2 to 4 consecutive amino acids having basic functional groups with pKa of 7 or lower in side chains, and more preferably an amino acid sequence consisting of 2 or 3 consecutive amino acids having basic functional groups with pKa of 7 or lower in side chains. The upper limit of the number of "amino acids having basic functional groups with pKa of 7 or lower in side chains" is not limited, and it is preferably 20, more preferably 15, and further preferably 12.

In the present invention, as described above, the "cluster of basic amino acids" refers to an amino acid sequence consisting of 2 or more consecutive amino acids having basic functional groups with pKa of 7 or lower in side chains or an amino acid sequence derived from the aforementioned amino acid sequence by insertion or addition of an arginine or lysine residue at any site (including an amino or carboxyl terminus). When a particular amino acid is selected as an amino acid having basic functional groups with pKa of 7 or lower in side chains, a type of the amino acid may be denoted by the addition of the term "cluster." When histidine is selected as an amino acid having basic functional groups with pKa of 7 or lower in a side chain, for example, an amino acid sequence consisting of two or more consecutive histidine residues and an amino acid sequence derived therefrom by insertion or addition of an arginine or lysine residue at any site (including an amino or carboxyl terminus) are each referred to as a "histidine (His) cluster."

In the second peptide, amino acids other than the cluster of basic amino acids are not particularly limited, provided that such amino acids are other than an amino acid having basic functional groups with pKa of 7 or lower in side chains, arginine, lysine, and an amino acid that is negatively charged under acidic pH conditions. Examples of such amino acids include natural amino acids, such as glycine, serine, alanine, proline, and threonine. A preferable amino acid sequence comprises glycine and/or serine (so-called GS linker) or constitutes a protease substrate sequence. Such amino acids may be comprised in either or both of the amino terminal side and the carboxyl terminal side of the cluster of basic amino acids, and the number of such amino acids may be 1 to 30, preferably 1 to 20, and more preferably 1 to 10.

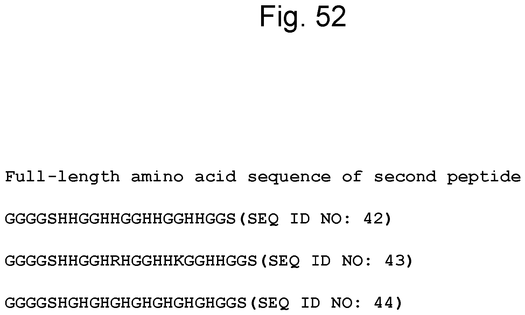

The amino acid having basic functional groups with pKa of 7 or lower in a side chain among natural amino acids is histidine only. When the number of the clusters of basic amino acids in the second peptide composed of natural amino acids is to be counted, the number of the histidine clusters may be counted. When the second peptide consists of the amino acid sequence: GGGGSHHGGHHGGHHGGHHGGS (SEQ ID NO: 42, Figure 52), for example, it comprises 4 amino acid sequences each consisting of 2 consecutive histidine residues. Thus, the second peptide can be determined to comprise 4 clusters of basic amino acids. When the second peptide consists of the amino acid sequence: GGGGSHHGGHRHGGHHKGGHHGGS (SEQ ID NO: 43, Figure 52), alternatively, the second peptide comprises 2 amino acid sequences each consisting of 2 consecutive histidine residues, an amino acid sequence comprising arginine inserted into a site between 2 consecutive histidine residues, and an amino acid sequence comprising lysine added to the amino acid sequence consisting of 2 consecutive histidine residues. Thus, the second peptide can be determined to comprise 4 clusters of basic amino acids. Examples of amino acid sequences derived from the amino acid sequence consisting of 2 or more consecutive histidine residues by insertion or addition of an arginine or lysine residue at any site (including an amino or carboxyl terminus) include HRH, RHH, HHR, HRHH, HHRH, RHHH, HHHR, HKH, KHH, HHK, HKHH, HHKH, KHHH, and HHHK. Such amino acid sequences are examples when an amino acid having basic functional groups with pKa of 7 or lower in a side chain is histidine, and an amino acid represented by H may be an amino acid having basic functional groups with pKa of 7 or lower in a side chain other than histidine.

In order to enhance affinity of the target-antigen-binding molecule to the target antigen at an acidic pH, the cluster of basic amino acids in the second peptide comprises preferably an amino acid sequence derived from the amino acid sequence consisting of 2 or more consecutive amino acids having basic functional groups with pKa of 7 or lower in side chains by insertion or addition of 1 or more arginine residues at any site (including an amino or carboxyl terminus), more preferably an amino acid sequence derived from the amino acid sequence consisting of 2 or more consecutive amino acids having basic functional groups with pKa of 7 or lower in side chains by insertion or addition of an arginine residue at any site (including an amino or carboxyl terminus), and further preferably a cluster of basic amino acids having an amino acid sequence represented by: RHHH.

Arginine is an amino acid having a guanidine group, which is a basic functional group, in a side chain, and pKa thereof is 12.48. Lysine is an amino acid having an amino group, which is a basic functional group, in a side chain, and pKa thereof is 10.53. Accordingly, basic functional groups in side chains of such amino acids are considered to be positively charged under neutral conditions. Accordingly, the arginine or lysine can enhance a structural change in response to a pH change of the second peptide. Accordingly, the second peptide comprising a cluster of basic amino acids comprising arginine or lysine inserted or added thereto may be able to induce a structural change under mildly acidic conditions (e.g., a pH 6.5 to lower than 7.0).

In the present invention, the EC₅₀ ratio (neutral pH conditions/acidic pH conditions) of an amino acid sequence derived from the amino acid sequence consisting of 2 or more consecutive amino acids having basic functional groups with pKa of 7 or lower in side chains comprising no arginine inserted or added to any site (including an amino or carboxyl terminus) may be compared with the EC₅₀ ratio (neutral pH conditions/acidic pH conditions) of an amino acid sequence derived from the amino acid sequence consisting of 2 or more consecutive amino acids having basic functional groups with pKa of 7 or lower in side chains by insertion of addition of one or more arginine residues at any site (including an amino or carboxyl terminus). Thus, effects of enhancing pH responsiveness caused by insertion or addition of arginine can be quantified. Specifically, the EC₅₀ ratio can be compared by calculating (the EC50 ratio of the latter/the EC50 ratio of the former), and the ratio is preferably 4.0 or higher.

A basic functional group in an amino acid having basic functional groups in a side chain comprised in the cluster of basic amino acids preferably has pKa of 5.0 to 7.0, and more preferably 5.5 to 6.5. It is most preferable that the cluster of basic amino acids comprise histidine having a basic functional group with pKa of 6.00 as an amino acid having a basic functional group in a side chain.

The number of the clusters of basic amino acids in the second peptide is not particularly limited, provided that the cluster is positively charged under acidic pH conditions to induce a structure change in a linker. The number thereof is preferably 1 to 4, more preferably 2 to 4, and further preferably 3 or 4.

The number of amino acids having basic functional groups in side chains comprised in the second peptide is not particularly limited, provided that the cluster is positively charged under acidic pH conditions to induce a structure change in a linker. The number thereof is preferably 10 or less, and it is more preferably 5% to 30% of the total number of amino acids in the amino acid sequence of the second peptide.

When the second peptide consists of (1) at least 1 of (a) an amino acid sequence consisting of 2 or more consecutive amino acids having basic functional groups with pKa of 7 or lower in side chains or (b) an amino acid sequence derived from the amino acid sequence (a) by insertion or addition of an arginine or lysine residue at any site (including an amino or carboxyl terminus) and (2) an amino acid sequence comprising 4 or more of the amino acids having basic functional groups with pKa of 7 or lower in side chains, the number of amino acids comprised in the second peptide is 10 to 60, preferably 12 to 50, more preferably 15 to 40, and further preferably 20 to 35.

The position of the cluster of basic amino acids in the second peptide is not particularly limited. When the amino acid sequence of the second peptide comprises 2 or more clusters of basic amino acids, specifically, (1) a cluster of basic amino acids may be selectively positioned at the amino or carboxyl terminus of the amino acid sequence, (2) a cluster of basic amino acids may be positioned at the amino terminus of the amino acid sequence and the other cluster of basic amino acids may be positioned at the carboxyl terminus of the amino acid sequence, or (3) none of the clusters of basic amino acids may be positioned at the amino or carboxyl terminus of the amino acid sequence. In the present invention, the second peptide may comprise an amino acid sequence serving as a substrate for an intra/extracellular protease and cleaved by the protease (hereafter, it may be simply referred to as a "substrate"). When the amino acid sequence of the second peptide further comprises an amino acid sequence cleaved by an intra/extracellular protease, the amino acid sequence preferably comprises a cluster of basic amino acids, an amino acid sequence cleaved by an intra/extracellular protease, and the amino acid sequence of a moiety binding to a target antigen (a moiety [a]) connected in that order from the amino terminus toward the carboxyl terminus or from the carboxyl terminus toward the amino terminus.

The second peptide may consist of an amino acid sequence comprising amino acids having basic functional groups with pKa of 7 or lower in side chains at every other position. The number of amino acids having basic functional groups with pKa of 7 or lower in side chains is 4 to 15, preferably 5 to 12, and more preferably 6 to 10. An example of such sequence is GGGGSHGHGHGHGHGHGHGHGGS (SEQ ID NO: 44, Figure 52).

The second peptide may comprise both of an amino acid sequence comprising amino acids having basic functional groups with pKa of 7 or lower in side chains at every other position and at least 1 of (a) an amino acid sequence consisting of 2 or more consecutive amino acids having basic functional groups with pKa of 7 or lower in side chains or (b) an amino acid sequence derived therefrom by insertion or addition of an arginine or lysine residue at any site (including an amino or carboxyl terminus).

Under acidic pH conditions, a pH may be 7.5 or lower, provided that a charged state of an amino acid having basic functional groups in side chains of the second peptide is changed. Under acidic pH conditions, accordingly, a pH is preferably 6.5 or lower, more preferably 6.0 or lower, and further preferably 5.5 or lower.

The first peptide, the second peptide, and the moiety binding to a target antigen may each bind to a linker. Specifically, any of the first peptide, the second peptide, and the moiety binding to a target antigen may bind to either or both of the other two moieties by a linker (or linkers). The linker is a peptide consisting of 2 to 30, preferably 2 to 20, and more preferably 2 to 10 amino acids.

The second peptide may comprise an amino acid sequence cleaved by an intra/extracellular protease. When the second peptide comprises an amino acid sequence serving as a substrate for an intra/extracellular protease and cleaved by the protease (it is simply referred to as a "substrate," a substrate cleaved by a given protease is referred to as "the protease substrate"), an amino acid sequence serving as a substrate for an intracellular protease can be referred to as a first cleavable amino acid sequence, and an amino acid sequence serving as a substrate for an extracellular protease can be referred to as a second cleavable amino acid sequence. In the present invention, the second peptide comprising the first cleavable amino acid sequence and the second cleavable amino acid sequence incorporated thereinto is also referred to as the second peptide comprising, as protease cleavable sequences, the first cleavable amino acid sequence in combination with the second cleavable amino acid sequence.

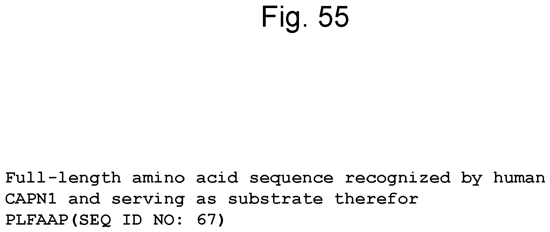

The term "intracellular protease" is also referred to as an "intracellularly active protease." After it is expressed in a cell, it acts in the cell without being secreted extracellularly, and it is associated with cell apoptosis. Examples of intracellular proteases include cytoplasmic cysteine proteases, such as caspase, calpain (also referred to as "CAPN"), and tripeptidyl peptidase. Examples of calpain isoforms include CAPN1 (µ-calpain), CAPN2 (m-calpain), CAPN3, CAPN4, CAPN5, CAPN6, CAPN7, CAPN8, CAPN9, CAPN10, CAPN11, CAPN12, CAPN13, CAPN14, CAPN15, CAPN16, and CAPN17. In the present invention, any of such isoforms can be used, and CAPN1 (calpain 1) or CAPN2 (calpain 2) is preferably used. Examples of tripeptidyl peptidase isoforms include tripeptidyl peptidase 1 and tripeptidyl peptidase 2. In the present invention, any of such isoforms can be used, and tripeptidyl peptidase 2 is preferably used. Accordingly, the first cleavable amino acid sequence in the second peptide is not particularly limited, provided that it is an amino acid sequence serving as a substrate for the intracellular protease; that is, an amino acid sequence that is recognized and cleaved by the protease. A preferable example thereof is an amino acid sequence that is recognized by human CAPN1 and serves as a substrate therefor (it may be referred to as a "CAPN1 substrate" or "CAPN substrate"), and a preferable example of the CAPN substrate is PLFAAP (SEQ ID NO: 67, Figure 55).

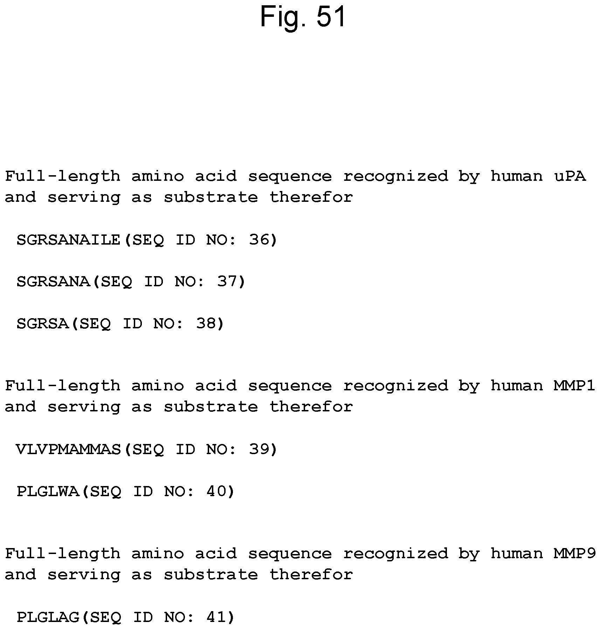

An extracellular protease is also referred to as an "extracellularly active protease," it is expressed in a form comprising a signal sequence, it is secreted to the outside of a cell, and it acts outside the cell. Examples of extracellular proteases include the urokinase type plasminogen activator (uPA), matrix metalloproteinase (MMP), plasmin, cathepsin, matriptase, and legumain. Examples of MMP isoforms include MMP1, MMP2, MMP3, MMP7, MMP8, MMP9, MMP10, MMP11, MMP12, MMP13, MMP14, MMP15, MMP16, MMP17, MMP18, MMP19, MMP20, MMP21, MMP23A, MMP23B, MMP24, MMP25, MMP26, MMP27, and MMP28. In the present invention, any of such isoforms can be used. Examples of cathepsin isoforms include cathepsin A, cathepsin B, cathepsin C, cathepsin D, cathepsin E, cathepsin F, cathepsin G, cathepsin H, cathepsin K, cathepsin L1, cathepsin L2, cathepsin O, cathepsin S, cathepsin W, and cathepsin X/Z. In the present invention, any of such isoforms can be used. Accordingly, the second cleavable amino acid sequence in the second peptide is not particularly limited, provided that it is an amino acid sequence serving as a substrate for the extracellular protease; that is, an amino acid sequence that is recognized and cleaved by the protease. A preferable example of the second cleavable amino acid sequence is an amino acid sequence that is recognized by human uPA and serves as a substrate therefor (it may be referred to as a "uPA substrate"). Preferable examples of the uPA substrates include SGRSANAILE (SEQ ID NO: 36, Figure 51) and SGRSA (SEQ ID NO: 38, Figure 51). Preferable examples of the amino acid sequences that are recognized by human MMP1 and serves as a substrate therefor (it may be referred to as a "MMP1 substrate" or "MMP substrate") include VLVPMAMMAS (SEQ ID NO: 39, Figure 51) and PLGLWA (SEQ ID NO: 40, Figure 51). A preferable example of an amino acid sequence that is recognized by human MMP9 and serves as a substrate therefor (it may be referred to as a "MMP9 substrate") is PLGLAG (SEQ ID NO: 41, Figure 51).

When the second peptide comprises both the first cleavable amino acid sequence, which is a substrate for an intracellular protease, and the second cleavable amino acid sequence, which is a substrate for an extracellular protease, the order thereof is not limited. For example, a molecule binding to a target antigen comprising the first peptide, the second peptide, and the moiety binding to a target antigen connected in that order may comprise the second cleavable amino acid sequence, which is a substrate for an extracellular protease, in a region closer to the first peptide and the first cleavable amino acid sequence, which is a substrate for an intracellular protease, in a region closer to the target-binding moiety. A molecule binding to a target antigen comprising the first peptide, the second peptide, and the moiety binding to a target antigen connected in that order may comprise the first cleavable amino acid sequence, which is a substrate for an intracellular protease, in a region closer to the first peptide and the second cleavable amino acid sequence, which is a substrate for an extracellular protease, in a region closer to the target-binding moiety. Accordingly, a molecule binding to a target antigen preferably comprises the first peptide, the second cleavable amino acid sequence, the first cleavable amino acid sequence, and the moiety binding to a target antigen connected in that order or the first peptide, the first cleavable amino acid sequence, the second cleavable amino acid sequence, and the moiety binding to a target antigen connected in that order.

While the preceding several paragraphs mainly describe the case in which the second cleavable amino acid sequence is comprised in the second peptide, the second cleavable amino acid sequence may be comprised in a region other than the second peptide, provided that it is comprised in a molecule that binds to the target antigen according to the present invention. For example, the second cleavable amino acid sequence may be comprised in the terminus of the first peptide or a third peptide inserted into a region between the second peptide and the moiety binding to a target antigen.

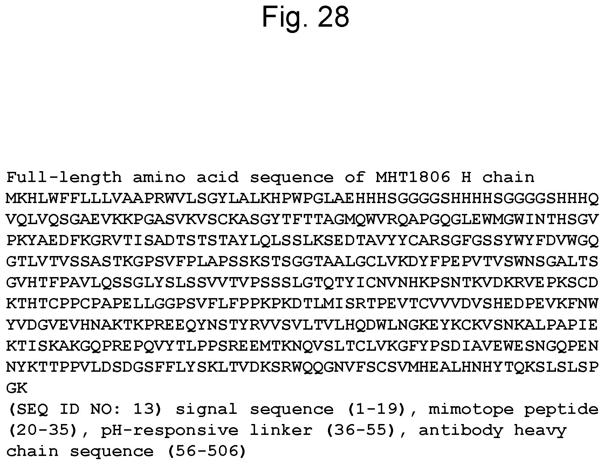

The first peptide may partially overlap with the second peptide. In the full-length amino acid sequence of the MHT1806 H chain (SEQ ID NO: 13, Figure 28), for example, the C terminus (HH) of the mimotope peptide (amino acids 20 to 35) is a part of a histidine cluster, and a part of the first peptide overlaps with a part of the second peptide.

When the second peptide comprises both of (1) at least 1 of (a) an amino acid sequence consisting of 2 or more consecutive amino acids having basic functional groups with pKa of 7 or lower in side chains or (b) an amino acid sequence derived from the amino acid sequence (a) by insertion or addition of an arginine or lysine residue at any site (including an amino or carboxyl terminus) and (2) an amino acid sequence comprising 4 or more of the amino acids having basic functional groups with pKa of 7 or lower in side chains and an amino acid sequence(s) cleaved by a protease (or proteases), which are the first cleavable amino acid sequence and/or the second cleavable amino acid sequence, preferably, the target-antigen-binding molecule comprise (1) at least 1 of (a) the amino acid sequence consisting of 2 or more consecutive amino acids having basic functional groups with pKa of 7 or lower in side chains or (b) an amino acid sequence derived therefrom by insertion or addition of an arginine or lysine residue at any site (including an amino or carboxyl terminus) and that the target-antigen-binding molecule do not comprise an amino acid sequence comprising 4 or more of the amino acids having basic functional groups with pKa of 7 or lower in side chains, when the amino acid sequence is cleaved by the protease.

When the second peptide comprises both of (1) at least 1 of (a) an amino acid sequence consisting of 2 or more consecutive amino acids having basic functional groups with pKa of 7 or lower in side chains or (b) an amino acid sequence derived from the amino acid sequence (a) by insertion or addition of an arginine or lysine residue at any site (including an amino or carboxyl terminus) and (2) an amino acid sequence comprising 4 or more of the amino acids having basic functional groups with pKa of 7 or lower in side chains and an amino acid sequence(s) cleaved by a protease (or proteases), which are the first cleavable amino acid sequence and/or the second cleavable amino acid sequence, the number of amino acids comprised in the second peptide is 15 to 150, preferably 15 to 120, more preferably 15 to 70, and further preferably 15 to 50.

### Another moiety

The target-antigen-binding molecule according to the present invention may further comprise another moiety. In the present invention, such another moiety is referred to as "a moiety [d]." The moiety [d] binds to the moiety binding to a target antigen of the molecule. The moiety [d] consists of one or more compounds selected from the group consisting of an antibody, which is not the moiety binding to a target antigen, or an antigen-binding fragment thereof, a peptide comprising an amino acid sequence that is not comprised in the first peptide or the second peptide, a cytokine, a toxin, a radioactive isotope, a label molecule, a photosensitive substance (it may be referred to as a "photosensitizer"), an immunostimulant, an antitumor compound, a drug, a payload, and a polymer.

Examples of a peptide comprising an amino acid sequence that is not comprised in the first peptide or the second peptide include, but are not limited to, an antibody, an antigen-binding fragment of an antibody, a non-immunoglobulin protein existing in nature, an artificial protein, a receptor protein or a ligand-binding fragment thereof, a ligand protein, and a protein that regulates the blood kinetics (e.g., antibody Fc or albumin). Examples of an antibody include, but are not limited to, an antibody, which is not a molecule binding to a target antigen, an antibody binding to a moiety other than the target-binding moiety in a molecule binding to a target antigen, and an antibody or the like binding to a molecule binding to a target antigen to serve as a multispecific molecule (e.g., bispecific antibody). Examples of cytokine include, but are not limited to, interleukin, interferon, chemokine, colony-stimulating factor, tumor necrosis factor, and growth factor. Examples of toxin include, but are not limited to, biotoxins, such as cyanotoxin, hemotoxin, necrotoxin, neurotoxin, and cytotoxin, and environmental toxin. Examples of a radioactive isotope include, but are not limited to, ¹³¹I, ²¹¹AT, and ⁸⁹Sr. Examples of a label molecule include, but are not limited to, fluorescent substances, such as FITC and PE, enzymes, such as HRP and AP, and biotin. Examples of a photosensitizer include, but are not limited to, a phthalocyanine derivative, a chlorin derivative, and a bacteriochlorin derivative. An example of an immunostimulant is, but is not limited to, an adjuvant. Examples of a polymer include, but are not limited to, a natural or artificial glycan, synthetic resin, and polyethylene glycol. Examples of an antitumor compound include, but are not limited to, a topoisomerase inhibitor, a mitotic inhibitor, a cell division inhibitor, a microtubule polymerization/depolymerization inhibitor, a modulator of a glucocorticoid receptor, a DNA binder, an alkylating agent, a radioactive isotope, siRNA, and an antibody or an antigen-binding fragment thereof. Examples of drugs include, but are not limited to, an antitumor agent, an immunostimulant, a cytokine, an antitumor compound, a drug, and a payload comprised in ADC described below.

When a moiety [d] is a polypeptide, the target-antigen-binding molecule according to the present invention may consist of a polypeptide. When a moiety [d] is a compound other than a polypeptide, the target-antigen-binding molecule according to the present invention may consist of the moiety [d] and a polypeptide.

The moiety [d] may be connected to the target-antigen-binding molecule according to the present invention by a linker.

When the moiety [d] is an antitumor compound, drug, or payload and the molecule binding to a target antigen is an antibody or an antigen binding site thereof (an antibody or the like), the molecule binding to a target antigen comprising the moiety [d] can be referred to as an "antibody-drug conjugate (ADC)." ADC is described in, for example, Methods Mol. Biol., 2013, 1045: 1-27; Nature Biotechnology, 2005, 23, pp.1137-1146. An antitumor compound is not particularly limited, provided that such substance can exert pharmacological effects when an antibody or the like binds thereto. Examples of antitumor compounds that can be used as antitumor agents include emtansine (a 4-({3-[(3-{[(1S)-2-{[(1S,2R,3S,5S,6S,16E,18E,20R,21S)-11-chloro-21-hydroxy-12,20-dimethoxy-2,5,9,16-tetramethyl-8,23-dioxo-4,24-dioxa-9,22-diazatetracyclo[19.3.1.110,14.03,5]hexacosa-10,12,14(26),16,18-pentaen-6-yl]oxy } -1 -methyl-2-oxoethyl]methylamino } -3 - oxopropyl)sulfanyl]-2,5-dioxopyrrolidin-1-yl}methyl)cyclohexylcarbonyl group) (e.g., WO 2001/000244 and WO 2001/000245) and a topoisomerase inhibitor (e.g., Liang, X. et al., Eur. J. Med. Chem., vol. 171, 2019, pp. 129-168), with topoisomerase type I inhibitors, such as a camptothecin derivative, irinotecan and an active metabolite thereof: SN-38 (e.g., EP 137145 A1 and US 4604463 A), exatecan (e.g., EP 495432 A1 and US 5637770 A), and an exatecan derivative (e.g., WO 2014/057687), being preferable. A preferable example of an exatecan derivative is N-[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]-2-hydroxyacetamide (e.g., WO 2014/057687, WO 2014/061277, WO 2015/146132, WO 2020/100954, and WO 2015/098099). Examples of other antitumor compounds include a pyrrolobenzodiazepine derivative (e.g., WO 2019/065964, WO 2013/173496, WO 2014/130879, WO 2017/004330, WO 2017/004025, WO 2017/020972, WO 2016/036804, WO 2015/095124, WO 2015/052322, WO 2015/052534, WO 2016/115191, WO 2015/052321, WO 2015/031693, and WO 2011/130613). Preferable examples of pyrrolobenzodiazepine derivatives include (11a'S)-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-1',11a'-dihydro-5'H-spiro[cyclopropane-1,2'-pyrrolo[2,1-c][1,4]benzodiazepin]-5'-one, (11a'S)-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepin]-8'-yl]oxy}pentyl)oxy]-1',10',11',11a'-tetrahydro-5'H-spiro[cyclopropane-1,2'-pyrrolo[2,1-c][1,4]benzodiazepin]-5'-one, (11a'S,11aʺʺS)-8',8"-[1,5-pentanediylbis(oxy)]bis(7'-methoxy-1',11a'-dihydro-5'H-spiro[cyclopropane-1,2'-pyrrolo[2,1-c][1,4]benzodiazepin]-5'-one), and (11a'S)-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-1',11a'-dihydro-5'H-spiro[cyclopropane-1,2'-pyrrolo[2,1-c][1,4]benzodiazepin]-5'-one (WO 2019/065964). Drugs may be immunostimulant, such as the STING agonist (e.g., WO 2021/202984, WO 2020/229982, WO 2020/050406, and WO 2021/177438), the TLR7/8 agonist, or the TLR8 agonist (e.g., WO 2018/009916 and WO 2019/084060). A preferable example of the STING agonist is a cyclic dinucleotide derivative. Examples of cyclic dinucleotide derivatives include (5R,7R,8R,12aR,14R,15R,15aS,16R)-15,16-dihydroxy-7-[1-(2-hydroxyethyl)-6-oxo-1,6-dihydro-9H-purin-9-yl]-2,10-bis(sulfanyl)-14-(6,7,8,9-tetrahydro-2H-2,3,5,6-tetraazabenzo[cd]azulen-2-yl)octahydro-2H,10H,12H-5,8-methano-2λ⁵,10λ⁵-flo[3,2-1][1,3,6,9,11,2,10]pentaoxadiphosphacyclotetradecine-2,10-dione, (5R,7R,8R,12aR,14R,15R,15aR,16R)-15-fluoro-16-hydroxy-7-[1-(2-hydroxyethyl)-6-oxo-1,6-dihydro-9H-purin-9-yl]-2,10-bis(sulfanyl)-14-(6,7,8,9-tetrahydro-2H-2,3,5,6-tetraazabenzo[cd]azulen-2-yl)octahydro-2H,10H,12H-5,8-methano-2λ⁵,10λ⁵-flo[3,2-1][1,3,6,9,11,2,10]pentaoxadiphosphacyclotetradecine-2,10-dione, (5R,7R,8R,12aR,14R,15R,15aR,16R)-7-[1-(2-aminoethyl)-6-oxo-1,6-dihydro-9H-purin-9-yl]-14-(8,9-dihydro-6-thia-2,3,5-triazabenzo[cd]azulen-2(7H)-yl)-15-fluoro-16-hydroxy-2,10-bis(sulfanyl)octahydro-2H,10H,12H-5,8-methano-2λ⁵,10λ⁵-flo[3,2-l][1,3,6,9,11,2,10]pentaoxadiphosphacyclotetradecine-2,10-dione, and N -(2- {9-[(5R,7R,8R,12aR,14R,15R,15aR,16R)-14-(8,9-dihydro-6-thia-2,3,5-triazabenzo[cd]azulen-2(7H)-yl)-15-fluoro-16-hydroxy-2,10-dioxo-2,10-bis(sulfanyl)octahydro-2H,10H,12H-5,8-methano-2λ⁵,10λ⁵-flo[3,2-l][1,3,6,9,11,2,10]pentaoxadiphosphacyclotetradecin-7-yl]-6-oxo-6,9-dihydro-1H-purin-1-yl}ethyl)-2-hydroxyacetamide (WO 2021/177438). In the present invention, the moiety [d] is bound to the moiety binding to a target antigen. When the moiety binding to a target antigen is an antibody or an antigen-binding fragment thereof, the moiety [d] can be bound to the antibody or an antigen-binding fragment thereof in accordance with a conventional technique. For producing a glycoprotein molecule comprising a therapeutic or preventive antibody or an Fc region thereof, technologies which modify glycans thereon to be homogeneous have been known. As a method of making glycans attached to a glycoprotein homogeneous, a transglycosylation reaction using an enzyme has been known. This reaction is a multi-step procedure comprising *in vitro* cleavage (hydrolysis) of a glycan and *in vitro* condensation of another glycan (transglycosylation). When conversion of the N- glycan is intended, in particular, a group of enzymes referred to as endo-β-N-acetylglucosaminidase (ENGase) is used. Such enzymes are required to have 1) an ability to hydrolyze a complex-type glycan in a substrate-specific manner and 2) an ability to perform a transglycosylation reaction to a predetermined structure. As transglycosylation reactions, an oxazoline method comprising transferring a glycan with an activated reducing end, such as a glycan with an oxazolylated reducing end, to a GlcNAc (N-acetylglucosamine) acceptor with the use of a single ENGase and a one-pot method comprising directly transferring a glycan having a reducing end that is not activated to a GlcNAc acceptor with the use of two types of ENGases are known (WO 2022/050300 and WO 2018/003983). In the present invention, a moiety [d], such as an antitumor compound, drug, or payload, can bind to an antibody or an antigen-binding fragment thereof directly or by a linker by, for example, a transglycosylation reaction in accordance with the methods described in the literatures indicated above. When the moiety [d] is a photosensitizer and a moiety binding to a target antigen is or comprises an antibody or an antigen-binding fragment thereof (an antibody or the like), a molecule binding to a target antigen comprising the moiety [d] can be used for photodynamic therapy (PDT), and such molecule can be referred to as an "antibody-directed phototherapy (ADP) molecule." The ADP molecule is described in, for example, Antibodies, 2013, 2, pp. 270-305. A photosensitizer is not particularly limited, provided that it can exert pharmacological effects by applying light to a site to which an antibody or the like has bound. Examples of photosensitizers include (2S)-2-[[2-[(2S,3S)-7-carboxy-3-(2-carboxyethyl)-17-ethenyl-12-ethyl-2,8,13,18-tetramethyl-2,3,23,24-tetrahydroporphyrin-5-yl]acetyl]amino]butanedioic acid (e.g., US Patent No. 5633275 and US Patent No. RE37180), IR700 (IRDye^{®} 700DX) (e.g., WO 2013/009475, WO 2004/038378, WO 2015/187677, WO 2017/031363, WO 2017/031367, WO 2018/156815, WO 2019/232478, and WO 202020/5623), and 2,4-difluoro-N-methyl-3-[10,15,20-tris[2,6-difluoro-3-(methylsulfamoyl)phenyl]-2,3,12,13,22,24-hexahydroporphyrin-5-yl]benzenesulfonamide (e.g., WO 2016/151458).

In tissue comprising a cell having a target antigen or a region in the vicinity thereof, a cleavable linker comprised in a molecule binding to a target antigen (i.e., a second peptide) is cleaved, and an active ingredient comprised in the molecule binding to a target antigen (e.g., a drug, antitumor compound, or immunostimulant) exerts its effects.

### 3. Method for producing molecule binding to target antigen

### (1) Method for identifying mimotope comprised in first peptide

A peptide binding to a complementarity determining region (CDR) in an antibody of interest or an antigen-binding fragment thereof can be identified using a peptide library. A peptide library may be constructed in accordance with a conventional technique. For example, a peptide library by various display systems, such as a ribosome composed of completely random amino acids may be constructed, and a peptide exhibiting high affinity to the CDR may be selected. Also, a peptide library by various display systems having a repeat motif of aromatic amino acid and Pro (a ZPZP motif) near the center (ZPZP lib) may be constructed, and a peptide exhibiting high affinity to the CDR may be selected. Z represents an aromatic amino acid selected from among histidine (His), phenylalanine (Phe), tyrosine (Tyr), and tryptophan (Trp), and P represents proline.

A mimotope comprised in the first peptide can be identified by the method described above.

An antibody CDR loop comprises a large quantity of aromatic amino acids. Since aromatic amino acids easily interact with each other, aromatic amino acids are preferably present near the center of a peptide.

The present invention includes a peptide library by various display systems used to identify the first peptide obtained by the method described above, mimotopes, and peptides binding to molecules other than antibodies (e.g., cytokines).

### (2) Method for producing molecule binding to target antigen

A peptide, which is a target-antigen-binding molecule according to the present invention and comprises an amino acid sequence comprised in the first peptide and/or an amino acid sequence comprised in the second peptide, can be prepared by, for example, recombination, *in vitro* translation, chemical synthesis, or peptide synthesis.

For example, a polynucleotide comprising a nucleotide sequence encoding an amino acid sequence comprised in a moiety binding to a target antigen and, according to need, an amino acid sequence comprised in the first peptide and/or an amino acid sequence comprised in the second peptide is introduced into a cell, the cell is cultured, and a polypeptide binding to the target antigen is collected from the culture product. Thus, the target-antigen-binding molecule according to the present invention can be produced.

To a polynucleotide comprising a nucleotide sequence encoding an amino acid sequence comprised in a moiety binding to a target antigen and, according to need, an amino acid sequence comprised in the first peptide and/or an amino acid sequence comprised in the second peptide, DNAs each encoding a relevant peptide may be connected, and an element, such as a promoter, an enhancer, or a polyadenylation signal, may further be operably connected thereto. When DNA is "operably connected(linked)" herein, DNA is connected to an element, so that the element can exert its functions. DNA may be inserted into an expression vector, a host cell may be transformed with the aid of the vector, and the host cell may then be cultured, produced, and collected. The vector may comprise DNA encoding a signal peptide that accelerates secretion of a molecule binding to a target antigen from a host cell. In such a case, DNA encoding a signal peptide is connected in-frame to DNA encoding a molecule binding to a target antigen. After the molecule binding to a target antigen is produced, the signal peptide may be removed, so as to obtain the molecule binding to a target antigen as a mature protein.

An expression vector is not particularly limited, as long as it can replicate DNA of interest in animal, bacterial, yeast, or other host cells, and examples thereof include known plasmids and phages. Examples of a vector used to construct an expression vector include pcDNA^{™} (Thermo Fisher Scientific), Flexi^{®} vector (Promega), pUC19, pUEX2 (Amersham), pGEX-4T, pKK233-2 (Pharmacia), and pMAM-neo (Clontech). As host cells, prokaryotic cells such as *Escherichia coli* and *Bacillus subtilis* and eukaryotic cells such as yeasts and animal cells can be used, with the use of eukaryotic cells being preferable. Examples of animal cells include the human embryonic kidney cell line HEK293 and the Chinese hamster ovary (CHO) cell. It is sufficient to introduce an expression vector into a host cell by a known method to transform the host cell. Examples of methods include an electroporation method, a calcium phosphate precipitation method, and a DEAE-dextran transfection method. The produced antibody can be purified by usual protein isolation or purification methods. For example, affinity chromatography or other chromatography techniques, filtration, ultrafiltration, salting out, dialysis, and the like can be suitably selected and performed in combination.

### (3) Method for producing peptide consisting of amino acid sequence comprising amino acid sequence of second peptide

A peptide consisting of an amino acid sequence comprising the amino acid sequence of the second peptide according to the present invention may be prepared by, for example, recombination, *in vitro* translation, chemical synthesis, or peptide synthesis.

For example, a polynucleotide encoding a peptide consisting of an amino acid sequence comprising the amino acid sequence of the second peptide is introduced into a cell, the cell is cultured, and a target peptide is collected from the culture product. Thus, the peptide consisting of an amino acid sequence comprising the amino acid sequence of the second peptide according to the present invention can be produced.

To a polynucleotide encoding a peptide consisting of an amino acid sequence comprising the amino acid sequence of the second peptide, DNAs each encoding a relevant peptide may be connected, and an element, such as a promoter, an enhancer, or a polyadenylation signal, may further be operably connected thereto. When DNA is "operably connected" herein, DNA is connected to an element, so that the element can exert its functions. DNA may be inserted into an expression vector, a host cell may be transformed with the aid of the vector, and the host cell may then be cultured, produced, and collected. The vector may comprise DNA encoding a signal peptide that accelerates secretion of a peptide consisting of an amino acid sequence comprising the amino acid sequence of the second peptide from a host cell. In such a case, DNA encoding a signal peptide is connected in-frame to DNA encoding a peptide consisting of an amino acid sequence comprising the amino acid sequence of the second peptide. After the peptide consisting of an amino acid sequence comprising the amino acid sequence of the second peptide is produced, the signal peptide may be removed, so as to obtain the peptide consisting of an amino acid sequence comprising the amino acid sequence of the second peptide as a mature protein.

An expression vector is not particularly limited, as long as it can replicate DNA of interest in animal, bacterial, yeast, or other host cells, and examples thereof include known plasmids and phages. Examples of a vector used to construct an expression vector include pcDNA^{™} (Thermo Fisher Scientific), Flexi^{®} vector (Promega), pUC19, pUEX2 (Amersham), pGEX-4T, pKK233-2 (Pharmacia), and pMAM-neo (Clontech). As host cells, prokaryotic cells such as *Escherichia coli* and *Bacillus subtilis* and eukaryotic cells such as yeasts and animal cells can be used, with the use of eukaryotic cells being preferable. Examples of animal cells include the human embryonic kidney cell line HEK293 and the Chinese hamster ovary (CHO) cell. It is sufficient to introduce an expression vector into a host cell by a known method to transform the host cell. Examples of methods include an electroporation method, a calcium phosphate precipitation method, and a DEAE-dextran transfection method. The produced antibody can be purified by usual protein isolation or purification methods. For example, affinity chromatography or other chromatography techniques, filtration, ultrafiltration, salting out, dialysis, and the like can be suitably selected and performed in combination.

### 4. Use of molecule binding to target antigen according to the present invention

The target-antigen-binding molecule according to the present invention, a salt thereof, or a hydrate of the molecule or salt (hereafter, they are referred to as "the molecule or the like binding to the target antigen according to the present invention") can be used as an agent for prevention and/or treatment of a disease caused by a diseased cell.

When a diseased cell is a tumor cell, the molecule or the like binding to the target antigen according to the present invention can be used as an anticancer agent.

The anti-cancer agent can be used for one type or two or more types of cancer species selected from among carcinoma, sarcoma, lymphoma, leukemia, myeloma, germinoma, brain tumor, carcinoid, neuroblastoma, retinoblastoma, and nephroblastoma. Specific examples of carcinoma include kidney cancer, melanoma, squamous cell cancer, basal cell cancer, conjunctival cancer, oral cavity cancer, laryngeal cancer, pharyngeal cancer, thyroid gland cancer, lung cancer, breast cancer, esophageal cancer, gastric cancer, duodenal cancer, small bowel cancer, large bowel cancer, rectal cancer, appendiceal cancer, anal cancer, liver cancer, gallbladder cancer, bile duct cancer, pancreatic cancer, adrenal cancer, bladder cancer, prostate cancer, uterine cancer, and vaginal cancer. Specific examples of sarcoma include liposarcoma, angiosarcoma, chondrosarcoma, rhabdomyosarcoma, Ewing's sarcoma, osteosarcoma, undifferentiated pleomorphic sarcoma, myxofibrosarcoma, malignant peripheral neurilemmoma, retroperitoneal sarcoma, synoviosarcoma, uterine sarcoma, gastrointestinal stromal tumor, leiomyosarcoma, and epithelioid sarcoma. Specific examples of lymphoma include B-cell lymphoma, T/NK-cell lymphoma, and Hodgkin's lymphoma. Specific examples of leukemia include myelogenic leukemia, lymphatic leukemia, myeloproliferative disorder, and myelodysplastic syndrome. A specific example of myeloma is multiple myeloma. Specific examples of germinoma include testicular cancer and ovarian cancer. Specific examples of brain tumor include neuroglioma and meningioma.

The anti-cancer agent of the present invention can comprise a molecule that binds to the target antigen of the present invention in an amount effective for treatment, as well as pharmaceutically acceptable carriers, diluents, solubilizers, emulsifiers, preservatives, aids, and the like. The "pharmaceutically acceptable carriers" and the like can be suitably selected from a broad range according to the type of a target disease and the dosage form of a drug. An administration method for the anti-tumor agent of the present invention can be suitably selected. For example, the anti-tumor agent can be injected, and local injection, intraperitoneal injection, selective intravenous infusion, intravenous injection, subcutaneous injection, organ perfusate infusion, and the like can be employed. Further, an injection solution can be formulated using a carrier comprising a salt solution, a glucose solution, or a mixture of a salt solution and a glucose solution, various types of buffer solutions, or the like. Further, a powder may be formulated and mixed with a liquid carrier to prepare an injection solution before use.

Other administration methods can be suitably selected along with development of a formulation. For example, oral solutions, powders, pills, capsules, tablets, and the like can be applied for oral administration. For oral solutions, oral liquid preparations such as suspensions and syrups can be produced using water, saccharides such as sucrose, sorbitol, and fructose, glycols such as polyethylene glycol, oils such as sesame oil and soybean oil, preservatives such as alkyl parahydroxybenzoates, flavors such as strawberry flavor and peppermint, and the like. Powders, pills, capsules, and tablets can be formulated using excipients such as lactose, glucose, sucrose, and mannitol, disintegrating agents such as starch and alginate soda, lubricants such as magnesium stearate and talc, binders such as polyvinyl alcohol, hydroxypropyl cellulose, and gelatin, surfactants such as fatty acid esters, plasticizers such as glycerin, and the like. Tablets and capsules are preferred unit dosage forms for the composition of the present invention in that they are easily administered. Solid production carriers are used to produce tablets and capsules.

The effective dose of the molecule or the like that binds to the target antigen of the present invention used for treatment may be changed according to characteristics of symptoms to be treated and the patient's age and condition and may be finally determined by a physician. For example, one dose is 0.0001 mg to 100 mg per kg of body weight. The predetermined dose may be administered once every 1 to 180 days, or the dose may be divided into two doses, three doses, four doses, or more doses per day and administered at appropriate intervals.

The molecule or the like that binds to the target antigen of the present invention can be used in combination with another drug as an agent for prevention or treatment of a disease caused by a diseased cell. The molecule or the like that binds to the target antigen of the present invention can be administered to a person who has or is at a risk of a disease caused by a diseased cell before, simultaneously with, or after the administration of the other drug. When the molecule or the like that binds to the target antigen of the present invention is administered simultaneously with the other drug, they may be administered in the form of a combination drug thereof (i.e., a preparation comprising both of the molecule and the drug) or a single agent (i.e., a preparation comprising either thereof).

### 5.Test drug, diagnostic drug, and reagent

The target-antigen-binding molecule according to the present invention or the like can be used as, for example, a test drug, a diagnostic drug, or a reagent. The target-antigen-binding molecule according to the present invention or the like binds to a target antigen with higher affinity under acidic pH conditions than under neutral pH conditions. Thus, a pH change in the vicinity of the target antigen can be inspected based on the amount of the antibody bound to the target antigen. For example, the target-antigen-binding molecule according to the present invention or the like may be used as an analyte or may be administered to a subject, and the molecule or the like can be used as a test drug, a diagnostic drug, or a reagent for a disease caused by a pH change in the vicinity of the target antigen or a disease with a phenotype indicating such pH change. The target-antigen-binding molecule according to the present invention or the like can be preferably used as, for example, a test drug, a diagnostic drug, or a reagent for a disease caused by a pH change in the vicinity of the target antigen existing in the cancer microenvironment, the endosomal environment, or the lysosomal environment or a disease with a phenotype indicating such pH change. Examples of test drugs, diagnostic drugs, and reagents include those used for determination or measurement of a risk of acquiring a disease, determination of disease acquisition, measurement of the extent of progression or exacerbation, measurement or determination of effects of drug treatment with the use of a pharmaceutical composition comprising the target-antigen-binding molecule, measurement or determination of effects of treatment other than the drug treatment, measurement of a risk of recurrence, or determination of recurrence. It should be noted that use of the molecule or the like is not limited thereto, provided that it is used as a test drug, a diagnostic drug, or a reagent. The present invention provides a composition used as a test drug, a diagnostic drug, or a reagent comprising the target-antigen-binding molecule according to the present invention or the like (hereafter, referred to as a composition for testing or other purposes). Such composition for testing or other purposes can comprise a pH buffer, an osmo-regulator, a salt, a stabilizer, a preservative, a developer, a sensitizer, an anticoagulant, or the like.

### Examples

Hereafter, the present invention is described in greater detail with reference to the examples, although the present invention is not limited to these examples.

In the following examples, genetic engineering procedures were performed in accordance with the method described in Molecular Cloning (Sambrook, J., Fritsch, E. F. and Maniatis, T., Cold Spring Harbor Laboratory Press, 1989) and methods described in other experimental protocols employed by a person skilled in the art, unless otherwise specified. When a commercially available reagent or kit was to be used, the procedures in accordance with the instructions of such commercial product were employed. Synthesis of primers required for gene synthesis or vector construction was outsourced, according to need (Fasmac Co., Ltd. and Thermo Fisher Scientific).

### (Example 1) Preparation of anti-TROP2 antibody HT1-11

### 1)-1 Expression and purification of anti-TROP2 antibody HT1-11

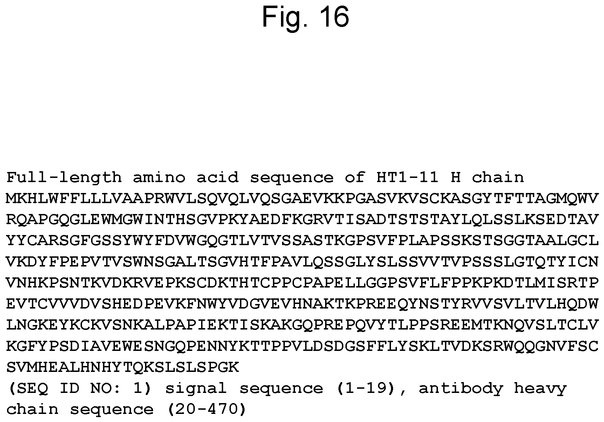

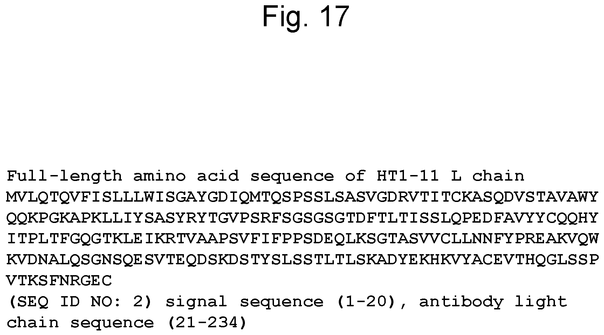

A mammalian cell expression vector comprising, as the backbone, pcDNA 3.3 (Thermo Fisher Scientific) into which DNA encoding the heavy chain (SEQ ID NO: 1, Figure 16) or light chain (SEQ ID NO: 2, Figure 17) of the known anti-TROP2 antibody described in WO 2015/098099 has been cloned was introduced into Expi293F cells, and an antibody molecule was purified from the transient expression culture supernatant. Expi293F cells (Thermo Fisher Scientific) were subcultured in accordance with the instructions. A culture solution of the Expi293F cells in a logarithmic growth phase was diluted to 2.5 × 10⁶ cells/ml in the Expi293 Expression medium (Thermo Fisher Scientific), heavy chain and light chain expression vectors and polyethyleneimine (Polyscience) were added to Opti-Pro SFM medium (Thermo Fisher Scientific), the reaction was allowed to proceed, and the reaction product was then added to the Expi293F cells. Agitation culture was performed in an incubator at 37°C in the presence of 8% CO₂ at 135 rpm for 5 days. MabSelectSuRe (Cytiva) equilibrated with PBS (pH 7.4) was added to the culture supernatant after centrifugation to allow the target antibody molecules to adsorb thereto. After the non-adsorbed components were removed with the aid of PBS, the adsorbed components were eluted using acetate buffer (pH 3.5). The elution fraction was neutralized with the aid of Tris buffer (pH 9.0), and the buffer was exchanged with 25 mM histidine, 5(w/v)% sorbitol (pH 6.0) using an ultrafiltration membrane. The resultant was concentrated, according to need, and applied to a gel filtration column Superdex 200 Increase (Cytiva) equilibrated with 25 mM histidine, 300 mM NaCl, 5(w/v)% sorbitol (pH 5.5) in advance to collect a monomer fraction. The purified sample was subjected to SDS-polyacrylamide electrophoresis (SDS-PAGE) and size exclusion chromatography (SEC) to evaluate the degree of monomer purification. The concentration of the purified antibody was determined by a method known to a person skilled in the art based on the absorbance at 280 nm obtained with the use of a spectrophotometer and the absorbance coefficient determined by the PACE method (Protein Science; 4 (11): 2411-2423, 1995).

### 1)-2 Evaluation of binging intensity between anti-TROP2 antibody HT1-11 and anti-TROP2 antigen by ELISA

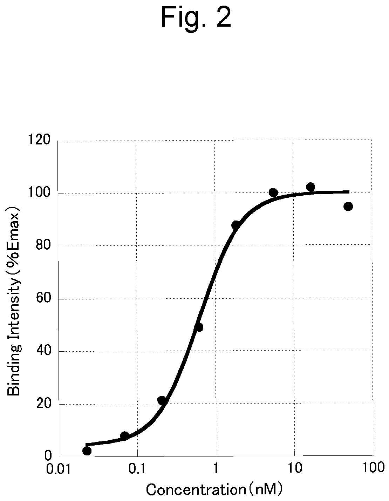

NeutrAvidin (Thermo Fisher Scientific) diluted to 1 µg/ml with PBS was added at 50 µl/well to a 96-well Maxi-sorp plate (Black, Nunc) and immobilized at 4°C overnight. The plate was washed with PBS containing 0.05% (w/v) Tween-20 (BioRad) (ELISA buffer) and then blocked with Blocker Casein (Thermo Fisher Scientific). The plate was washed with ELISA buffer, the biotinylated human TROP2 antigen (Accession Number: P09758; the extracellular domain was purified by the method known to a person skilled in the art and the C-terminal Avi tag sequence was then biotinylated) diluted to 1 µg/ml with PBS was added at 50 µl/well, and the resultant was then agitated for 30 minutes at room temperature. The plate was washed with ELISA buffer, the antibody of Example 1)-1 with the concentration thereof being adjusted with ELISA buffer was added at 50 µl/well, and the resultant was then agitated for 30 minutes at room temperature. The plate was washed with ELISA buffer, 50 µl of the horseradish peroxidase (HRP)-labeled anti-human IgG antibody (Jackson Immuno Research Laboratories) diluted to 2,500-fold with ELISA buffer was added, and the resultant was then agitated for 30 minutes at room temperature. The plate was washed with ELISA buffer, the SuperSignal Pico ELISA chemiluminescent substrate (Thermo Fisher Scientific) was added, and chemiluminescence 10 minutes thereafter was then assayed using a plate reader. As shown in Figure 2, HT1-11 was found to have bound to the human TROP2 antigen in a concentration-dependent manner.

### (Example 2) Concentration of mimotope peptide binding to anti-TROP2 antibody HT1-11

A peptide library by ribosome display composed of completely random 15 amino acids (Linear 15mer lib) was constructed, and a peptide capable of binding to HT1-11 was concentrated by a method known to a person skilled in the art. At the outset, human serum-derived IgG (Sigma Aldrich) or HT1-11 biotinylated with EZ-Link NHS-PEG4-Bioin (Thermo Fisher Scientific) was immobilized on Dynabeads Streptavidin M-280 (Thermo Fisher Scientific), and the ribosome displaying a peptide (hereafter referred to as "RD") was allowed to react with human serum-derived IgG-bound beads. RDs that did not bind to the beads were collected using a magnet stand (DynaMag-2, Thermo Fisher Scientific) and then allowed to react with the HT1-11-bound beads. RDs that did not bind to HT1-11 were removed by washing with the use of a magnet stand, and mRNAs were purified from RDs that had bound to HT1-11. Thereafter, RDs were prepared again by RT-PCR and *in vitro* translation. This process of panning was performed 3 times.

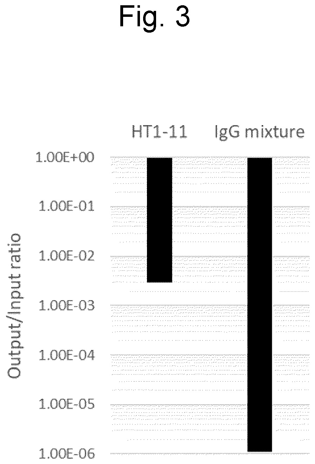

mRNAs after the third round of panning were subjected to *in vitro* translation to prepare RDs, and the prepared RDs were then allowed to react with Dynabeads Streptavidin M-280 comprising biotinylated HT1-11 or human serum-derived IgG immobilized thereon (amount of input: 6 × 10¹¹). RDs that did not bind were removed by washing with the use of a magnet stand, mRNAs were collected from the RDs that had bound, and the amount collected (output) was quantified and evaluated by RT-qPCR. As shown in Figure 3, the number of mRNAs collected under the HT1-11-immobilized conditions was 2,624 times greater than that collected under the human serum IgG-immobilized conditions. The results indicate concentration of peptides binding specifically to HT1-11.

### (Example 3) Screening of mimotope peptide binding to anti-TROP2 antibody 3)-1 Preparation of HT1-11 scFv comprising peptide obtained by panning fused thereto

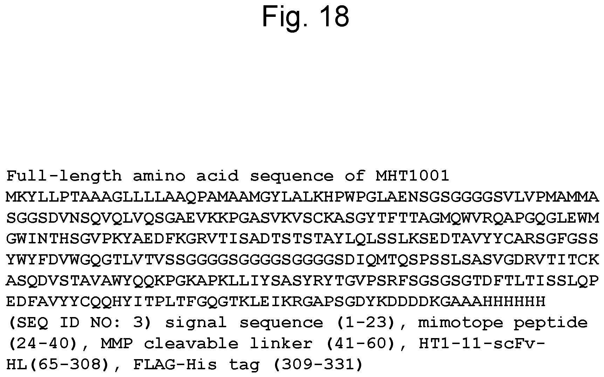

A restriction enzyme was added to the DNA fragment after the third round of panning, and a DNA fragment encoding a random peptide fragment was purified by a method known to a person skilled in the art. A DNA fragment was ligated to an *E. coli* expression vector by a method known to a person skilled in the art, so that the pelB signal sequence, the DNA fragment, the MMP cleavable linker (comprising a conventional MMP substrate (Journal of Controlled Release; 161: 804-812, 2012); amino acids 41 to 60 in the amino acid sequence as shown in SEQ ID NO: 3 and Figure 18), HT1-11 scFv (in the order of VH-VL or VL-VH), the FLAG tag, and the His tag would be translated in that order, so as to transform *E. coli* XL-1 Blue (Agilent Technologies). *E. coli* cells were cultured in the presence of isopropyl-β-D-thiogalactopyranoside (IPTG) (Sigma-Aldrich), and a culture supernatant comprising peptide-fused scFv was collected.

### 3)-2 Acquisition of mimotope peptide

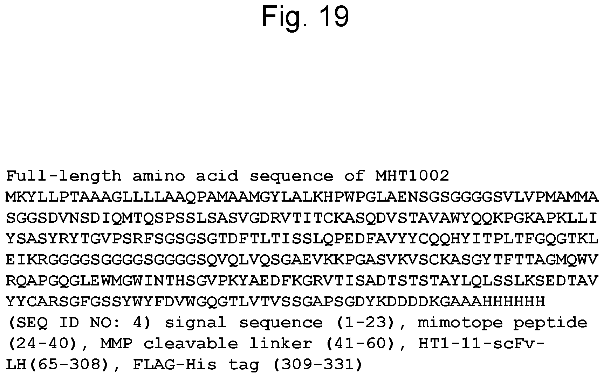

Binding intensity to the human TROP2 antigen was evaluated by ELISA in the same manner as in Example 1)-2. A culture supernatant comprising the peptide-fused scFv was diluted to 10-fold using TBS comprising 2 mM calcium chloride (MMP buffer) and allowed to react with 100 nM active human MMP1 (Accession Number: P03956) at 37°C for 15 minutes to cleave the MMP cleavable linker. The resultant was then added to the plate comprising the human TROP2 antigen immobilized thereon at 50 µl/well. The bound scFv was detected using the HRP-labeled anti-FLAG antibody (Sigma-Aldrich) diluted to 5,000-fold with ELISA buffer. The same procedure was implemented without the addition of MMP1, and clones exhibiting the high binding intensity ratio (under the condition with the addition of MMP1/under the condition without the addition of MMP1) were selected as positive clones. After an expression vector of positive clones was purified, the sequence of the translated region was analyzed by a method known to a person skilled in the art, and unique clones MHT1001 (SEQ ID NO: 3, Figure 18) and MHT1002 (SEQ ID NO: 4, Figure 19) were obtained.

### 3)-3 Evaluation of binding intensity of positive clone

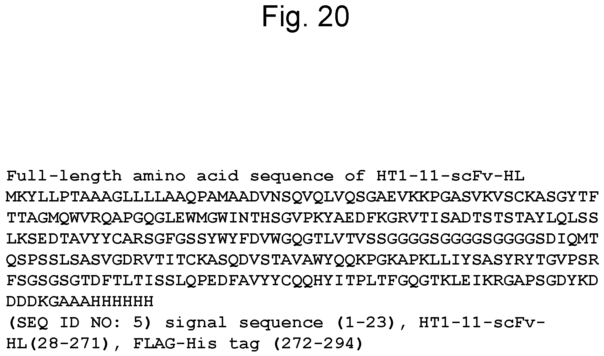

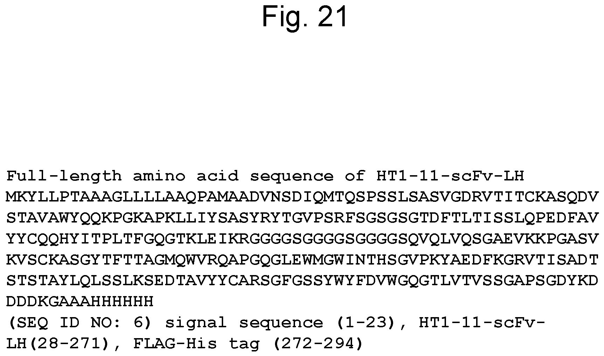

HT1-11-scFv-HL (SEQ ID NO: 5, Figure 20), HT1-11-scFv-LH (SEQ ID NO: 6, Figure 21), and the positive clones MHT1001 and MHT1002 identified in Example 3)-2 were expressed in *E. coli* XL-1 Blue, purified from the culture supernatant with the use of Ni Sepharose excel (Cytiva), and buffer-exchanged to TBS. The concentration of the purified antibody was determined by a method known to a person skilled in the art based on the absorbance at 280 nm obtained with the use of a spectrophotometer and the absorbance coefficient determined by the PACE method (Protein Science; 4 (11): 2411-2423, 1995). With the use of the purified samples, binding intensity to the human TROP2 antigen was evaluated by ELISA under the condition with the addition of MMP1 and under the condition without the addition of MMP1 in the same manner as in Example 3)-2. The antibody was adjusted to 1 µM with the use of MMP buffer, MMP1 was added to the final concentration of 1 µM or 0 µM, and the reaction was allowed to proceed at 37°C for 15 minutes. Thereafter, the antibody with the concentration thereof being adjusted with ELISA buffer was added to the wells.

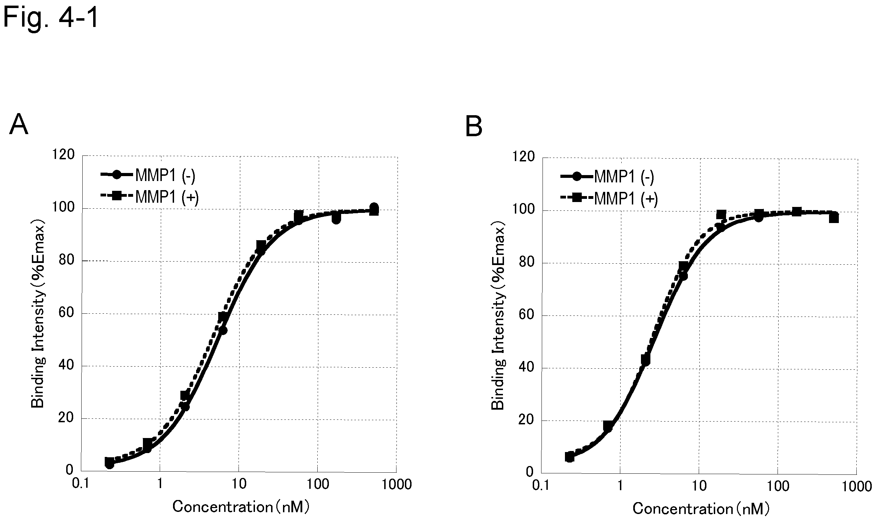

As shown in Figure 4-1 (A) and Figure 4-1 (B), HT1-11-scFv-HL and HT1-11-scFv-LH to which the mimotope peptide had not fused exhibited an equivalent binding intensity regardless of the addition of M1VVIP1. Also, MHT1001 and MHT1002 to which the mimotope peptide had fused was found to exhibit a higher binding intensity under the condition with the addition of MMP1 than under the condition without the addition of MMP 1, and the EC₅₀ ratios of binding intensity (under the condition without the addition of M1VVIP1/under the condition with the addition of MMP1) were 7.9 and 4.3 (Figure 4-2 (C) and Figure 4-2 (D)).

### (Example 4) Preparation of anti-TROP2 masked antibody and evaluation of binding intensity thereof

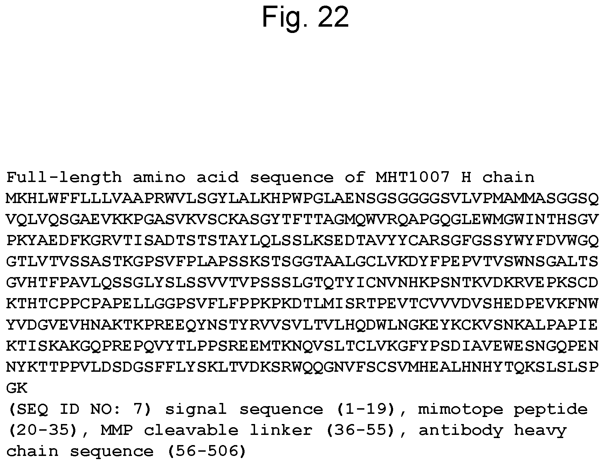

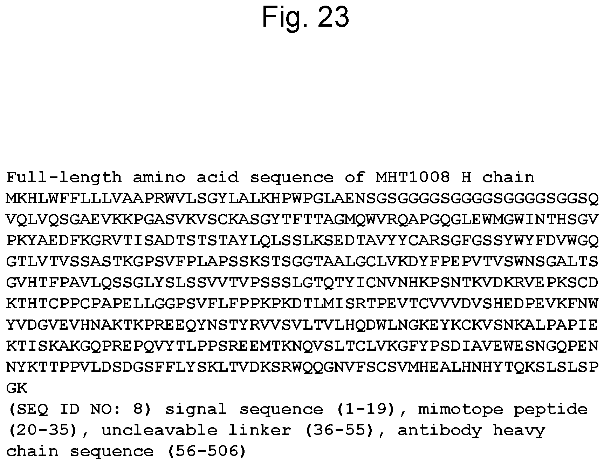

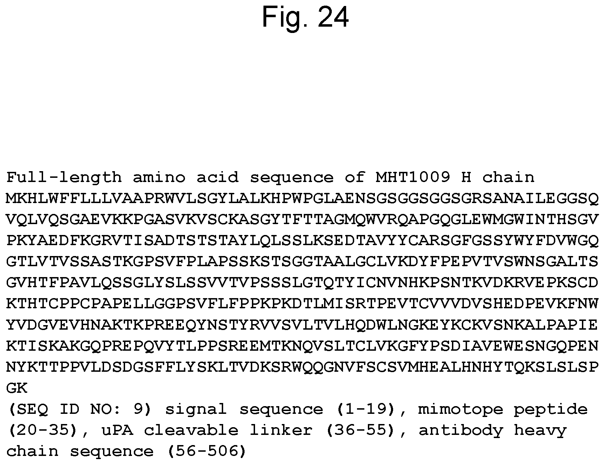

An expression vector for a heavy chain of the anti-TROP2 masked antibody MHT1007 comprising the MHT1001 mimotope peptide and the MMP cleavable linker (see Example 3)-1) (Table 2, SEQ ID NO: 7, Figure 22) fused thereto was constructed by a method known to a person skilled in the art. Also, an expression vector for a heavy chain of MHT1008 in which the MMP cleavable linker portion had been modified into an uncleavable linker that would not be cleaved by a protease (Table 2, SEQ ID NO: 8, Figure 23) and an expression vector for a heavy chain of MHT1009 in which the MMP cleavable linker portion had been modified into an uPA cleavable linker (comprising a conventional uPA substrate (Journal of Biological Chemistry; 272 (33): 20456-20462, 1997): amino acids 36 to 55 in the amino acid sequence shown in Figure 24 and SEQ ID NO: 9) were constructed. An expression vector for the antibody heavy chain was used in combination with the expression vector for the HT1-11 light chain, masked antibodies were purified from the culture supernatant of the Expi293F cells in the same manner as in Example 1)-1, and the protein concentration was then determined. The masked antibodies prepared were designated as MHT1007, MHT1008, and MHT1009 corresponding to the names of heavy chain expression vectors (Table 2). In Table 2, the "pH responsive linker" in the heavy chain sequence of the masked antibody corresponds to the second peptide in the target-antigen-binding molecule according to the present invention.

**Table 2: Anti-TROP2 masked antibody and amino acid sequence information**

| Masked antibody | Heavy chain sequence | Light chain sequence |
|---|---|---|
| MHT1007 | MHT1007 (SEQ ID NO: 7, Fig. 22) | HT1-11 (SEQ ID NO: 2, Fig. 17) |
| MHT1008 | MHT1008 (SEQ ID NO: 8, Fig. 23) | HT1-11 (SEQ ID NO: 2, Fig. 17) |
| MHT1009 | MHT1009 (SEQ ID NO: 9, Fig. 24) | HT1-11 (SEQ ID NO: 2, Fig. 17) |
| MHT1803 | MHT1803 (SEQ ID NO: 10, Fig. 25) | HT1-11 (SEQ ID NO: 2, Fig. 17) |
| MHT1804 | MHT1804 (SEQ ID NO: 11, Fig. 26) | HT1-11 (SEQ ID NO: 2, Fig. 17) |
| MHT1805 | MHT1805 (SEQ ID NO: 12, Fig. 27) | HT1-11 (SEQ ID NO: 2, Fig. 17) |
| MHT1806 | MHT1806 (SEQ ID NO: 13, Fig. 28) | HT1-11 (SEQ ID NO: 2, Fig. 17) |
| MHT1808 | MHT1808 (SEQ ID NO: 14, Fig. 29) | HT1-11 (SEQ ID NO: 2, Fig. 17) |
| MHT1809 | MHT1809 (SEQ ID NO: 15, Fig. 30) | HT1-11 (SEQ ID NO: 2, Fig. 17) |
| MHT1810 | MHT1810 (SEQ ID NO: 16, Fig. 31) | HT1-11 (SEQ ID NO: 2, Fig. 17) |
| MHT1811 | MHT1811 (SEQ ID NO: 17, Fig. 32) | HT1-11 (SEQ ID NO: 2, Fig. 17) |
| MHT1817 | MHT1817 (SEQ ID NO: 18, Fig. 33) | HT1-11 (SEQ ID NO: 2, Fig. 17) |
| MHT1818 | MHT1818 (SEQ ID NO: 19, Fig. 34) | HT1-11 (SEQ ID NO: 2, Fig. 17) |
| MHT1819 | MHT1819 (SEQ ID NO: 20, Fig. 35) | HT1-11 (SEQ ID NO: 2, Fig. 17) |
| MHT1221 | MHT1221 (SEQ ID NO: 30, Fig. 45) | HT1-11 (SEQ ID NO: 2, Fig. 17) |

Except for the ways described below, binding intensity to the human TROP2 antigen was evaluated by ELISA under the condition with the addition of a protease and under the condition without the addition thereof in the same manner as in Example 1)-2 and Example 3)-2. Under the condition with the addition of a protease, MMP 1 or active human uPA (Accession Number: P00749) (final concentration: 300 nM) was added to the 3 µM antibody, the reaction was allowed to proceed at 37°C, and the antibody with the concentration thereof being adjusted with ELISA buffer was added to wells comprising the human TROP2 antigen immobilized thereon.

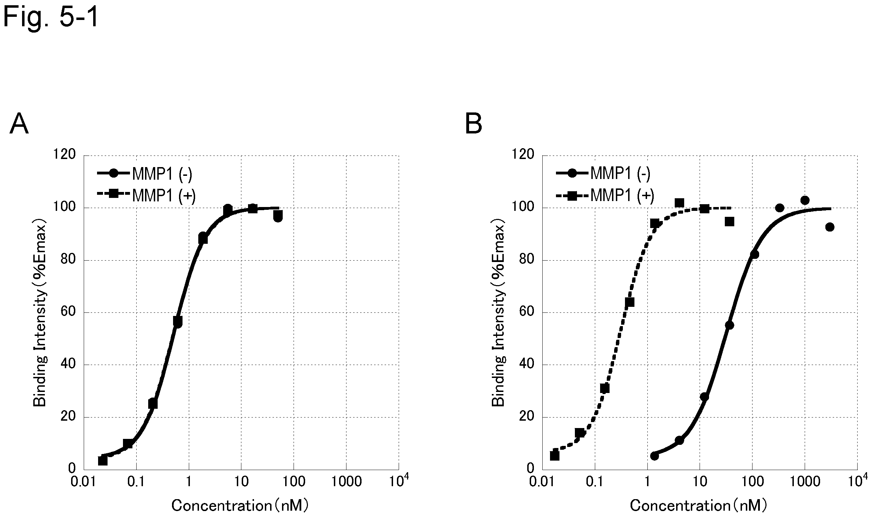

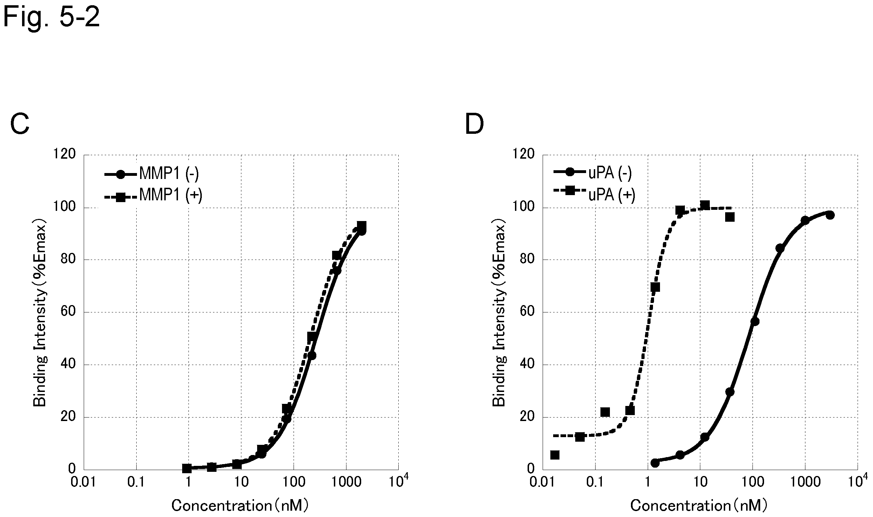

As shown in Figure 5-1 (A), HT1-11 to which the mimotope peptide had not fused exhibited an equivalent binding intensity regardless of the addition of MMP1. Under the condition without the addition of a protease, binding intensity of MHT1007 comprising the mimotope peptide fused thereto was lower than that of HT1-11, and masking effects were also observed as with the case of scFv. Also, binding intensity was higher under the condition with the addition of MMP1 than under the condition without the addition of MMP1, and the EC₅₀ ratio of binding intensity (under the condition without the addition of MMP1/under the condition with the addition of MMP1) was 104 (Figure 5-1 (B)). MHT1008 comprising an uncleavable linker exhibited a lowered binding intensity regardless of the addition of MMP1 (Figure 5-2 (C)). MHT1009 comprising the uPA cleavable linker exhibited a higher binding intensity under the condition with the addition of uPA than under the condition without the addition of uPA, the EC₅₀ ratio of binding intensity (under the condition without the addition of uPA/under the condition with the addition of uPA) was 83 (Figure 5-2 (D)). The results demonstrate that the mimotope obtained in Example 3 would serve as a masking domain not only in the scFv form but also in the IgG form and that a cleavable linker sequence could be modified while maintaining the masking effects.

In order to verify that the peptide obtained was the mimotope, the Fab region of MHT1007 comprising the masking peptide and the MMP cleavable linker (see Example 3)-1) was prepared by a method known to a person skilled in the art, and the Fab region was crystallized and subjected to X-ray crystallography. The results demonstrate that the peptide had bound to the CDR of HT1-11 (the data are not shown). In addition, a chemically synthesized masking peptide was found to bind to HT1-11 competitively with the TROP2 antigen by SPR (the data are not shown).

### (Example 5) Design of a linker comprising His cluster and evaluation of pH responsiveness of binding

His with pKa of 6.0 in a side chain is an amino acid that is easily protonated in the tumor microenvironment in which pHe is lowered. The anti-TROP2 masked antibody comprising a plurality of His residues in the linker was designed, and the interaction thereof with the human TROP2 antigen was evaluated by ELISA in the same manner as in Example 1)-2, except for the ways shown below. The antibody was diluted to 2000 nM with a neutral buffer (50 mM Tris or 50 mM HEPES, 150 mM NaCl, 0.05% Tween-20, pH 7.5) or an acidic buffer (50 mM Bis-Tris, 150 mM NaCl, 0.05% Tween-20, pH 6.5-5.5), the reaction was allowed to proceed at 37°C, the concentration was adjusted with each buffer, and the resultant was added to wells comprising the human TROP2 antigen immobilized thereon. Washing and dilution of the horseradish peroxidase (HRP)-labeled anti-human IgG antibody (Jackson Immuno Research Laboratories) were performed for relevant pH conditions.

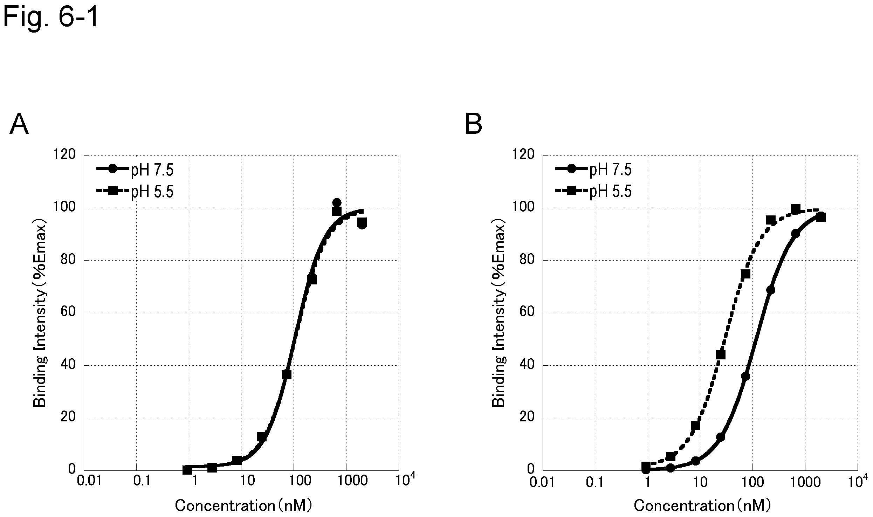

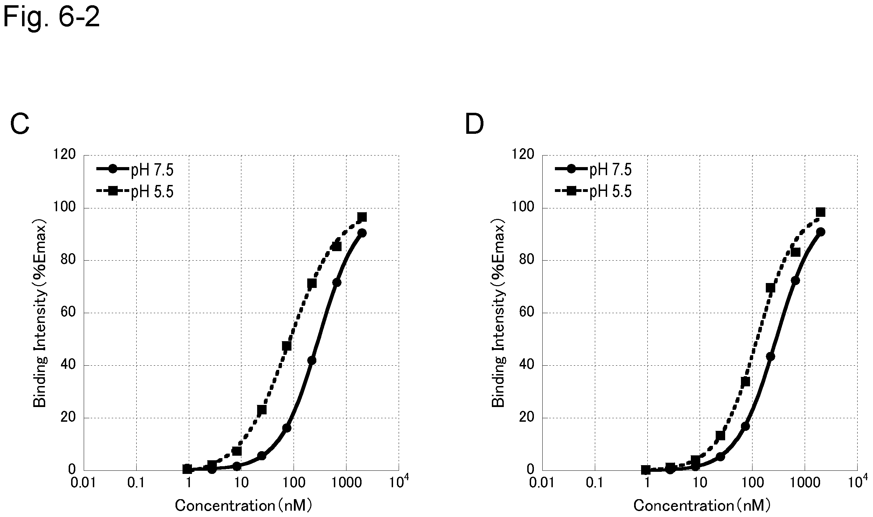

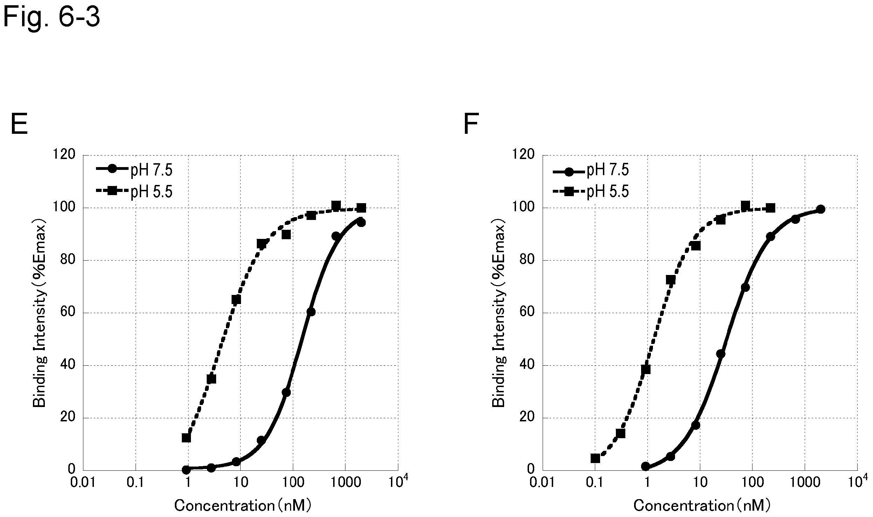

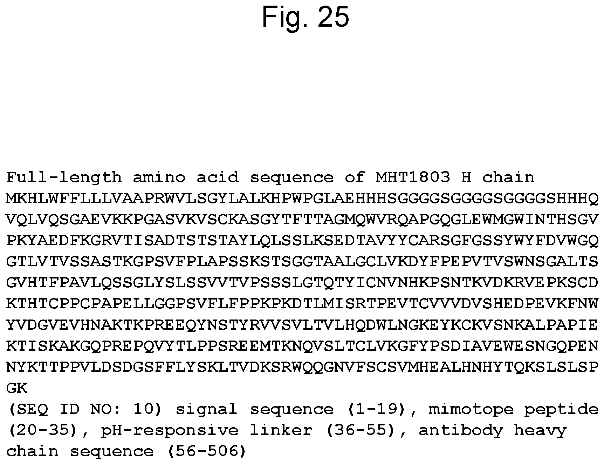

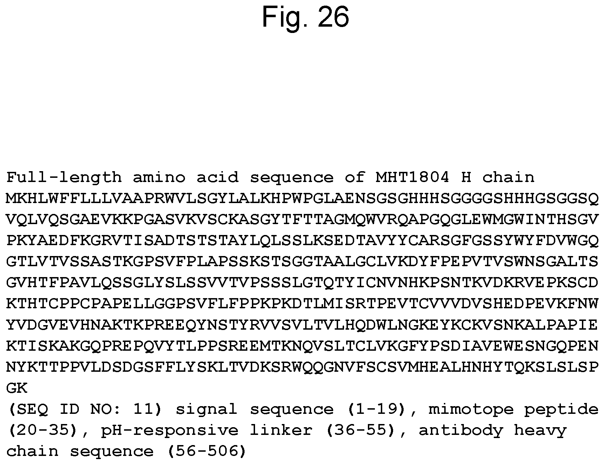

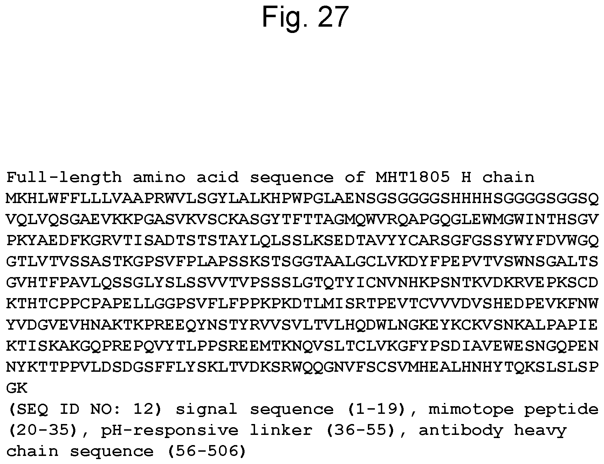

MHT1008 comprising no His as shown in Figure 6-1 (A) exhibited an equivalent binding intensity under neutral pH conditions (pH 7.5) and under acidic pH conditions (pH 5.5). In contrast, MHT1803 (Table 2, SEQ ID NO: 10, Figure 25), MHT1804 (Table 2, SEQ ID NO: 11, Figure 26), and MHT1805 (Table 2, SEQ ID NO: 12, Figure 27) each comprising 1 or 2 consecutive His residues (the His cluster) in the linker exhibited a higher binding intensity under acidic pH conditions than under neutral pH conditions (Figure 6-1 (B), Figure 6-2 (C), and Figure 6-2 (D)). The EC₅₀ ratios (neutral pH conditions/acidic pH conditions) of binding intensity of MHT1803, MHT1804, and MHT1805 were 3.9, 3.6, and 2.3, respectively. Also, MHT1806 comprising 3 His clusters (Table 2, SEQ ID NO: 13, Figure 28) exhibited a high binding intensity under acidic pH conditions (Figure 6-3 (E)), and the EC₅₀ ratio (neutral pH conditions/acidic pH conditions) thereof was 38.8.

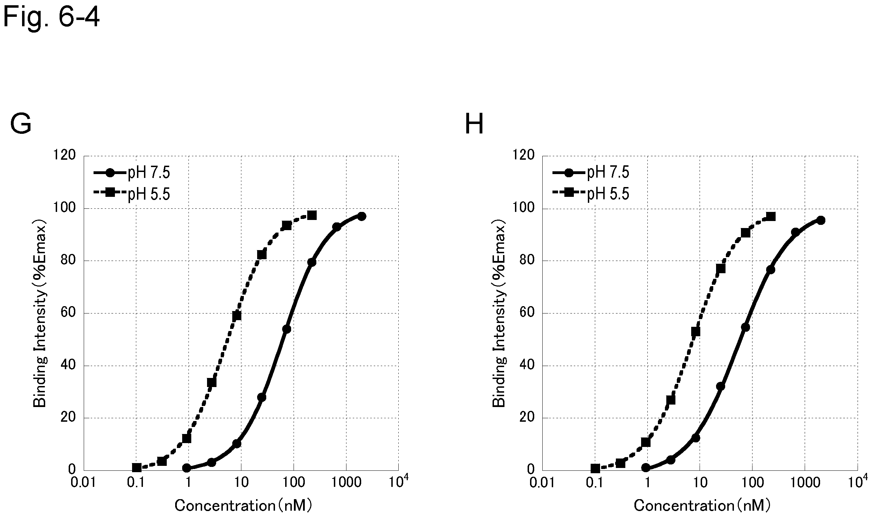

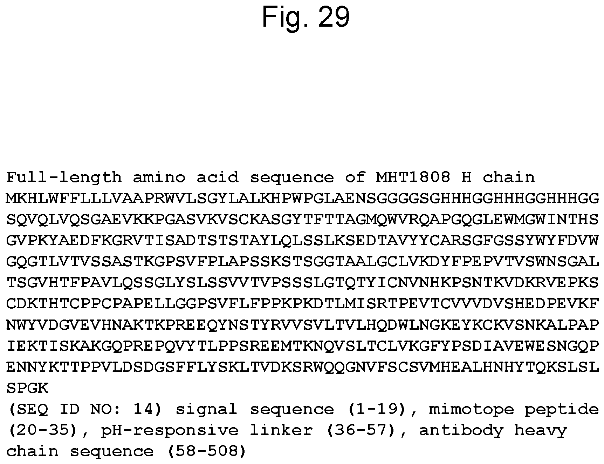

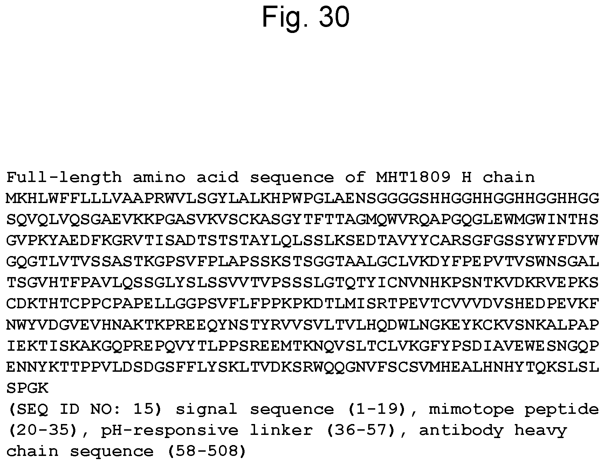

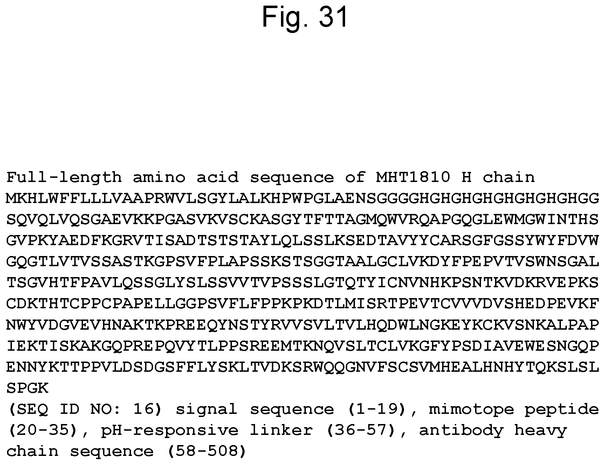

In order to examine the number of consecutive His residues forming the His cluster, the binding intensities of MHT1808 (Table 2, SEQ ID NO: 14, Figure 29), MHT1809 (Table 2, SEQ ID NO: 15, Figure 30), and MHT1810 (Table 2, SEQ ID NO: 16, Figure 31) were evaluated. All the clones exhibited higher binding intensities under acidic pH conditions than under neutral pH conditions (Figure 6-3 (F), Figure 6-4 (G), and Figure 6-4 (H)), and EC₅₀ ratios (neutral pH conditions/acidic pH conditions) thereof were 24.4, 11.4, and 7.8, respectively.

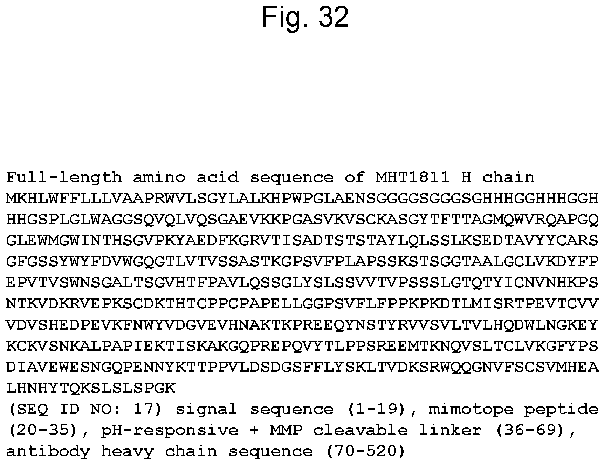

The binding intensity of MHT1811 (Table 2, SEQ ID NO: 17, Figure 32) comprising the known MMP substrate (Biopolymers; 40 (4): 399-416, 1996) composed of the His cluster and PLGLWA (SEQ ID NO: 40, Figure 51) was evaluated. When MHT1811 comprised the protease substrate, MHT1811 exhibited a higher binding intensity under acidic pH conditions than under neutral pH conditions (Figure 6- 5(I)), and the EC₅₀ ratio (neutral pH conditions/acidic pH conditions) thereof was 28.1. MHT1811 exhibited a high binding intensity under the condition with the addition of MMP1, and the EC₅₀ ratio (without the addition of MMP1/with the addition of MMP1) thereof was 48.4.

Acidic pH conditions under which the binding intensity would be enhanced were analyzed. As shown in Figure 6-5 (J), MHT1806 exhibited an enhanced binding intensity under acidic conditions at a pH 6.5 or lower than neutral pH conditions (pH 7.5). Also, MHT1808 exhibited an enhanced binding intensity under acidic conditions at a pH 6.0 or lower than neutral pH conditions (pH 7.5) (Figure 6-6 (K)).

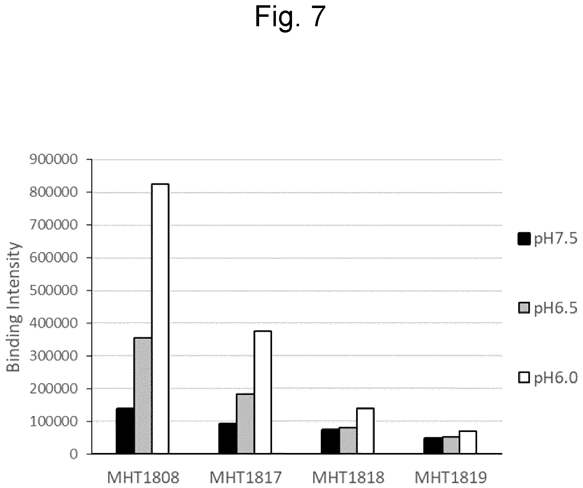

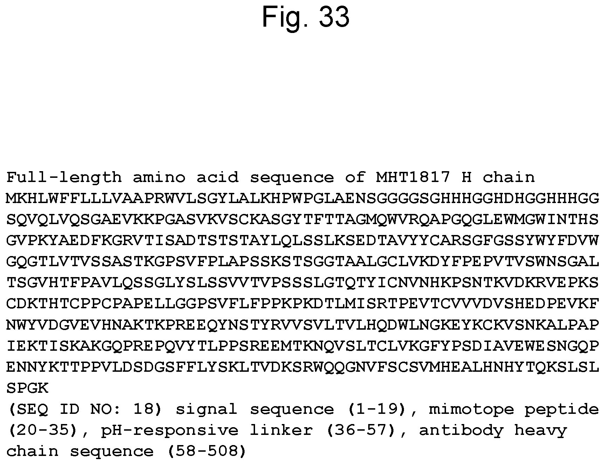

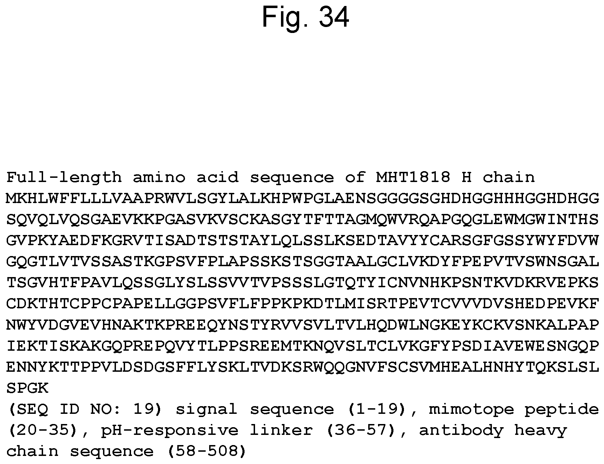

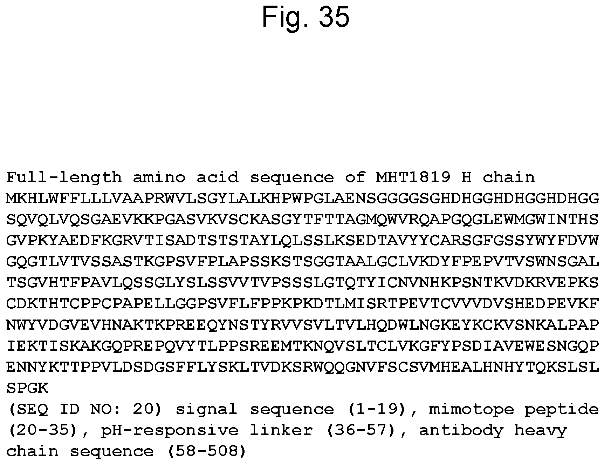

In order to examine as to whether or not protonation of His residues would enhance the binding intensity depending on pH lowering, MHT1817 (Table 2, SEQ ID NO: 18, Figure 33), MHT1818 (Table 2, SEQ ID NO: 19, Figure 34), and MHT1819 (Table 2, SEQ ID NO: 20, Figure 35) each comprising negatively charged Asp residues in the His cluster were designed and prepared. The antibodies were diluted to 20 nM with buffers (pH 7.5, pH 6.5, and pH 6.0), the reaction was allowed to proceed at 37°C for 30 minutes, and the binding activities thereof to the human TROP2 antigen were evaluated by ELISA. While MHT1808 exhibited an enhanced binding intensity as a pH was lowered, at a pH 6.5 or 6.0, the binding activities of MHT 1817, MHT1818, and MHT1819 were not enhanced as much as the binding intensity of MHT1808 (Figure 7). As the number of Asp residues in the His cluster was increased, a change in the binding intensity in response to a pH change was decreased. The results demonstrate that the pH-responsive linker does not preferably comprise Asp residues.

### (Example 6) Evaluation of binding activities of anti-CD98 antibody, anti-EGFR antibody, anti-GPRC5D antibody

### 6)-1 Evaluation of binding intensity of anti-CD98 antibody hM23H1L1

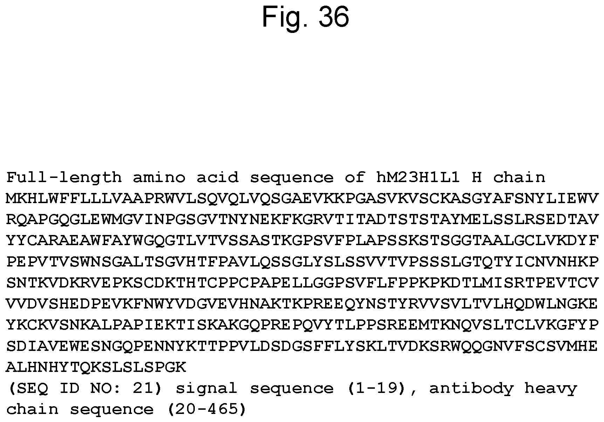

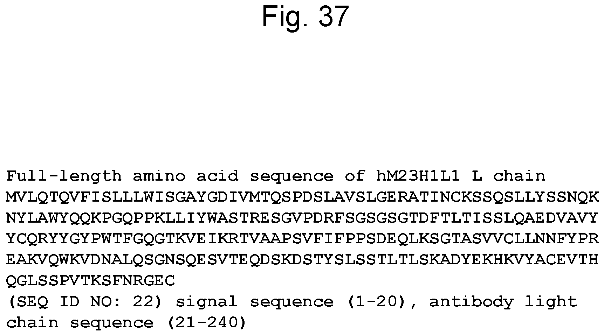

The binding intensity of the known anti-CD98 antibody hM23H1L1 described in WO 2015/146132 (heavy chain sequence (Table 3, SEQ ID NO: 21, Figure 36), light chain sequence (Table 3, SEQ ID NO: 22, Figure 37)) to the human CD98 antigen was evaluated by ELISA.

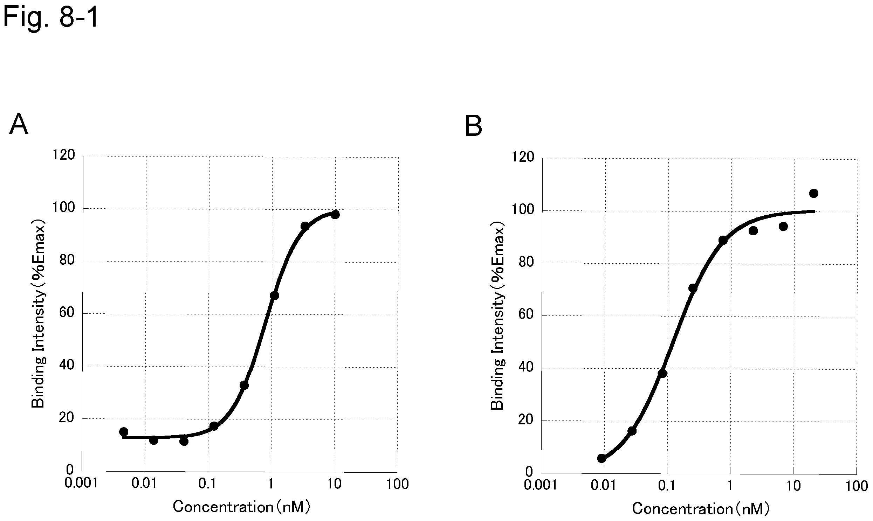

NeutrAvidin (Thermo Fisher Scientific) diluted to 1 µg/ml with PBS was added at 50 µl/well to a 96-well Maxi-sorp plate (Black, Nunc) and immobilized at 4°C overnight. The plate was washed with PBS (ELISA buffer) containing 0.05% Tween-20 (BioRad) and then blocked with Blocker Casein (Thermo Fisher Scientific). The plate was washed with ELISA buffer, the biotinylated human CD98 antigen (Accession Number: P08195; the extracellular domain was purified by the method known to a person skilled in the art and then biotinylated with EZ-Link NHS-PEG4-Bioin (Thermo Fisher Scientific)) diluted to 1 µg/ml with PBS was added at 50 µl/well, and the resultant was agitated for 30 minutes at room temperature. The plate was washed with ELISA buffer, the ELISA-buffered anti-CD98 antibody hM23H1L1 was added at 50 µl/well, and the resultant was then agitated for 30 minutes at room temperature. The plate was washed with ELISA buffer, 50 µl of the horseradish peroxidase (HRP)-labeled anti-human IgG antibody (Jackson Immuno Research Laboratories) diluted to 2,500-fold with ELISA buffer was added, and the resultant was then agitated for 30 minutes at room temperature. The plate was washed with ELISA buffer, the SuperSignal Pico ELISA chemiluminescent substrate (Thermo Fisher Scientific) was added, and chemiluminescence 10 minutes thereafter was then assayed using a plate reader. As shown in Figure 8-1 (A), hM23H1L1 was found to have bound to the human CD98 antigen in a concentration-dependent manner.

### 6)-2 Preparation of anti-EGFR antibody Cetuximab and evaluation of binding intensity thereof

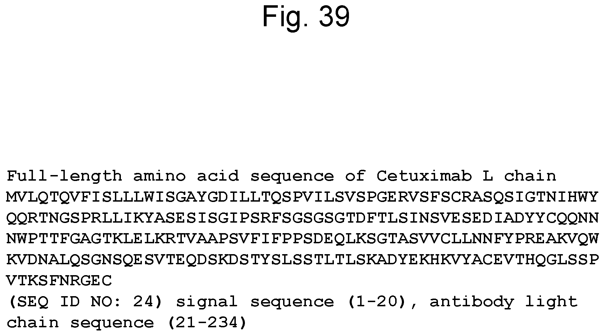

The conventional anti-EGFR antibody Cetuximab (Table 3, the heavy chain sequence (Figure 23, SEQ ID NO: 38); the light chain sequence (SEQ ID NO: 24, Figure 39)) was prepared in the same manner as in Example 1)-1, and the binding intensity thereof to human EGFR was evaluated by ELISA.

The human EGFR-Fc (a fusion protein of the extracellular domain of human EGFR (Accession Number: P00533) and the human IgG1 Fc region (Accession Number: P01857) purified by a method known to a person skilled in the art) diluted to 0.2 µg/ ml with PBS was added at 50 µl/well to a 96-well Maxi-sorp plate (Black, Nunc) and immobilized at 4°C overnight. The plate was washed with PBS (ELISA buffer) containing 0.05% Tween-20 (BioRad) and then blocked with Blocker Casein (Thermo Fisher Scientific). The plate was washed with ELISA buffer, Cetuximab prepared with MMP buffer was added at 50 µl/well, and the resultant was agitated for 30 minutes at room temperature. The plate was washed with ELISA buffer, 50 µl of the horseradish peroxidase (HRP)-labeled anti-human IgG antibody (Jackson Immuno Research Laboratories) diluted to 2,500-fold with ELISA buffer was added, and the resultant was then agitated for 30 minutes at room temperature. The plate was washed with ELISA buffer, the SuperSignal Pico ELISA chemiluminescent substrate (Thermo Fisher Scientific) was added, and chemiluminescence 10 minutes thereafter was then assayed using a plate reader. As shown in Figure 8-1 (B), Cetuximab was found to have bound to the human EGFR antigen in a concentration-dependent manner.

### 6)-3 Preparation of anti-GPRC5D antibody and evaluation of binding intensity thereof

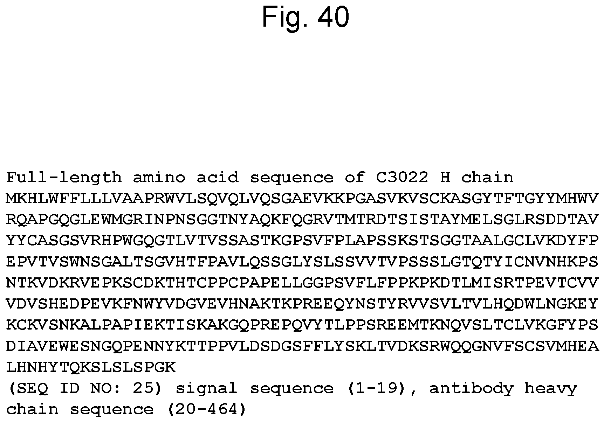

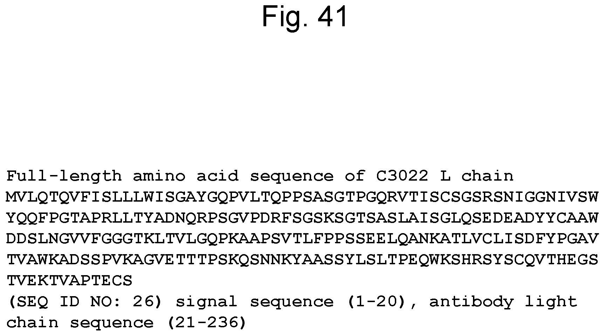

The conventional anti-GPRC5D antibody C3022 described in WO 2018/147245 (Table 3; the heavy chain sequence (SEQ ID NO: 25, Figure 40); the light chain sequence (SEQ ID NO: 26, Figure 41)) was prepared in the same manner as in Example 1)-1, and the binding intensity thereof to the humanGPRC5D antigen was evaluated by ELISA.

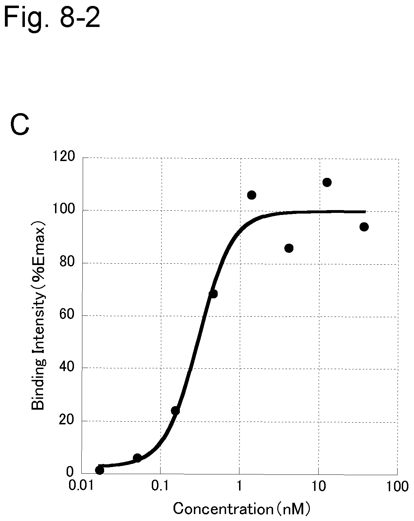

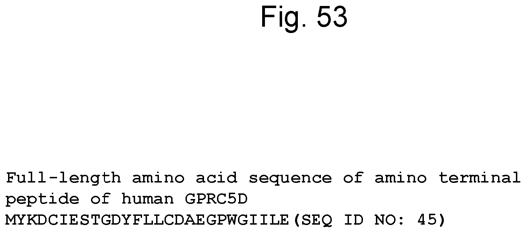

NeutrAvidin (Thermo Fisher Scientific) diluted to 1 µg/ml with PBS was added at 50 µl/well to a 96-well Maxi-sorp plate (Black, Nunc) and immobilized at 4°C overnight. The plate was washed with PBS (ELISA buffer) containing 0.05% Tween-20 (BioRad) and then blocked with Blocker Casein (Thermo Fisher Scientific). The plate was washed with ELISA buffer, the biotinylated amino terminal peptide of human GPRC5D (MYKDCIESTGDYFLLCDAEGPWGIILE (SEQ ID NO: 45, Figure 53)-K(Biotin)-NH₂, Peptide Institute, Inc.)) diluted to 1 µg/ml with PBS was added at 50 µl/well, and the resultant was agitated for 30 minutes at room temperature. The plate was washed with ELISA buffer, the MMP-buffered antibody was added at 50 µl/well, and the resultant was then agitated for 30 minutes at room temperature. The plate was washed with ELISA buffer, 50 µl of the horseradish peroxidase (HRP)-labeled anti-human IgG antibody (Jackson Immuno Research Laboratories) diluted to 2,500-fold with ELISA buffer was added, and the resultant was then agitated for 30 minutes at room temperature. The plate was washed with ELISA buffer, the SuperSignal Pico ELISA chemiluminescent substrate (Thermo Fisher Scientific) was added, and chemiluminescence 10 minutes thereafter was then assayed using a plate reader. As shown in Figure 8-2 (C), C3022 was found to have bound to the human GPRC5D antigen in a concentration-dependent manner. In Table 3, the "pH responsive linker" in the heavy chain sequence or light chain sequence of the masked antibody corresponds to the second peptide in the target-antigen-binding molecule according to the present invention.

**Table 3: Amino acid sequence information of anti-CD98 antibody, anti-EGFR antibody, and anti-GPRC5D antibody**

| Masked antibody | Heavy chain sequence | Light chain sequence |
|---|---|---|
| hM23H1L1 | hM23H1L1 (SEQ ID NO: 21, Fig. 36) | hM23H1L1 (SEQ ID NO: 22, Fig. 37) |
| Cetuximab | Cetuximab (SEQ ID NO: 23, Fig. 38) | Cetuximab (SEQ ID NO: 24, Fig. 39) |
| C3022 | C3022 (SEQ ID NO: 25, Fig. 40) | C3022 (SEQ ID NO: 26, Fig. 41) |
| MhM8001 | hM23H1L1 (SEQ ID NO: 21, Fig. 36) | MhM8001 (SEQ ID NO: 27, Fig. 42) |
| MCE-2101 | Cetuximab (SEQ ID NO: 23, Fig. 38) | MCE-2101 (SEQ ID NO: 28, Fig. 43) |
| MC3-9001 | MC3-9001 (SEQ ID NO: 29, Fig. 44) | C3022 (SEQ ID NO: 26, Fig. 41) |
| hM23-M1 | hM23-M1 (SEQ ID NO: 31, Fig. 46) | hM23-M1 (SEQ ID NO: 32, Fig. 47) |
| MhM1013 | hM23H1L1 (SEQ ID NO: 21, Fig. 36) | MhM1013 (SEQ ID NO: 33, Fig. 48) |
| MhM1013-M1 | hM23-M1 (SEQ ID NO: 31, Fig. 46) | MhM1013 (SEQ ID NO: 33, Fig. 48) |
| MhM1018-M1 | hM23-M1 (SEQ ID NO: 31, Fig. 46) | MhM1018 (SEQ ID NO: 34, Fig. 49) |
| MhM1024-M1 | hM23-M1 (SEQ ID NO: 31, Fig. 46) | MhM 1024 (SEQ ID NO: 35, Fig. 50) |

### (Example 7) Concentration of mimotope peptide binding to hM23H1L1, Cetuximab, and C3022

### 7)-1 Concentration of mimotope peptide binding to hM23H1L1

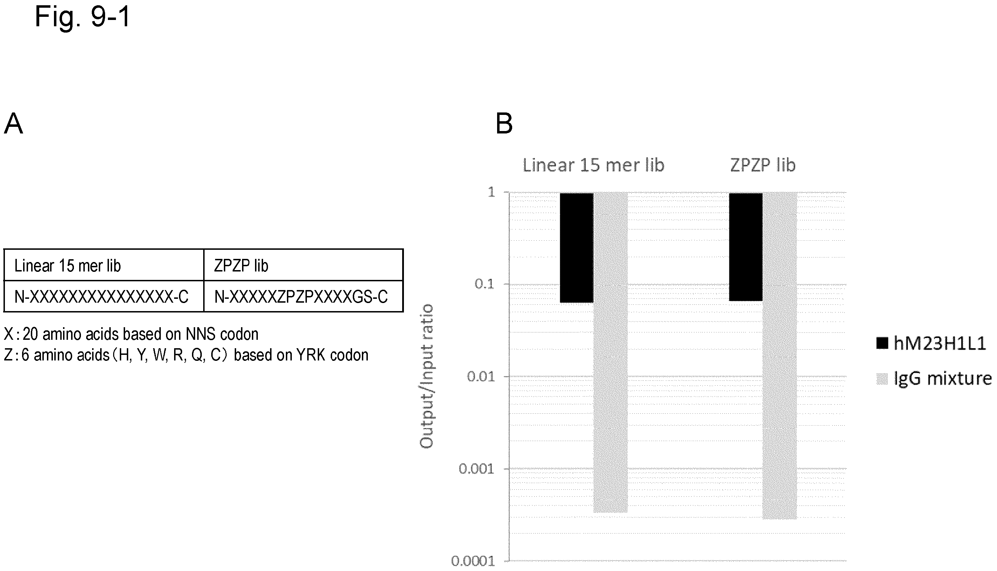

A peptide library composed of completely random 15 amino acids (Linear 15mer lib) or a peptide library by ribosome display having a repeat motif of aromatic amino acid and Pro (ZPZP motif) near the center (ZPZP lib) were constructed, and a peptide capable of binding to hM23H1L1 was concentrated (Figure 9-1 (A)). At the outset, RDs were added to Dynabeads Streptavidin M-280 (Thermo Fisher Scientific) and Dynabeads Protein A (Thermo Fisher Scientific) each comprising human serum-derived IgG (Sigma Aldrich) biotinylated with EZ-Link NHS-PEG4-Bioin (Thermo Fisher Scientific) immobilized thereon and allowed to react with the human serum-derived IgG or the Protein A. RDs that did not bind were collected using a magnet stand (DynaMag-2, Thermo Fisher Scientific) and then allowed to react with the Dynabeads Protein A comprising hM23H1L1 bound thereto. RDs that did not bind to hM23H1L1 were removed by washing with the use of a magnet stand, and mRNAs were purified from RDs that had bound to hM23H1L1. Thereafter, RDs were prepared again by RT-PCR and *in vitro* translation. This process of panning was performed 3 times.

mRNAs after the third round of panning were subjected to *in vitro* translation to prepare RDs, and the prepared RDs were then allowed to react with Dynabeads Protein A comprising hM23H1L1 or human serum-derived IgG immobilized thereon (amount of input: 6 × 10¹¹). RDs that did not bind were removed by washing with the use of a magnet stand, mRNAs were collected from the RDs that had bound, and the amount collected (output) was quantified and evaluated by RT-qPCR. As shown in Figure 9-1 (B), the number of mRNAs collected under the hM23H1L1-immobilized conditions was approximately 200 times greater than that collected under the human serum IgG-immobilized conditions from both the peptide libraries Linear 15mer lib and ZPZP lib. The results indicate concentration of peptides binding specifically to hM23H1L1.

### 7)-2 Concentration of mimotope peptide binding to Cetuximab

A peptide library by ribosome display composed of completely random 15 amino acids (Linear 15mer lib) was used to concentrate peptides capable of binding to Cetuximab. At the outset, RDs were added to Dynabeads Protein A (Thermo Fisher Scientific) and Dynabeads Protein A (Thermo Fisher Scientific) comprising human serum-derived IgG (Sigma Aldrich) immobilized thereon and allowed to react with Protein A or human serum-derived IgG. RDs that did not bind were collected using a magnet stand (DynaMag-2, Thermo Fisher Scientific) and then allowed to react with the Dynabeads Protein A comprising Cetuximab bound thereto. RDs that did not bind to Cetuximab were removed by washing with the use of a magnet stand, and mRNAs were purified from RDs that had bound to Cetuximab. Thereafter, RDs were prepared again by RT-PCR and *in vitro* translation. This process of panning was performed 4 times.

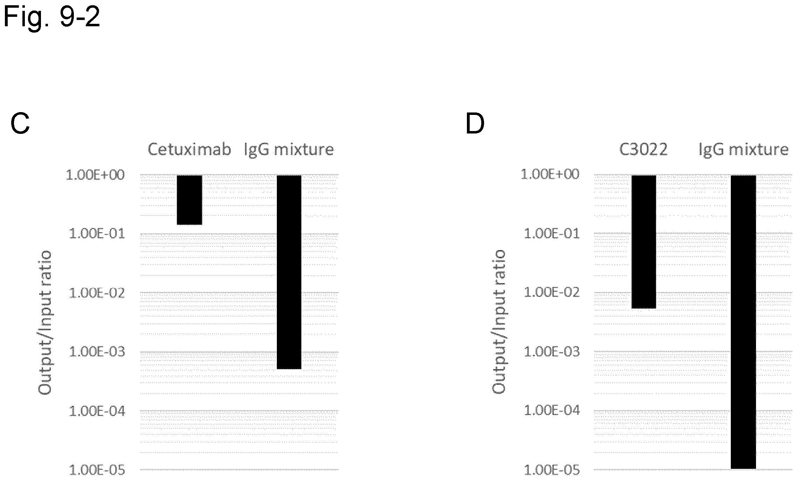

mRNAs after the fourth round of panning were subjected to *in vitro* translation to prepare RDs, and the prepared RDs were then allowed to react with Dynabeads Protein A comprising Cetuximab or human serum-derived IgG immobilized thereon (amount of input: 6 × 10¹¹). RDs that did not bind were removed by washing with the use of a magnet stand, mRNAs were collected from the RDs that had bound, and the amount collected (output) was quantified and evaluated by RT-qPCR. As shown in Figure 9-2 (C), the number of mRNAs collected under the Cetuximab-immobilized conditions was 276 times greater than that collected under the human serum IgG-immobilized conditions. The results indicate concentration of peptides binding specifically to Cetuximab.

### 7)-3 Concentration of mimotope peptide binding to C3022

A peptide library by ribosome display composed of completely random 15 amino acids (Linear 15mer lib) was used to concentrate peptides capable of binding to C3022. At the outset, RDs were added to Dynabeads Protein A (Thermo Fisher Scientific) and Dynabeads Protein A (Thermo Fisher Scientific) comprising human serum-derived IgG (Sigma-Aldrich) immobilized thereon and allowed to react with Protein A or human serum-derived IgG. RDs that did not bind were collected using a magnet stand (DynaMag-2, Thermo Fisher Scientific) and then allowed to react with the Dynabeads Protein A comprising C3022 bound thereto. RDs that did not bind to C3022 were removed by washing with the use of a magnet stand, and mRNAs were purified from RDs that had bound to C3022. Thereafter, RDs were prepared again by RT-PCR and *in vitro* translation. This process of panning was performed 3 times.

mRNAs after the third round of panning were subjected to *in vitro* translation to prepare RDs, and the prepared RDs were then allowed to react with Dynabeads Protein A comprising C3022 or human serum-derived IgG immobilized thereon (amount of input: 6 × 10¹¹). RDs that did not bind were removed by washing with the use of a magnet stand, mRNAs were collected from the RDs that had bound, and the amount collected (output) was quantified and evaluated by RT-qPCR. As shown in Figure 9-2 (D), the number of mRNAs collected under the C3022-immobilized conditions was 503 times greater than that collected under the human serum IgG-immobilized conditions. The results indicate concentration of peptides binding specifically to C3022.

### (Example 8) General applicability of pH-responsive linker

In the same manner as in Example 3, mimotope peptides capable of inhibiting binding of the anti-CD98 antibody and the anti-GPRC5D antibody were selected. Concerning the anti-EGFR antibody Cetuximab, mimotopes were selected in the IgG form instead of the scFv form. In the same manner as in Example 3, a DNA fragment encoding a random peptide portion was digested with a restriction enzyme, purified, and then inserted into a mammalian cell expression vector, so that the secretory signal sequence, a DNA fragment, an MMP cleavable linker, and Cetuximab (the heavy chain or the light chain) would be translated in that order. Cetuximab comprising a peptide and an MMP cleavable linker fused to the N terminus of the heavy chain or the light chain was expressed in the Expi293F cell, and mimotope peptides capable of inhibiting binding of Cetuximab were selected.

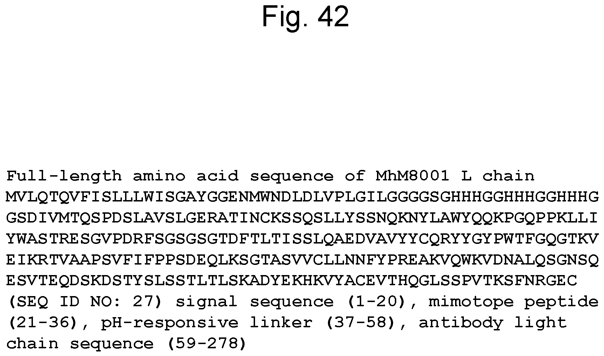

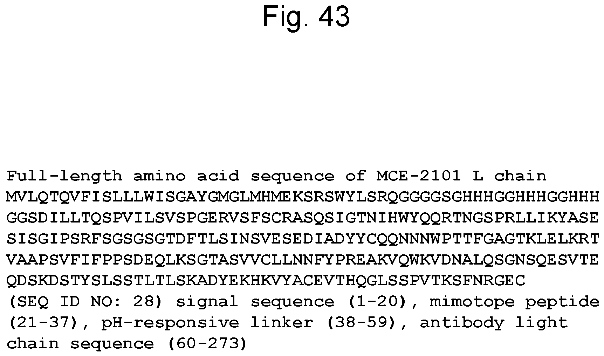

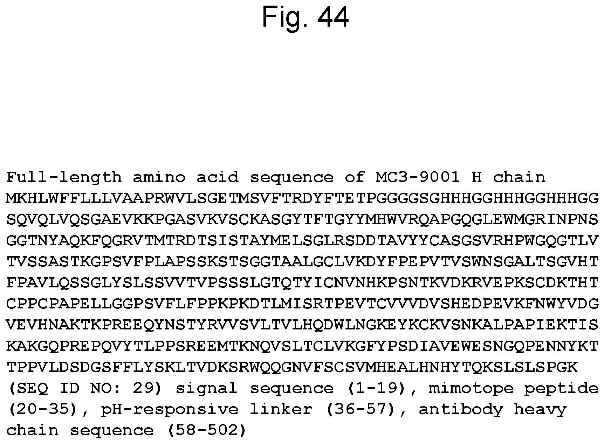

With the use of the pH-responsive linker comprising 3 His clusters, whether or not masked antibodies would be generally activated under acidic pH conditions was examined. The anti-CD98 masked antibody MhM8001 (Table 3, SEQ ID NO: 27, Figure 42), the anti-EGFR masked antibody MCE-2101 (Table 3, SEQ ID NO: 28, Figure 43), and the anti-GPRC5D masked antibody MC3-9001 (Table 3, SEQ ID NO: 29, Figure 44) each comprising the mimotope identified by the screening above were designed. The masked antibodies were prepared in the same manner as in Example 1)-1, and the binding intensity thereof under neutral pH conditions and under acidic pH conditions was evaluated by ELISA in the same manner as in Example 5 and Example 6.

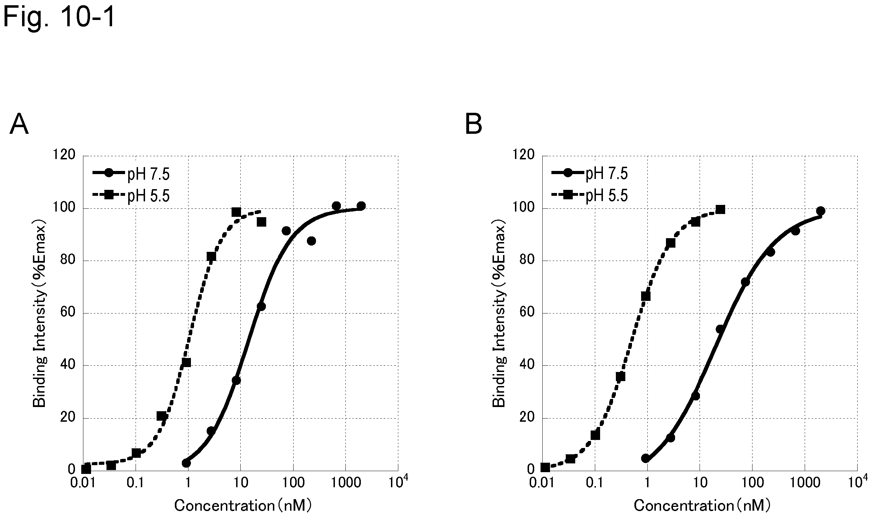

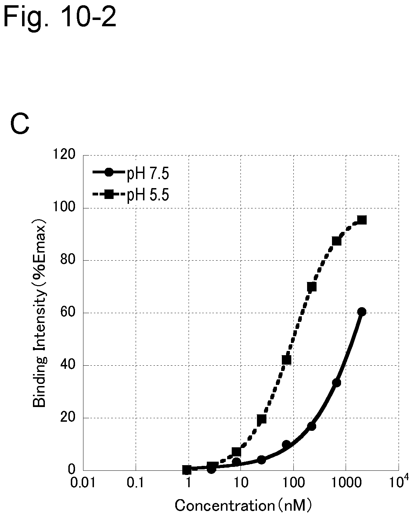

MhM8001, MCE-2101, and MC3-9001 exhibited higher binding activities under acidic pH conditions (pH 5.5) than under neutral pH conditions (pH 7.5), and the EC₅₀ ratios (neutral pH conditions/acidic pH conditions) thereof were 13.1, 39.0, and 40.4, respectively (Figure 10-1 (A), Figure 10-1 (B), and Figure 10-2 (C)). The results demonstrate that the masked antibodies comprising the His cluster would generally be activated under acidic pH conditions.

### (Example 9) Preparation of anti-TROP2 masked antibody subjected to ADC preparation and evaluation of binding intensity thereof

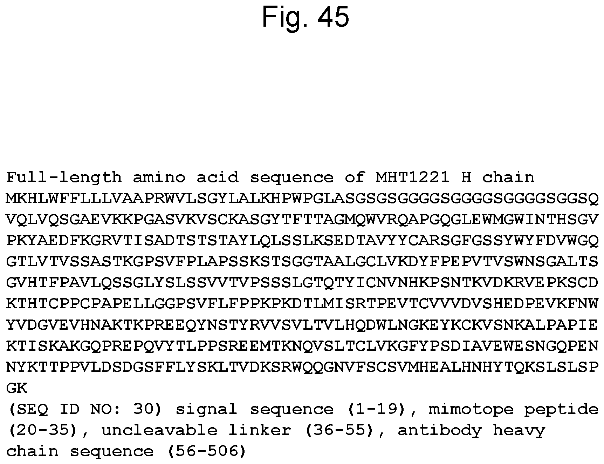

In order to examine as to whether or not cytotoxic activity of a masked antibody comprising the His cluster in a linker portion would be enhanced as a pH was lowered, the anti-TROP2 masked antibody MHT1808 comprising the His cluster and MHT1221 (Table 2, SEQ ID NO: 30, Figure 45) and MHT1008 not comprising the His cluster were designed. The antibodies were prepared in the same manner as in Example 1)-1, and the binding intensity thereof to the human TROP2 antigen under neutral pH conditions (pH 7.5) and under acidic pH conditions (pH 5.5) were evaluated by ELISA in the same manner as in Example 5.

As shown in Figure 11, MHT1808 exhibited a higher binding intensity under acidic pH conditions than under neutral pH conditions, and the EC₅₀ ratio (neutral pH conditions/acidic pH conditions) thereof was 25.4. In contrast, MHT1221 and MHT1008 exhibited an equivalent binding intensity under both pH conditions, and the EC₅₀ ratios (neutral pH conditions/acidic pH conditions) thereof were 1.7 and 1.2, respectively.

### (Example 10) Production of antibody-drug conjugate

As drug linkers to produce antibody-drug conjugates, N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10, 13-dioxo-2,3,9, 10, 13, 15-hexahydro-1H, 12H-benzo[de]pyrano[3',4':6,7]indolizino[1,2-b]quinolin-1-yl]amino}-2-oxoethoxy)methyl]glycinamide (Example 58, Step 8, WO 2014/057687, hereafter, referred to as "Linker 1") and N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alanineamide (Example 2-1, WO 2020/196474, hereafter, referred to as "Linker 2") were used.

### Common operation for production and identification of antibody-drug conjugate

### Common operation A: Concentration of aqueous solution of antibody and aqueous solution of antibody-drug conjugate

Aqueous solutions were concentrated using Amicon Ultra (50,000 MWCO, Millipore Corporation) and Allegra X-15R Centrifuge (Beckman Coulter) (SX4750A) (4000 g).

### Common operation B: Measurement of antibody concentration

Antibody concentration was measured with the use of the UV photometer (Nanodrop 1000, Thermo Fisher Scientific Inc.) in accordance with the manufacturer's instructions.

### Common operation C: Antibody buffer exchange

### C-1: Buffer exchange with phosphate buffer comprising NaCl (50 mM) and EDTA (5 mM) (50 mM, pH 6.0) (hereafter, referred to as "PBS 6.0/EDTA")

The NAP-25 columns (Cat. No. 17085202, Cytiva, NAP-25 Columns Sephadex, hereafter referred to as "NAP-25") were equilibrated with phosphate buffer comprising NaCl (50 mM) and EDTA (5 mM) (50 mM, pH 6.0) (hereafter, referred to as "PBS 6.0/EDTA") in accordance with the manufacturer's instructions. To a NAP-25 column, 2.5 ml of an aqueous antibody solution was applied, and a fraction (3.5 ml) was eluted with the use of 3.5 ml of PBS 6.0/EDTA. In accordance with the common operation A and the common operation B, this fraction was concentrated, and the concentration thereof was measured. Thereafter, the concentration was adjusted to 10 mg/ml with the use of PBS 6.0/EDTA.

### C-2: Buffer exchange with phosphate buffer (50 mM, pH 6.0) (hereafter, referred to as "PB6.0")

PB6.0 was added to the aqueous antibody solution, and the aqueous solution was concentrated in accordance with the common operation A. This operation was repeated several times, the concentration was measured (the common operation B), and the concentration was adjusted to 10 mg/ml with the use of PB6.0.

### Common operation D: Purification of antibody-drug conjugate

An antibody fraction was eluted with the use of NAP-25 in accordance with the manufacturer's instructions. For column equilibration and elution, 5 (w/v)% sorbitol-10 mM acetate buffer (hereafter referred to as "ABS") was used.

### Common operation E: Measurement of antibody concentration in antibody-drug conjugate

The concentration C' (mg/ml) of the antibody-drug conjugate was determined in accordance with Equation (I): Absorbance A280 = molar absorption coefficient ε₂₈₀ (L·mol⁻¹·cm⁻¹) × molar concentration C (mol·L⁻¹) × cellular optical path length l (cm) based on the Lambert-Beer's law. $C ′ \left(mg⋅{mL}^{- 1}\right) = MW \left(g⋅{mol}^{- 1}\right) ⋅ C \left(mol⋅{L}^{- 1}\right) = \frac{{A}_{280} ⋅ MW \left(g⋅{mol}^{- 1}\right)}{{ε}_{280} \left(L⋅{mol}^{- 1}⋅{cm}^{- 1}\right) ⋅ l \left(cm\right)}$

Values used to calculate C' (mg/ml) were determined as described below.
- Absorbance A280: Actually measured UV absorbance of the aqueous antibody solution-drug conjugate at 280 nm
- Measurement was performed with the use of Lamba25 (PerkinElmer)
- Molar weight MW (g·mol⁻¹): Estimated antibody molecular weight determined based on the amino acid sequence of the antibody (used as the approximate value of the molar mass of the antibody-drug conjugate).
- Optical path length l (cm): 1 cm
- Molar absorption coefficient ε₂₈₀ of the antibody drug conjugate: determined in accordance with Equation (II) below
ε280 = molar absorption coefficient of antibody εAb,280 + molar absorption coefficient of drug εDL,280 × number of drugs bound
ε_{Ab,280}: the molar absorption coefficient of the antibody at 280 nm
ε_{DL,280}: the molar absorption coefficient of the drug linker at 280 nm
Number of drugs bound: determined in accordance with the common operation F (see the description below)
ε_{Ab,280} determined based on the amino acid sequence of the antibody in accordance with a known calculation method (Protein Science, 1995, vol. 4, 2411-2423) was used (see Table 4 below).

ε_{DL,280} of the Linker 1 was determined by allowing the Linker 1 to react with mercaptoethanol or N-acetylcysteine, converting a maleimide group into succinimidethioether to obtain a compound, and measuring a molar ab sorption coefficient (280 nm) of the compound. As ε_{DL,280} of the Linker 2, the actually measured molar absorption coefficient (280 nm) was used.

### Common operation F: Measurement of average number of drugs bound to an antibody molecule in an antibody-drug conjugate

The average number of drugs bound to an antibody molecule in an antibody-drug conjugate was measured by reversed-phase chromatography (RPC).

### F-1. Sample preparation (reduction of disulfide between antibody molecules)

After an aqueous solution of dithiothreitol (DTT) (100 mM, 15 µl) was added to the solution of the antibody-drug conjugate (approximately 1 mg/ml, 60 µl), the mixture was incubated at 37°C for 30 minutes, and the antibody's disulfide bonds between its light chains and heavy chains were cleaved.

### F-2. HPLC analysis

HPLC analysis was performed under the following conditions.
- HPLC system: Agilent1290 HPLC system (Agilent Technologies)
- Detector: ultraviolet photometer (measurement wavelength: 280 nm)
- Column: ACQUITY UPLC BEH Phenyl (2.1 × 50 mm, 1.7 µm, 130 Å; Waters, P/N186002884)
- Column temperature: 75°C
- Mobile phase A: 0.10 (v/v)% trifluoroacetic acid (TFA), 15 (v/v)% 2-propanol solution
- Mobile phase B: 0.075 (v/v)% TFA, 15 (v/v)% 2-propanol, acetonitrile solution
- Gradient program: (min, B%): (0, 14)-(15, 36)-(17, 80)-(17.01, 14)-(25, 14)
- Amount of sample injection: 10 µl

### F-3. Data analysis

### F-3-1

Compared to the antibody light chain to which no drug has bound (L0) and heavy chain to which no drug has bound (H0), the drug-bound light chain (a light chain comprising "i" number of drugs bound thereto: Li) and heavy chain (a heavy chain comprising "i" number of drugs bound thereto: Hi) exhibit enhanced hydrophobicity in proportion of the number of drugs bound thereto, and the retention time is prolonged. In comparison with the retention time of L0 and H0, accordingly, the detection peaks can be assigned to any of L0, L1, H0, H1, H2, and H3.

### F-3-2

Since a drug linker has UV absorption, the peak area values are corrected in accordance with the following formula with the use of the molar absorption coefficients of the light chain, the heavy chain, and the drug linker according to the number of binding of the drug linker.$\begin{matrix}Corrected peak area value of light chain comprising " i " number of drugs bound thereto \left({A}_{Li}\right) \\ = peak area \times \frac{molar absorption coefficient of light chain}{molar absorption coefficient of light chain + number of drugs bound \left(i\right) \times molar absorption coefficient of drug linker}\end{matrix}$ $\begin{matrix}Corrected peak area value of heavy chain comprising " i " number of drugs bound thereto \left({A}_{Hi}\right) \\ = peak area \times \frac{molar absorption coefficient of heavy chain}{molar absorption coefficient of heavy chain + number of drugs bound \left(i\right) \times molar absorption coefficient of drug linker}\end{matrix}$

The molar absorption coefficient (280 nm) of the light chain and that of the heavy chain of an antibody deduced based on the amino acid sequences of the light chain and the heavy chain of the antibody in accordance with the conventional method of calculation (Protein Science, 1995, vol. 4, 2411-2423) were used (see Table 4 below).

### F-3-3

An each chain peak area ratio (%) relative to the corrected total peak area value was determined in accordance with the equations below. $Peak area ratio of light chain comprising " i " number of drugs bound thereto = \frac{{A}_{Li}}{{A}_{L 0} + {A}_{L 1}} \times 100$ $Peak area ratio of heavy chain comprising " i " number of drugs bound thereto = \frac{{A}_{Hi}}{{A}_{H 0} + {A}_{H 1} + {A}_{H 2} + {A}_{H 3}} \times 100$ Corrected peak area of each of *A_{Li}, A_{Hi}*: Lᵢ, Hᵢ

### F-3-4

The average number of drugs bound to an antibody molecule in an antibody-drug conjugate was determined in accordance with the equation below. Average number of drugs bound = (L0 peak area ratio × 0 + L1 peak area ratio × 1 + H0 peak area ratio × 0 + H1 peak area ratio × 1 + H2 peak area ratio × 2 + H3 peak area ratio × 3) / 100 × 2

**Table 4: Molar absorption coefficient, absorption coefficient, and molecular weight of antibody**

| Antibody | Molar absorption coefficient at 280 nm (L·mol⁻¹·cm⁻¹) | | | Absorption coefficient (L·g⁻¹·cm⁻¹) | Molecular weight of antibody |
|---|---|---|---|---|---|
| | Whole | Heavy chain | Light chain | | |
| MhM1018-M1 | 254340 | 77008 | 50162 | 1.67 | 152093 |
| MhM1024-M1 | 254340 | 77008 | 50162 | 1.66 | 153582 |
| MHT1808 | 237380 | 90988 | 27702 | 1.56 | 152575 |
| MHT1221 | 237380 | 90988 | 27702 | 1.57 | 150888 |
| MHT1008 | 237380 | 90988 | 27702 | 1.57 | 151086 |

| Drug linker | Molar absorption coefficient at 280 nm (L·mol⁻¹·cm⁻¹) |
|---|---|
| Linker 1 | 5440 |
| Linker 2 | 23155 |

### 10)-1 Synthesis of MhM1018-M1-DXd-ADC

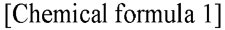

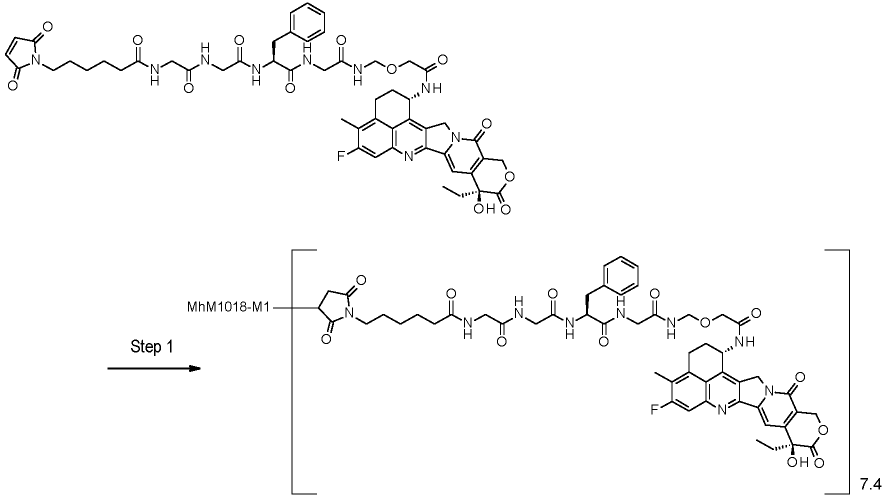

### Step 1: Antibody-drug conjugate (1)

### Antibody reduction:

A PBS6.0/EDTA solution of MhM1018-M1 produced in Example 12 was prepared in accordance with the common operation B and the common operation C-1 to be 10 mg/ml. To 1.45 ml of the resulting solution, an aqueous solution of 1 M potassium dihydrogen phosphate (Nacalai Tesque, Inc.; 0.0218 ml) and an aqueous solution of 10 mM TCEP (Tokyo Chemical Industry Co., Ltd.) (0.0581 ml; 6.0 molar equivalents per one molecule of the antibody) were added. The resultant was incubated at 37°C for 2 hours to reduce disulfide bonds between antibody chains.

### Conjugation of antibody to drug linker:

The solution was incubated at 15°C for 10 minutes. Subsequently, a solution of the Linker 1 in 10 mM dimethylsulfoxide (0.0969 ml; 10 molar equivalents per one molecule of the antibody) was added, and the mixture was incubated at 15°C for 1 hour to bind a drug linker to an antibody. Subsequently, an aqueous solution of 100 mM NAC (Sigma-Aldrich Co. LLC) (0.0097 ml; 10 molar equivalents per one molecule of the antibody) was added, the mixture was stirred, and the resultant was allowed to stand at room temperature for 20 minutes to terminate the reaction of the drug linker.

### Purification:

The solution was purified in accordance with the common operation D to obtain 7 ml of a solution comprising the title antibody-drug conjugate "MhM1018-M1-DXd-ADC."

### Property evaluation:

The property values indicated below were obtained in accordance with the common operation E and the common operation F.

### Antibody concentration: 1.75 mg/ml; antibody yield: 12.27 mg (83%); average number of drugs bound to an antibody molecule (n): 7.4

### 10)-2 Synthesis of MhM1024-M1-DXd-ADC

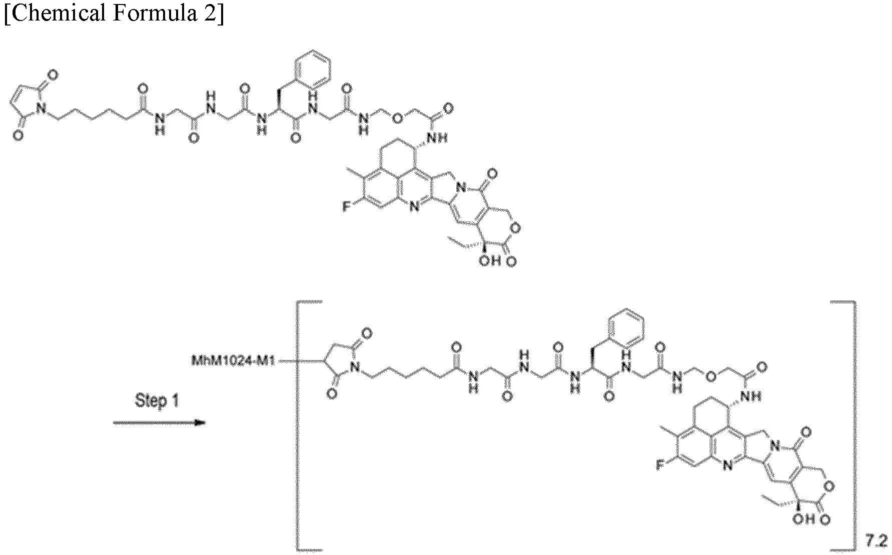

### Step 1: Antibody-drug conjugate (2)

With the use of 1.53 ml of MhM1024-M1 prepared in Example 12 (a 280 nm absorption coefficient (1.66 ml mg⁻¹ cm⁻¹) was used), the title antibody-drug conjugate "MhM1024-M1-DXd-ADC" was obtained in the same manner as in Step 1 of Example 10)-1.

### Property evaluation:

The property values indicated below were obtained in accordance with the common operation E and the common operation F.

### Antibody concentration: 1.91 mg/ml; antibody yield: 13.38 mg (87%); average number of drugs bound to an antibody molecule (n): 7.2

### 10)-3 Synthesis of MHT1221-PBD-ADC

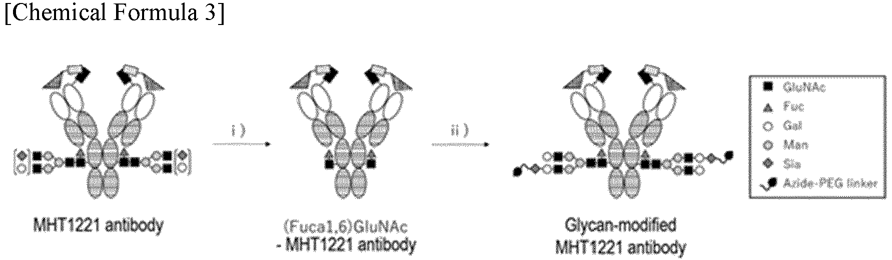

### i ) Endo S enzyme

### ii ) Endo S (D233Q/Q303L) enzyme, glycan oxazoline

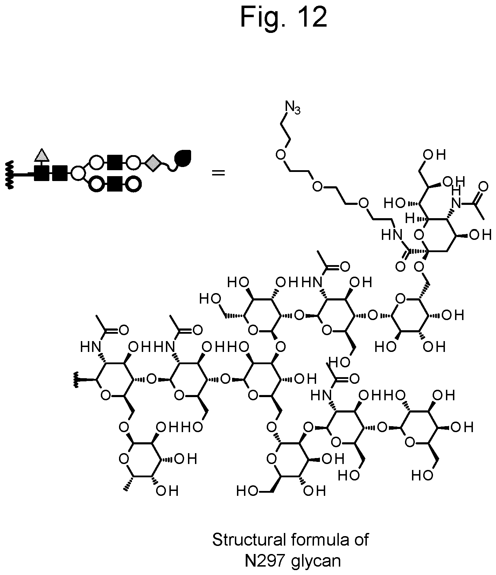

The N297 glycan of the glycan-modified antibody is the MSG1-type glycan comprising an azide group introduced into a sialic acid at the non-reducing terminus (WO 2019/065964) (Figure 12).

### Step 1: Preparation of (Fucα1,6)GlucNAc-MHT1221 antibody

The MHT1221 antibody solution prepared in Example 9 was buffer-exchanged to phosphate buffer (50 mM, pH 6.0) (hereafter referred to as "PB6.0") in accordance with the common operation C-2 to obtain 0.970 ml of a 19.3 mg/ ml antibody solution. To the resulting solution, 0.0124 ml of the Endo S solution (PBS, 7.52 mg/ml) was added, and the resultant was incubated at 37°C for 2 hours. After cleavage of the glycan was confirmed with the use of the Agilent 2100 bioanalyzer, the solution was purified with GST and a protein A column (AKTA). After the target fraction was substituted with PB6.0, the solution of (Fuca1,6)GlucNAc-MHT1221 antibody (20.0 mg/ml, 0.921 ml) was obtained.

### Step 2: Preparation of glycan-modified MHT1221 antibody

To the (Fuca 1, 6)GlucNAc-NMT 1221 antibody (PB6.0) (20.0 mg/ml, 0.921 ml) obtained in Step 1 above, a solution of glycan oxazoline (3aR,5R,6S,7R,7aR)-3a,6,7,7a-tetrahydro-7-hydroxy-5-(hydroxymethyl)-2-methyl-5H-pyrano[3,2-d]oxazol-6-yl O-[N5-acetyl-N1-[2-[2-[2-(2-azidoethoxy)ethoxy]ethoxy]ethyl]-α-neuraminamidosyl]-(2→6)-O-β-D-galactopyranosyl-(1→4)-O-2-(acetylamino)-2-deoxy-β-D-glucopyranosyl-(1→2)-O-α-D-mannopyranosyl-(1→3)-O-[O-β-D-galactopyranosyl-(1→4)-O-2-(acetylamino)-2-deoxy-β-D-glucopyranosyl-(1-2)-α-D-mannopyranosyl-(1→6)]-β-D-mannopyranoside (Example 56, WO 2019/065964, hereafter referred to as the "[N3-PEG(3)]-MSG1-Ox)" (Figure 12) (PB6.0) (50.0 mg/ml, 0.0561 ml) and the GST-Endo S D233Q/Q303L solution (WO 2017/010559) (PBS) (5.00 mg/ml, 0.0738 ml) were added, and the mixture was incubated at 30°C for 3 hours. After glycan transfer was confirmed with the use of the bioanalyzer, the solution was purified with GST and a CHT column (AKTA). A fraction comprising a target product was substituted with 10 mM acetate buffer solution, 5% sorbitol (pH 5.5) (hereafter referred to as "ABS") to obtain the glycan-modified MHT1221 antibody (10.6 mg/ml, 1.50 ml).

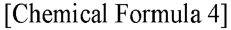

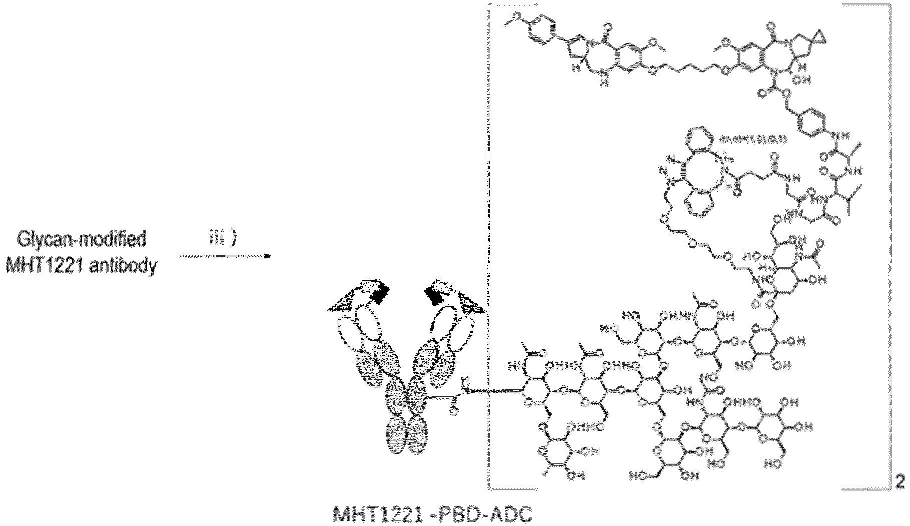

### iii) Linker 2

(m, n) in the structural formula comprise the (1 ,0) and/or (0, 1) components.

### Step 3: Conjugation of antibody to drug linker

To the glycan-modified MHT1221 (ABS) (10.6 mg/ml, 0.500 ml) obtained in Step 2 above, 1,2-propanediol (0.479 ml) and a solution of the Linker 2 in 10 mM dimethyl sulfoxide (0.0210 ml; 6 molar equivalents per one molecule of the antibody) were added at room temperature, and the reaction was allowed to proceed with the use of the tube rotator (MTR-103, AS ONE Corporation) at room temperature for 48 hours.

### Purification operation:

The solution was purified two times in accordance with the common operation D to obtain 2.50 ml of the MHT1221-PBD-ADC solution.

### Property evaluation:

The property values indicated below were obtained in accordance with the common operation E and the common operation F.

### Antibody concentration: 1.62 mg/ml; antibody yield: 4.06 mg (77%); average number of drugs bound to an antibody molecule (n): 1.9

### 10)-4 Synthesis of MHT1008-PBD-ADC

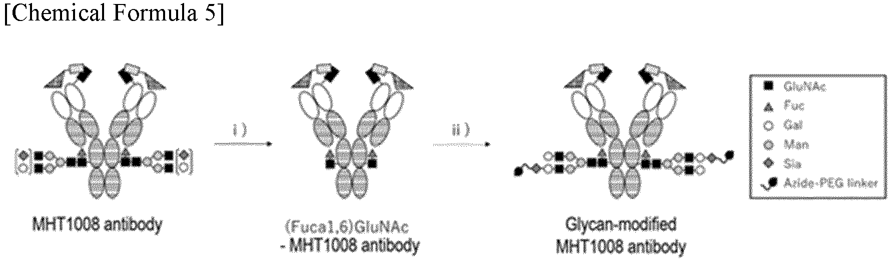

### i) Endo S enzyme

### ii) Endo S (D233Q/Q303L) enzyme, glycan oxazoline

The N297 glycan of the glycan-modified antibody is the MSG1-type glycan comprising an azide group introduced into a sialic acid at the non-reducing terminus (WO 2019/065964) (Figure 12).

### Step 1: Preparation of (Fucα1,6)GlcNAc-MHT1008 antibody

The MHT1008 antibody solution prepared in Example 4 was buffer-exchanged to PB6.0 and adjusted to ca. 16.6 mg/ml (0.880 ml). In the same manner as in Step 1 of Example 10)-3, the (Fucα1,6)GlcNAc-MHT1008 antibody solution (PB6.0) (16.8 mg/ml, 0.800 ml) was obtained.

### Step 2: Preparation of glycan-modified MHT1008 antibody

With the use of the (Fucα1,6)GlcNAc-MHT1008 antibody solution (PB6.0) (16.8 mg/ml, 0.800 ml) obtained in Step 1 above, the glycan-modified MHT1008 antibody (ABS) (11.4 mg/ml, 1.00 ml) was obtained in the same manner as in Step 2 of Example 10)-3.

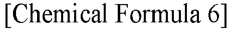

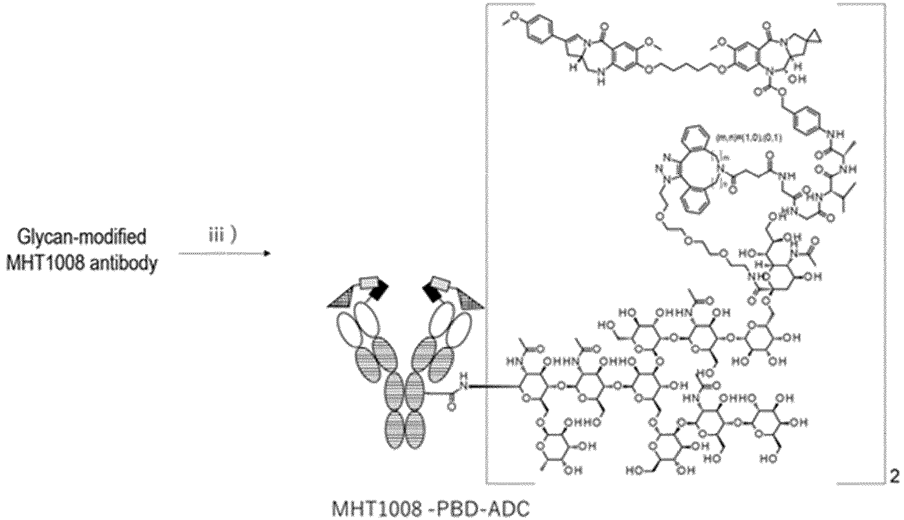

### iii) Linker 2

(m, n) in the structural formula comprise the (1 ,0) and/or (0, 1) components.

### Step 3: Conjugation of antibody to drug linker

With the use of the glycan-modified MHT1008 antibody (ABS) (11.4 mg/ml, 0.450 ml) obtained in Step 2 above, 2.50 ml of the MHT1008-PBD-ADC solution (ABS) was obtained in the same manner as in Step 3 of Example 10)-3.

### Property evaluation:

The property values indicated below were obtained in accordance with the common operation E and the common operation F.

### Antibody concentration: 1.55 mg/ml; antibody yield: 3.87 mg (77%); average number of drugs bound to an antibody molecule (n): 1.9

### 10)-5 Synthesis of MHT1808-PBD-ADC

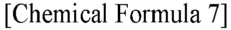

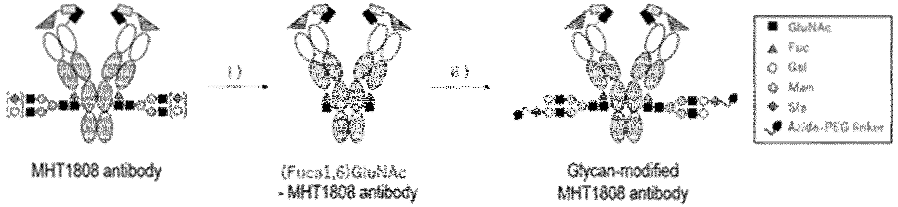

### i) Endo S enzyme

### ii) Endo S (D233Q/Q303L) enzyme, glycan oxazoline

The N297 glycan of the glycan-modified antibody is the MSG1-type glycan comprising an azide group introduced into a sialic acid at the non-reducing terminus (WO 2019/065964) (Figure 12).

### Step 1: Preparation of (Fucα1,6)GlcNAc-MHT1808 antibody

The MHT1808 antibody solution prepared in Example 9 was buffer-exchanged to PB6.0 and adjusted to ca. 19.1 mg/ml (1.00 ml). In the same manner as in Step 1 of Example 10)-3, the (Fucα1,6)GlcNAc-NMT1808 antibody solution (PB6.0) (17.1 mg/ml, 1.0 ml) was obtained.

### Step 2: Preparation of glycan-modified MHT1808 antibody

With the use of the (Fucα1,6)GlcNAc-MHT1008 antibody solution (PB6.0) (17.1 mg/ml, 1.00 ml) obtained in Step 1 above, the glycan-modified MHT1808 antibody (PBS7.9) (7.0 mg/ml, 1.60 ml) was obtained in the same manner as in Step 2 of Example 10)-3.

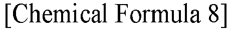

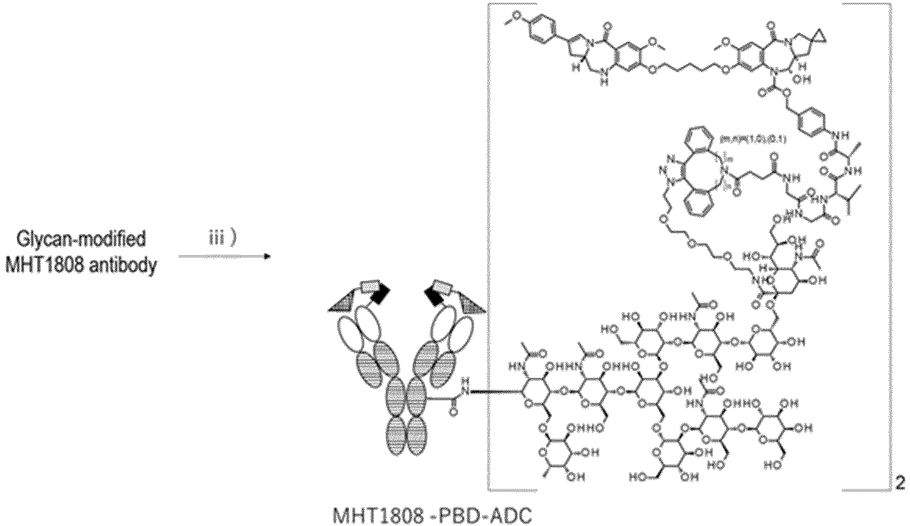

### iii) Linker 2

(m, n) in the structural formula comprise the (1 ,0) and/or (0, 1) components.

### Step 3: Conjugation of antibody to drug linker

With the use of the glycan-modified MHT1808 antibody (PBS7.4) (7.00 mg/ml, 0.450 ml) obtained in Step 2 above, 2.50 ml of the MHT1008-PBD-ADC solution (ABS) was obtained in the same manner as in Step 3 of Example 10)-3.

### Property evaluation:

The property values indicated below were obtained in accordance with the common operation E and the common operation F.

### Antibody concentration: 1.24 mg/ml; antibody yield: 4.35 mg (86%); average number of drugs bound to an antibody molecule (n): 1.9

### (Example 11) Masking effects of anti-CD98 antibody having different affinity 11)-1 Evaluation of binding affinity of anti-CD98 antibody

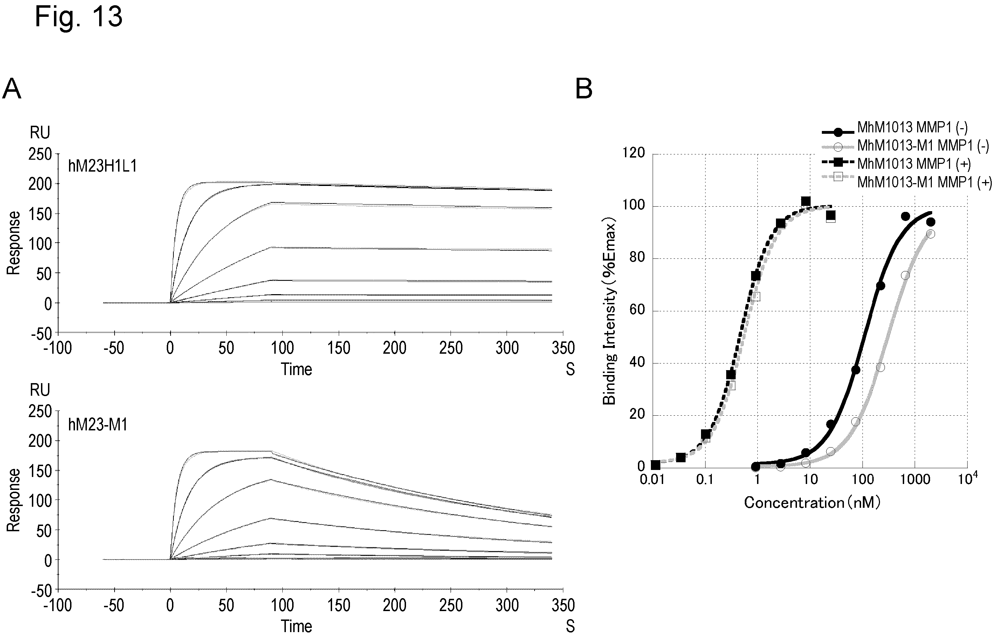

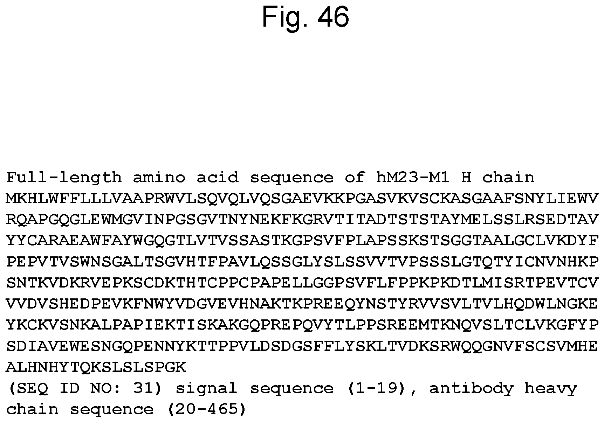

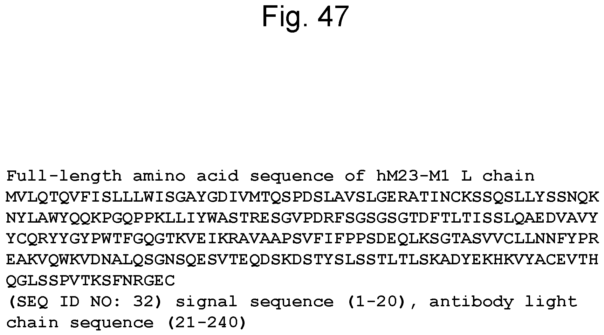

Biacore T200 was used to capture the anti-CD98 antibody (hM23H1L1 or hM23-M1 comprising a point mutation introduced into CDR1 of the H chain (Table 3, heavy chain sequence (SEQ ID NO: 31, Figure 46); light chain sequence (SEQ ID NO: 32, Figure 47)) as a ligand onto the immobilized anti-human IgG(Fc) antibody, and the CD98 antigen was analyzed as an analyte. The anti-human IgG (Fc) antibody (Human antibody Capture kit, Cytiva) was immobilized on Sensor Chip CM5 (Cytiva) in accordance with the instruction of the kit. The anti-CD98 antibodies diluted to 2 µg/ml with HBS-EP+ (Cytiva) to be evaluated were brought into contact at 5 µl/min for 60 seconds and immobilized. Thereafter, the CD98 antigen analytes diluted to various concentrations with HBS-EP+ were added at a flow rate of 30 µl/min for 90 seconds, and dissociation of the CD98 antigen was monitored for 250 seconds. K_{D} was determined by multi-cycle kinetics analysis. As shown in Figure 13 (A), binding affinity (K_{D}) of hM23H1L1 and that of hM23-M1 were 0.58 nM and 10.3 nM, respectively.

### 11)-2 Evaluation of masking effects of anti-CD98 masked antibody

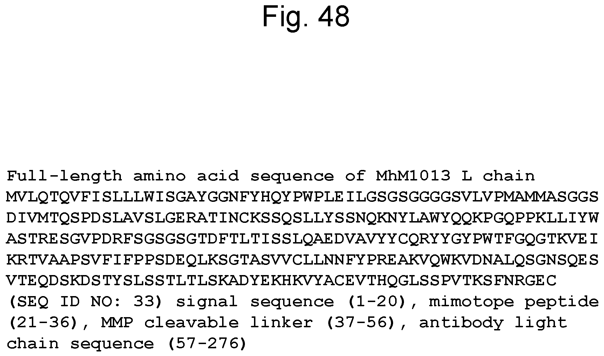

In the same manner as in Example 3, the ZPZP lib-derived mimotope was identified, and the anti-CD98 masked antibody MhM1013 (Table 3, SEQ ID NO: 33, Figure 48) and the anti-CD98 masked antibody MhM1013-M1 comprising the hM23-M1 heavy chain and the MhM1013 light chain (Table 3) were prepared. Except for the ways described below, the binding intensity thereof to the human CD98 antigen under the condition with the addition of MMP1 and under the condition without the addition thereof was evaluated by ELISA in the same manner as in Example 4 and Example 6. Under the condition with the addition of a protease, 200 nM MMP1 was added to the 2 µM antibody, the reaction was allowed to proceed at 37°C, and the antibody with the concentration thereof being adjusted with MMP buffer was added to the wells comprising the human CD98 antigen immobilized thereon. Under the condition without the addition of a protease, the MMP-buffered antibody was also added to the wells in the same manner.

As shown in Figure 13 (B), MhM1013 and MhM1013-M1 comprising the mimotope peptide fused thereto exhibited a higher binding intensity under the condition with the addition of MMP1 than under the condition without the addition of MMP1. The EC₅₀ ratio of binding intensity of MhM1013 and that of MhM1013-M1 (under the condition without the addition of MMP1/under the condition with the addition of MMP1) were 239 and 552, respectively.

### (Example 12) Preparation of anti-CD98 antibody subjected to ADC preparation and evaluation of binding intensity thereof

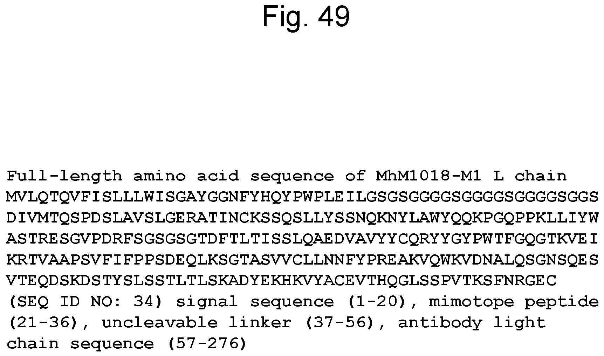

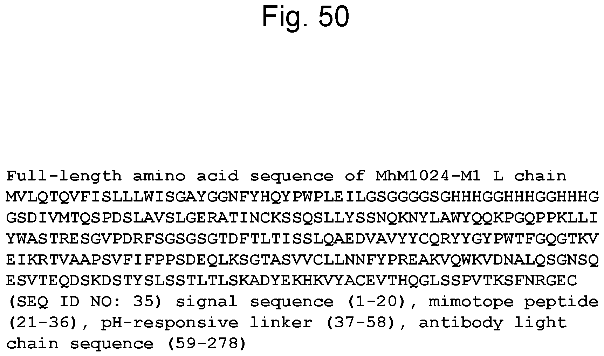

In order to examine as to whether or not cytotoxic activity of a masked antibody comprising the His cluster would be enhanced as a pH was lowered, the anti-CD98 masked antibody MhM1018-M1 (Table 3, SEQ ID NO: 34, Figure 49) not comprising the His cluster and the anti-CD98 masked antibody MhM1024-M1 (Table 3, SEQ ID NO: 35, Figure 50) comprising the His cluster were designed. The antibodies were prepared in the same manner as in Example 1)-1, and the binding intensity thereof to the human CD98 antigen under neutral pH conditions (pH 7.5) and under acidic pH conditions (pH 5.5) were evaluated by ELISA in the same manner as in Example 8.

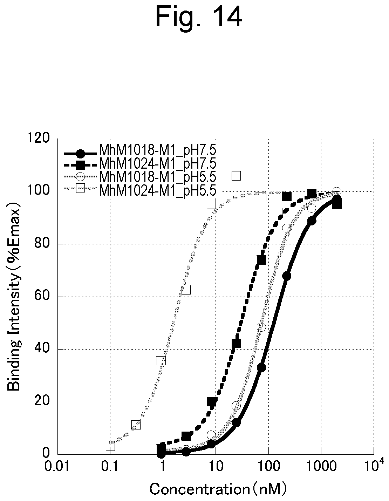

As shown in Figure 14, MhM1018-M1 exhibited an equivalent binding intensity under both pH conditions, and the EC₅₀ ratios (neutral pH conditions/acidic pH conditions) of both thereof were 1.7. In contrast, MhM1024-M1 exhibited a higher binding intensity under acidic pH conditions than under neutral pH conditions, and the EC₅₀ ratio (neutral pH conditions/acidic pH conditions) thereof was 19.2.

### (Example 13) Evaluation of growth inhibitory activity of ADC

The cells of the TROP2- and CD98-positive human head and neck cancer cell line FaDu (ATCC) were cultured in E-MEM medium (Wako) comprising 10% fetal bovine serum (Hyclone). FaDu was adjusted to 2.0 × 10⁴ cells/ml in E-MEM medium and added at 100 µl/well to a 96-well cell culture plate. Thereafter, FaDu was cultured at 37°C in the presence of 5% CO₂ overnight.

On the following day, anti-TROP2 masked PBD-ADC or anti-CD98 masked DXd-ADC were diluted to 300 nM, 100 nM, 33.3 nM, 11.1 nM, 3.7 nM, 1.2 nM, and 0.4 nM with pH adjusted E-MEM medium (7.4, 6.5, 6.0, and 5.5). The E-MEM medium was removed from the culture plate, and 100 µl of diluted anti-TROP2 masked PBD-ADC or anti-CD98 masked DXd-ADC were added to the culture plate. After culture was performed at 37°C in the presence of 5% CO₂ for 1 hour, the antibody-containing medium was removed, and 100 µl of the E-MEM medium adjusted to pH 7.4 was added. Culture was performed at 37°C in the presence of 5% CO₂ for 6 days. After the culture, the culture solution was mixed with the equivalent amount of CellTiter-Glo Liminescent Cell Viability Assay (Promega) and stirred with the use of a plate mixer. The resultant was allowed to stand at room temperature for 10 minutes, and the amount of luminescence was measured using a plate reader (PerkinElmer).

The cell viability was determined in accordance with the following equation:$Cell viability (%) = a \div b \times 100$
a: the average amount of luminescence of a well supplemented with a test material
b: the average amount of luminescence of a well supplemented with a medium

### 13)-1 Cytotoxic activity of anti-TROP2 masked PBD-ADC

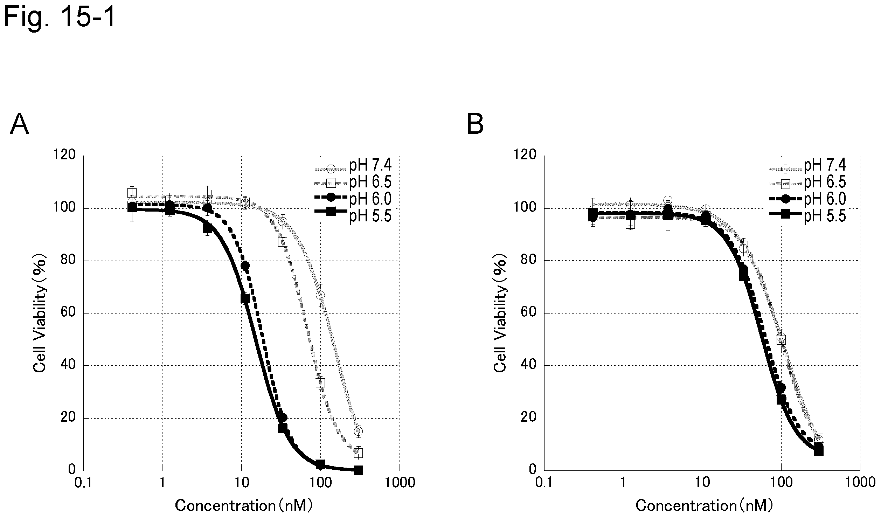

As shown in Figure 15-1 (A), MHT1808-PBD-ADC comprising the His cluster exhibited a higher growth inhibitory activity as a pH was lowered, and the EC₅₀ ratio (pH 7.4/pH 5.5) thereof was 10.7. MHT1221-PBD-ADC not comprising the His cluster an exhibited equivalent growth inhibitory activity under any conditions, and the EC₅₀ ratio (pH 7.4/pH 5.5) thereof was 1.9 (Figure 15-1 (B)).

### 13)-2 Cytotoxic activity of anti-CD98 masked DXd-ADC

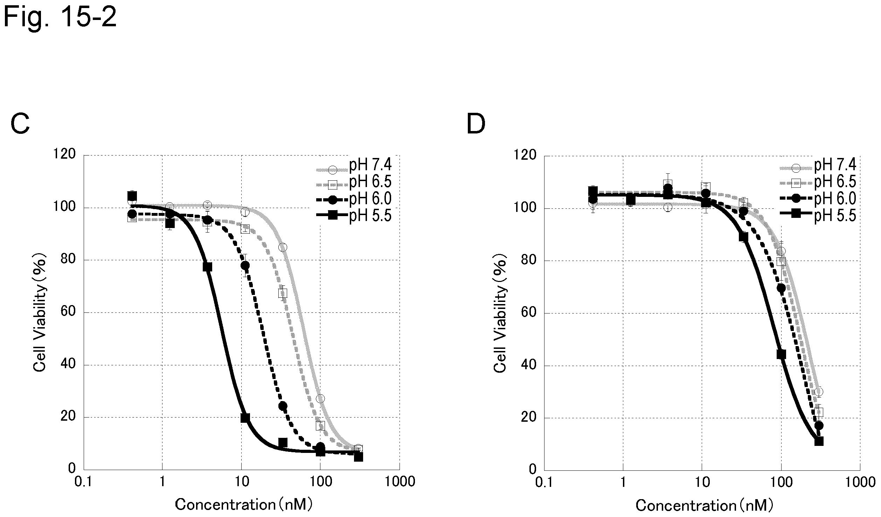

As shown in Figure 15-2 (C), MhM1024-M1-DXd-ADC comprising the His cluster exhibited a higher growth inhibitory activity as a pH was lowered, and the EC₅₀ ratio (pH 7.4/pH 5.5) thereof was 10.9. MhM1018-M1-DXd-ADC not comprising the His cluster exhibited an equivalent growth inhibitory activity under any conditions, and the EC₅₀ ratio (pH 7.4/pH 5.5) thereof was 2.8 (Figure 15-2 (D)).

### (Example 14) Enhancement of pH responsiveness

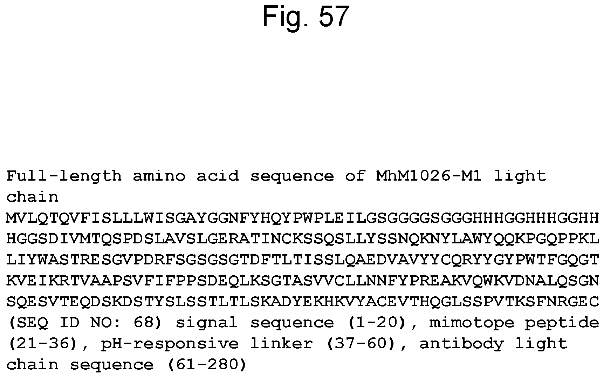

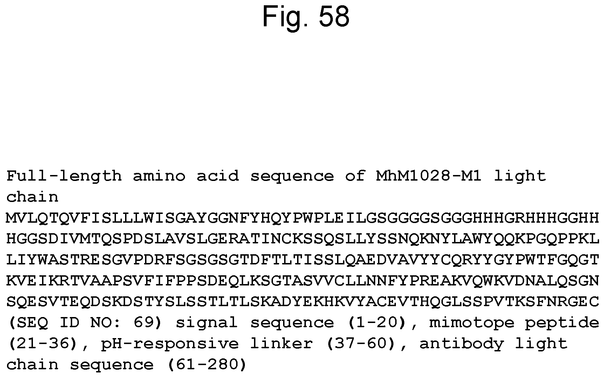

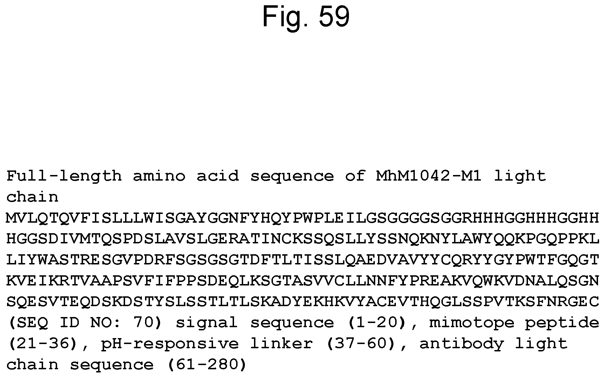

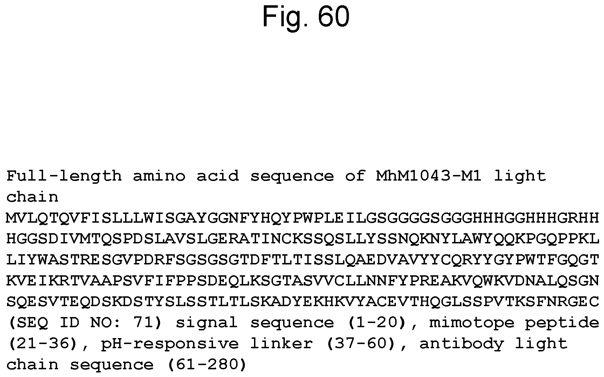

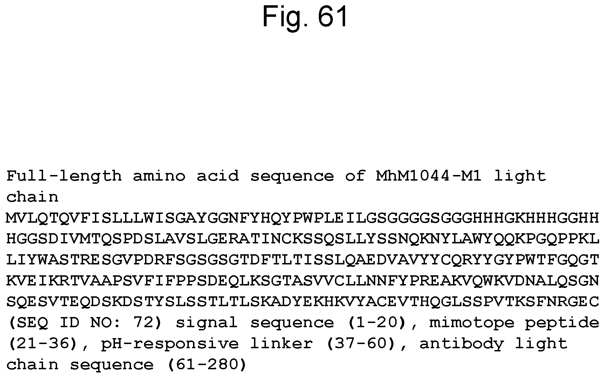

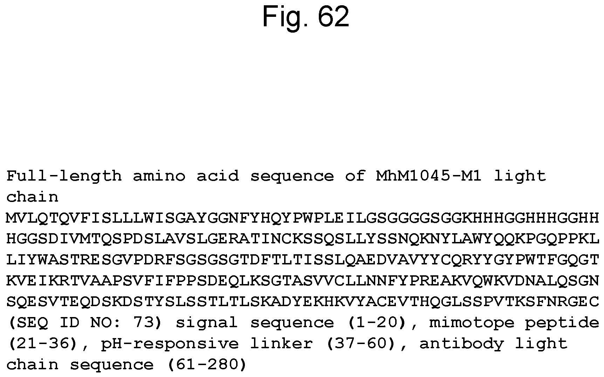

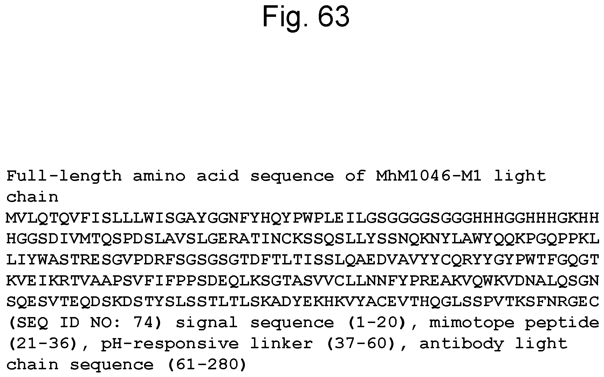

In order to enhance pH responsiveness, the anti-CD98 masked antibodies MhM1026-M1 (Table 5, SEQ ID NO: 68, Figure 57), MhM1028-M1 (Table 5, SEQ ID NO: 69, Figure 58), MhM1042-M1 (Table 5, SEQ ID NO: 70, Figure 59), MhM1043-M1 (Table 5, SEQ ID NO: 71, Figure 60), MhM1044-M1 (Table 5, SEQ ID NO: 72, Figure 61), MhM1045-M1 (Table 5, SEQ ID NO: 73, Figure 62), and MhM1046-M1 (Table 5, SEQ ID NO: 74, Figure 63) each comprising a positively charged amino acid (Arg or Lys) positioned in the vicinity of the His cluster were designed. Masked antibodies were prepared in the same manner as in Example 1 1)-1, and the binding intensity thereof under neutral pH conditions and under acidic pH conditions were evaluated by ELISA in the same manner as in Example 5 and Example 6.

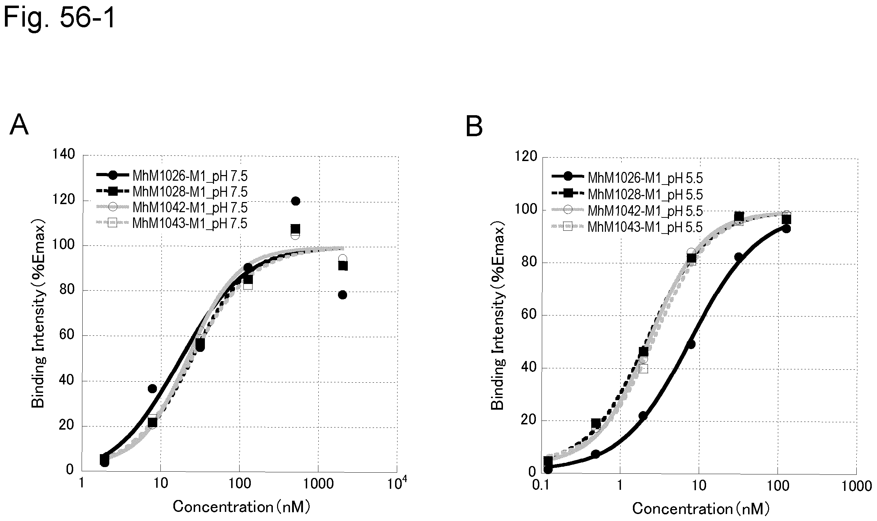

As shown in Figure 56-1 (A), MhM1026-M1, MhM1028-M1, MhM1042-M1, and MhM1043-M1 exhibited an equivalent binding intensity at a pH 7.5. At a pH 5.5, in contrast, MhM1028-M1, MhM1042-M1, and MhM1043-M1 exhibited a higher binding intensity than MhM1026-M1 (Figure 56-1 (B)). As a result of comparison of the EC₅₀ ratios (neutral pH conditions/acidic pH conditions) relative to the value of MhM1026-M1 designated as 1, pH responsiveness was found to be enhanced by 4.0 to 5.2 times by positioning Arg in the vicinity of the His cluster (Table 5).

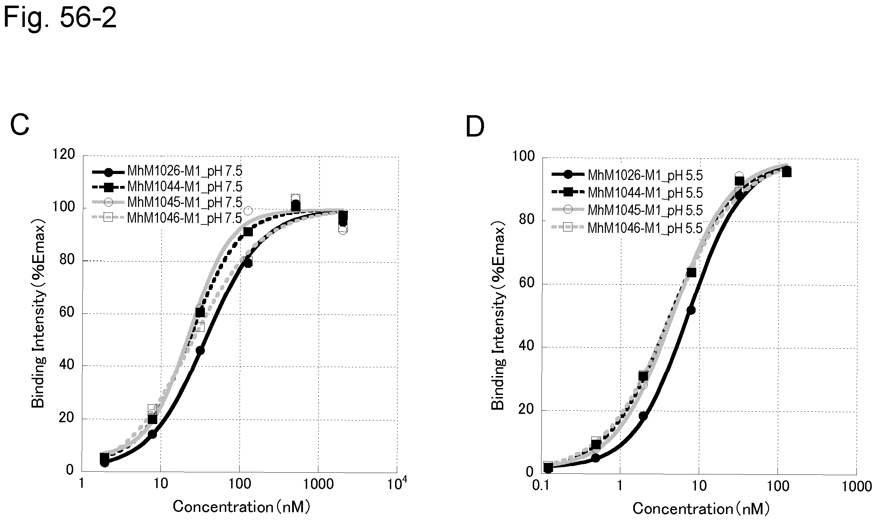

Also, the binding activities of MhM1044-M1, MhM1045-M1, and MhM1046-M1 each comprising Lys in the vicinity of the His cluster were compared with the binding intensity of MhM1026-M1. The results of comparison demonstrate that all the clones would exhibit an equivalent binding intensity, regardless of pH conditions, as shown in Figure 56-2 (C) and Figure 56-2 (D). As a result of comparison of the EC₅₀ ratios relative to the value of MhM1026-M1 designated as 1, no differences were observed among the clones (Table 5).

**Table 5: Amino acid sequence information of anti-CD98 antibody and comparison of EC₅₀ ratio**

| Masked antibody | Heavy chain sequence | Light chain sequence | Comparison of EC₅₀ ratio |
|---|---|---|---|
| MhM1026-M1 | hM23-M1 (SEQ ID NO: 31, Fig. 46) | MhM1026 (SEQ ID NO: 68, Fig. 57) | 1 |
| MhM1028-M1 | hM23-M1 (SEQ ID NO: 31, Fig. 46) | MhM1028 (SEQ ID NO: 69, Fig. 58) | 5.2 |
| MhM1042-M1 | hM23-M1 (SEQ ID NO: 31, Fig. 46) | MhM1042 (SEQ ID NO: 70, Fig. 59) | 4.3 |
| MhM1043-M1 | hM23-M1 (SEQ ID NO: 31, Fig. 46) | MhM1043 (SEQ ID NO: 71, Fig. 60) | 4.0 |
| MhM1044-M1 | hM23-M1 (SEQ ID NO: 31, Fig. 46) | MhM1044 (SEQ ID NO: 72, Fig. 61) | 1.1 |
| MhM1045-M1 | hM23-M1 (SEQ ID NO: 31, Fig. 46) | MhM1045 (SEQ ID NO: 73, Fig. 62) | 1.0 |
| MhM1046-M1 | hM23-M1 (SEQ ID NO: 31, Fig. 46) | MhM1046 (SEQ ID NO: 74, Fig. 63) | 1.1 |

### Industrial Applicability

The environmentally-responsive masked antibody according to the present invention can be used for treatment of various cancer species.

### [Sequence Listing Free Text]

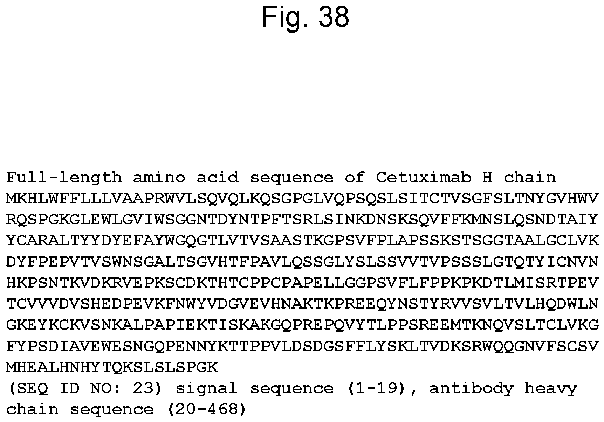

SEQ ID NO: 1: the amino acid sequence of the heavy chain of the anti-TROP2 antibody (Figure 16)
SEQ ID NO: 2: the amino acid sequence of the light chain of the anti-TROP2 antibody (Figure 17)
SEQ ID NO: 3: the amino acid sequence of MHT1001 (Figure 18)
SEQ ID NO: 4: the amino acid sequence of MHT1002 (Figure 19)
SEQ ID NO: 5: the amino acid sequence of HT1-11-scFv-HL (Figure 20)
SEQ ID NO: 6: the amino acid sequence of HT1-11-scFv-LH (Figure 21)
SEQ ID NO: 7: the amino acid sequence of the heavy chain of MHT1007 (Figure 22)
SEQ ID NO: 8: the amino acid sequence of the heavy chain of MHT1008 (Figure 23)
SEQ ID NO: 9: the amino acid sequence of the heavy chain of MHT1009 (Figure 24)
SEQ ID NO: 10: the amino acid sequence of the heavy chain of MHT1803 (Figure 25)
SEQ ID NO: 11: the amino acid sequence of the heavy chain of MHT1804 (Figure 26)
SEQ ID NO: 12: the amino acid sequence of the heavy chain of MHT1805 (Figure 27)
SEQ ID NO: 13: the amino acid sequence of the heavy chain of MHT1806 (Figure 28)
SEQ ID NO: 14: the amino acid sequence of the heavy chain of MHT1808 (Figure 29)
SEQ ID NO: 15: the amino acid sequence of the heavy chain of MHT1809 (Figure 30)
SEQ ID NO: 16: the amino acid sequence of the heavy chain of HMT1810 (Figure 31)
SEQ ID NO: 17: the amino acid sequence of the heavy chain of MHT1811 (Figure 32)
SEQ ID NO: 18: the amino acid sequence of the heavy chain of MHT1817 (Figure 33)
SEQ ID NO: 19: the amino acid sequence of the heavy chain of MHT1818 (Figure 34)
SEQ ID NO: 20: the amino acid sequence of the heavy chain of MHT1819 (Figure 35)
SEQ ID NO: 21: the amino acid sequence of the heavy chain of the anti-CD98 antibody hM23H1L1 (Figure 36)
SEQ ID NO: 22: the amino acid sequence of the light chain of the anti-CD98 antibody hM23H1L1 (Figure 37)
SEQ ID NO: 23: the amino acid sequence of the heavy chain of the anti-EGFR antibody Cetuximab (Figure 38)
SEQ ID NO: 24: the amino acid sequence of the light chain of the anti-EGFR antibody Cetuximab (Figure 39)
SEQ ID NO: 25: the amino acid sequence of the heavy chain of the anti-GPRC5D antibody C3022 (Figure 40)
SEQ ID NO: 26: the amino acid sequence of the light chain of the anti-GPRC5D antibody C3022 (Figure 41)
SEQ ID NO: 27: the amino acid sequence of the light chain of MhM8001 (Figure 42)
SEQ ID NO: 28: the amino acid sequence of the light chain of MCE-2101 (Figure 43)
SEQ ID NO: 29: the amino acid sequence of the heavy chain of MC3-9001 (Figure 44)
SEQ ID NO: 30: the amino acid sequence of the heavy chain of MHT1221 (Figure 45)
SEQ ID NO: 31: the amino acid sequence of the heavy chain of hM23-M1 (Figure 46)
SEQ ID NO: 32: the amino acid sequence of the light chain of hM23-M1 (Figure 47)
SEQ ID NO: 33: the amino acid sequence of the light chain of MhM1013 (Figure 48)
SEQ ID NO: 34: the amino acid sequence of the light chain of MhM1018-M1 (Figure 49)
SEQ ID NO: 35: the amino acid sequence of the light chain of MhM1024-M1 (Figure 50)
SEQ ID NO: 36: the amino acid sequence recognized by human uPA and serving as a substrate for human uPA (Figure 51)
SEQ ID NO: 37: the amino acid sequence recognized by human uPA and serving as a substrate for human uPA (Figure 51)
SEQ ID NO: 38: the amino acid sequence recognized by human uPA and serving as a substrate for human uPA (Figure 51)
SEQ ID NO: 39: the amino acid sequence recognized by human MMP1 and serving as a substrate for human MMP1 (Figure 51)
SEQ ID NO: 40: the amino acid sequence recognized by human MMP1 and serving as a substrate for human MMP1 (Figure 51)
SEQ ID NO: 41 the amino acid sequence recognized by human MMP9 and serving as a substrate for human MMP9 (Figure 51)
SEQ ID NO: 42: the amino acid sequence of the second peptide (Figure 52)
SEQ ID NO: 43: the amino acid sequence of the second peptide (Figure 52)
SEQ ID NO: 44: the amino acid sequence of the second peptide (Figure 52)
SEQ ID NO: 45: the amino acid sequence of the amino terminal peptide of human GPRC5D (Figure 53)
SEQ ID NO: 46: the amino acid sequence of the second peptide (Figure 54)
SEQ ID NO: 47: the amino acid sequence of the second peptide (Figure 54)
SEQ ID NO: 48: the amino acid sequence of the second peptide (Figure 54)
SEQ ID NO: 49: the amino acid sequence of the second peptide (Figure 54)
SEQ ID NO: 50: the amino acid sequence of the second peptide (Figure 54)
SEQ ID NO: 51: the amino acid sequence of the second peptide (Figure 54)
SEQ ID NO: 52: the amino acid sequence of the second peptide (Figure 54)
SEQ ID NO: 53: the amino acid sequence of the second peptide (Figure 54)
SEQ ID NO: 54: the amino acid sequence of the second peptide (Figure 54)
SEQ ID NO: 55: the amino acid sequence of the second peptide (Figure 54)
SEQ ID NO: 56: the amino acid sequence of the second peptide (Figure 54)
SEQ ID NO: 57: the amino acid sequence of the second peptide (Figure 54)
SEQ ID NO: 58: the amino acid sequence of the second peptide (Figure 54)
SEQ ID NO: 59: the amino acid sequence of the second peptide (Figure 54)
SEQ ID NO: 60: the amino acid sequence of the second peptide (Figure 54)
SEQ ID NO: 61: the amino acid sequence of the second peptide (Figure 54)
SEQ ID NO: 62: the amino acid sequence of the second peptide (Figure 54)
SEQ ID NO: 63: the amino acid sequence of the second peptide (Figure 54)
SEQ ID NO: 64: the amino acid sequence of the second peptide (Figure 54)
SEQ ID NO: 65: the amino acid sequence of the second peptide (Figure 54)
SEQ ID NO: 66: the amino acid sequence of the second peptide (Figure 54)
SEQ ID NO: 67: the amino acid sequence recognized by human CAPN1 and serving as a substrate for human CAPN1 (Figure 55)
SEQ ID NO: 68: the amino acid sequence of the light chain of MhM1026-M1 (Figure 57)
SEQ ID NO: 69: the amino acid sequence of the light chain of MhM1028-M1 (Figure 58)
SEQ ID NO: 70: the amino acid sequence of the light chain of MhM1042-M1 (Figure 59)
SEQ ID NO: 71: the amino acid sequence of the light chain of MhM1043-M1 (Figure 60)
SEQ ID NO: 72: the amino acid sequence of the light chain of MhM1044-M1 (Figure 61)
SEQ ID NO: 73: the amino acid sequence of the light chain of MhM1045-M1 (Figure 62)
SEQ ID NO: 74: the amino acid sequence of the light chain of MhM1046-M1 (Figure 63)
SEQ ID NO: 75: the amino acid sequence of the second peptide (Figure 64)
SEQ ID NO: 76: the amino acid sequence of the second peptide (Figure 64)
SEQ ID NO: 77: the amino acid sequence of the second peptide (Figure 64)
SEQ ID NO: 78: the amino acid sequence of the second peptide (Figure 64)
SEQ ID NO: 79: the amino acid sequence of the second peptide (Figure 64)
SEQ ID NO: 80: the amino acid sequence of the second peptide (Figure 64)
SEQ ID NO: 81: the amino acid sequence of the second peptide (Figure 64)

All publications, patents, and patent applications cited herein are incorporated herein by reference in their entirety.

## Claims

1. A molecule comprising a moiety [a], a moiety [b], and a moiety [c] indicated below and binding to a target antigen:
a moiety [a]: a moiety binding to the target antigen;
a moiety [b]: a first peptide recognizing a target antigen-binding site in the moiety [a]; and
a moiety [c]: a second peptide consisting of an amino acid sequence comprising amino acids that are converted from an uncharged state to a positively charged state in response to a pH change from neutral to acidic conditions at a pH 7.5 or lower.

2. The molecule according to claim 1, which exhibits higher binding affinity to the target antigen under acidic conditions than under neutral conditions.

3. The molecule according to claim 1 or 2, which comprises one or two or more of the moiety [a], the moiety [b], and the moiety [c].

4. The molecule according to any one of claims 1 to 3, which comprises one or two or more of the moiety [a], the moiety [b], and the moiety [c] and the number of the moiety [a], that of the moiety [b], and that of the moiety [c] are identical to each other.

5. The molecule according to claim 1 or 2, which comprises 2 moieties [a], 2 moieties [b], and 2 moieties [c].

6. The molecule according to any one of claims 1 to 5, wherein the second peptide consists of an amino acid sequence comprising 4 or more amino acids that are converted from an uncharged state to a positively charged state in response to a pH change from neutral to acidic conditions at a pH 7.5 or lower and has a pI value exceeding 6.4.

7. The molecule according to any one of claims 1 to 6, wherein the second peptide consists of an amino acid sequence comprising 4 or more amino acids that are converted from an uncharged state to a positively charged state in response to a pH change from neutral to acidic conditions at a pH 7.5 or lower and has a pI value of 6.8 or higher.

8. The molecule according to any one of claims 1 to 7, wherein the second peptide consists of an amino acid sequence comprising 4 or more amino acids that are converted from an uncharged state to a positively charged state in response to a pH change from neutral to acidic conditions at a pH 7.5 or lower and has a pI value of 7.2 or higher.

9. The molecule according to any one of claims 1 to 8, wherein the second peptide consists of an amino acid sequence comprising 4 or more amino acids that are converted from an uncharged state to a positively charged state in response to a pH change from neutral to acidic conditions at a pH 7.5 or lower and has a pI value of 7.6 or higher.

10. The molecule according to any one of claims 1 to 9, wherein the second peptide consists of an amino acid sequence comprising no negatively charged amino acids under acidic pH conditions.

11. The molecule according to any one of claims 1 to 10, wherein the second peptide consists of an amino acid sequence that does not comprise aspartic acid and/or glutamic acid.

12. The molecule according to any one of claims 1 to 11, wherein the second peptide consists of an amino acid sequence having the configurations (1) and (2) below:
(1) at least 1 of (a) an amino acid sequence consisting of 2 or more consecutive amino acids having basic functional groups with pKa of 7 or lower in side chains or (b) an amino acid sequence derived from the amino acid sequence (a) with insertion or addition of an arginine or lysine residue at any position (including an amino- or carboxyl terminus) (the amino acid sequence (a) and the amino acid sequence (b) are each referred to as a cluster of basic amino acids); and
(2) an amino acid sequence comprising 4 or more of the amino acids having basic functional groups with pKa of 7 or lower in side chains.

13. The molecule according to any one of claims 1 to 12, wherein the second peptide consists of an amino acid sequence having the configurations (1) and (2) below:
(1) comprising at least 1 amino acid sequence derived from the amino acid sequence consisting of 2 or more consecutive amino acids having basic functional groups with pKa of 7 or lower in side chains with insertion or addition of an arginine residue at any site (including an amino or carboxyl terminus); and
(2) comprising an amino acid sequence comprising 4 or more of the amino acids having basic functional groups with pKa of 7 or lower in side chains.

14. The molecule according to any one of claims 1 to 13, wherein the second peptide consists of an amino acid sequence having the configurations (1) and (2) below:
(1) comprising an amino acid sequence derived from the amino acid sequence consisting of 2 or more consecutive amino acids having basic functional groups with pKa of 7 or lower in side chains by insertion or addition of an arginine residue at any site (including an amino or carboxyl terminus); and
(2) comprising an amino acid sequence comprising 4 or more of the amino acids having basic functional groups with pKa of 7 or lower in side chains.

15. The molecule according to any one of claims 1 to 14, wherein the second peptide consists of an amino acid sequence having the configurations (1) and (2) below:
(1) comprising an amino acid sequence represented by RHHH; and
(2) comprising an amino acid sequence comprising 4 or more of the amino acids having basic functional groups with pKa of 7 or lower in side chains.

16. The molecule according to any one of claims 1 to 12, wherein the second peptide consists of an amino acid sequence comprising at least 1 of the 2 or more consecutive amino acids having basic functional groups with pKa of 7 or lower in side chains and 4 or more of the amino acids having basic functional groups with pKa of 7 or lower in side chains.

17. The molecule according to any one of claims 1 to 11, wherein the second peptide consists of an amino acid sequence comprising amino acids having basic functional groups with pKa of 7 or lower in side chains at every other position.

18. The molecule according to any one of claims 12 to 17, wherein the basic functional group has pKa of 5.0 to 7.0.

19. The molecule according to any one of claims 12 to 18, wherein the basic functional group has pKa of 5.5 to 6.5.

20. The molecule according to any one of claims 12 to 19, wherein the amino acids comprising basic functional groups with pKa of 7 or lower in side chains are natural amino acids.

21. The molecule according to any one of claims 12 to 19, wherein the amino acids comprising basic functional groups with pKa of 7 or lower in side chains are unnatural amino acids.

22. The molecule according to any one of claims 12 to 20, wherein the amino acids comprising basic functional groups with pKa of 7 or lower in side chains are histidine residues.

23. The molecule according to any one of claims 1 to 22, wherein the second peptide consists of an amino acid sequence comprising 1 to 4 clusters of basic amino acids.

24. The molecule according to any one of claims 1 to 23, wherein the second peptide consists of 10 to 60 amino acids.

25. The molecule according to any one of claims 1 to 24, wherein the second peptide consists of 12 to 50 amino acids.

26. The molecule according to any one of claims 1 to 25, wherein the second peptide consists of 15 to 40 amino acids.

27. The molecule according to any one of claims 1 to 26, wherein the second peptide consists of 20 to 35 amino acids.

28. The molecule according to any one of claims 1 to 16 and 18 to 27, wherein the second peptide comprises an amino acid sequence represented by any one of SEQ ID Nos: 49 to 53, 55, 59 to 61, 65, 66, and 75 to 81 (Figure 54 and Figure 64).

29. The molecule according to any one of claims 17 to 27, wherein the second peptide comprises an amino acid sequence represented by SEQ ID NO: 54 (Figure 54).

30. The molecule according to any one of claims 1 to 29, wherein the EC₅₀ ratio is 3 or higher.

31. The molecule according to any one of claims 1 to 30, wherein the EC₅₀ ratio is 10 or higher.

32. The molecule according to any one of claims 1 to 31, wherein the EC₅₀ ratio is 30 or higher.

33. The molecule according to any one of claims 1 to 32, wherein the second peptide consists of an amino acid sequence comprising 10 or fewer amino acids having basic functional groups with pKa of 7 or lower in side chains.

34. The molecule according to any one of claims 1 to 33, wherein the amino acid sequence of the second peptide comprises 2 or more clusters of basic amino acids, a cluster of basic amino acids is positioned at the amino or carboxyl terminus of the amino acid sequence, and other clusters of basic amino acids are not positioned at the amino or carboxyl terminus of the amino acid sequence.

35. The molecule according to any one of claims 1 to 33, wherein the amino acid sequence of the second peptide comprises 2 or more clusters of basic amino acids, a cluster of basic amino acids is positioned at the amino terminus of the amino acid sequence, and another cluster of basic amino acids is positioned at the carboxyl terminus.

36. The molecule according to any one of claims 1 to 33, wherein none of the clusters of basic amino acids comprised in the amino acid sequence of the second peptide are positioned at the amino or carboxyl terminus of the amino acid sequence.

37. The molecule according to any one of claims 1 to 34 and 36, wherein the amino acid sequence of the second peptide comprises an amino acid sequence cleaved by an intracellular and/or extracellular protease and an amino acid sequence comprising a cluster of basic amino acids, wherein the amino acid sequence of the second peptide comprises:
(1) an amino acid sequence comprising a cluster of basic amino acids and an amino acid sequence cleaved by an intracellular and/or extracellular protease connected in that order from the amino terminus toward the carboxyl terminus; or
(2) an amino acid sequence cleaved by an intracellular and/or extracellular protease and an amino acid sequence comprising a cluster of basic amino acids connected in that order from the carboxyl terminus toward the amino terminus.

38. The molecule according to any one of claims 12 to 37, wherein the number of amino acids having basic functional groups with pKa of 7 or lower in side chains in the amino acid sequence of the second peptide is 5% to 30% of the total number of amino acids comprised in the amino acid sequence.

39. The molecule according to any one of claims 1 to 38, which comprises the first peptide, the second peptide, and the moiety binding to a target antigen connected in that order.

40. The molecule according to any one of claims 1 to 39, wherein the moiety binding to a target antigen is a polypeptide consisting of an amino acid sequence that is not comprised in the first peptide or the second peptide.

41. The molecule according to any one of claims 1 to 40, which consists of a polypeptide.

42. The molecule according to claim 41, which comprises the first peptide, the second peptide, and the moiety binding to a target antigen connected from the amino terminus toward the carboxyl terminus.

43. The molecule according to any one of claims 1 to 42, wherein any one of a moiety selected from the group consisting of the first peptide, the second peptide, and the moiety binding to a target antigen is connected to either or both of the other two moieties by a linker or spacer (or linkers or spacers).

44. The molecule according to any one of claims 1 to 43, wherein a pH of acidic conditions is 6.5 or lower.

45. The molecule according to any one of claims 1 to 44, wherein a pH of acidic conditions is 6.0 or lower.

46. The molecule according to any one of claims 1 to 45, wherein a pH of acidic conditions is 5.5 or lower.

47. The molecule according to any one of claims 1 to 46, wherein the target antigen is an antigen that is present in a tumor tissue, an endosome, or a lysosome.

48. The molecule according to any one of claims 1 to 47, wherein another moiety [d] binds to a moiety binding to the target antigen and the moiety [d] does not comprise the first peptide and the second peptide.

49. The molecule according to claim 48, wherein the moiety [d] is at least 1 substance selected from the group consisting of an antibody or an antigen-binding fragment thereof that is not a moiety binding to the target antigen, a peptide comprising an amino acid sequence that is not comprised in the first peptide or the second peptide, a cytokine, a toxin, a radioactive isotope, a label molecule, a photosensitizer, an immunostimulant, an antitumor compound, a drug, a payload, and a polymer.

50. The molecule according to claim 49, wherein the antitumor compound is a camptothecin derivative or a pyrrolobenzodiazepine derivative and the camptothecin derivative is preferably N-[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]-2-hydroxyacetamide.

51. The molecule according to claim 49, wherein the immunostimulant is a cyclic dinucleotide derivative.

52. The molecule according to any one of claims 48 to 51, which consists of a polypeptide or the moiety [d] and a polypeptide.

53. The molecule according to any one of claims 1 to 52, wherein the first peptide is obtained by a method comprising steps (i) to (iii):
(i) a step of bringing a peptide library comprising a repeat sequence of aromatic amino acid and Pro into contact with the moiety binding to a target antigen as defined in [1];
(ii) a step of collecting a peptide binding to the moiety binding to a target antigen; and
(iii) a step of preparing the peptide by recombination, chemical synthesis, or peptide synthesis.

54. A method for producing the molecule according to any one of claims 1 to 52, which comprises a step of preparing a peptide comprising an amino acid sequence comprised in the first peptide and/or an amino acid sequence comprised in the second peptide by recombination, *in vitro* translation, chemical synthesis, or peptide synthesis.

55. A molecule binding to a target antigen, which is obtained by the method according to claim 54.

56. A method for producing the molecule according to any one of claims 41, 42, and 52 comprising a step (iv) below:
(iv) a step of introducing a polynucleotide comprising a nucleotide sequence encoding an amino acid sequence comprised in a moiety binding to a target antigen and, optionally, an amino acid sequence comprised in the first peptide, and/or an amino acid sequence comprised in the second peptide into a cell, culturing the cell, and collecting a polypeptide binding to the target antigen from the culture product.

57. A molecule binding to a target antigen, which is obtained by the method according to claim 56.

58. A pharmaceutical composition comprising the molecule according to any one of claims 1 to 53, 55, and 57, a salt thereof, or a hydrate of the molecule or salt.

59. The pharmaceutical composition according to claim 58, which is an anti-cancer agent.

60. The pharmaceutical composition according to claim 58 or 59, wherein an antigen-binding intensity of a molecule binding to a target antigen under neutral pH conditions is adjusted to be lower than the antigen-binding intensity under acidic pH conditions, so as to reduce the toxicity of the molecule binding to a target antigen imposed on a subject compared with the toxicity of a molecule comprising no amino acids converted from an uncharged state to a positively charged state in response to a pH change of the second peptide from neutral to acidic conditions at a pH 7.5 or lower.

61. The pharmaceutical composition according to claim 58 or 59, wherein an antigen-binding intensity of a molecule binding to a target antigen under neutral pH conditions is adjusted to be lower than the antigen-binding intensity under acidic pH conditions, so as to increase the blood concentration of the molecule which binds to the target antigen in a subject, compared with that of a molecule comprising no amino acids that are converted from an uncharged state to a positively charged state in response to a pH change of the second peptide from neutral to acidic conditions at a pH 7.5 or lower

62. A test drug, diagnostic drug, or reagent comprising the molecule according to any one of claims 1 to 53, 55, and 57 or the pharmaceutical composition according to claim 58.

63. A polynucleotide comprising a nucleotide sequence encoding the amino acid sequence of the molecule according to claim 41 or 52.

64. A vector comprising the polynucleotide according to claim 63.

65. A cell comprising the polynucleotide according to claim 63 or the vector according to claim 64 or producing the molecule according to claim 41 or 52.
